# EUROPEAN PATENT SPECIFICATION

(11) **EP 0 790 308 B1**
(45) Date of publication and mention of the grant of the patent: **04.07.2007**
(21) Application number: 97104682.6
(22) Date of filing: 09.12.1990
(51) Int. Cl.: C12N 15/24, C07K 14/54, A61K 38/20, C07K 16/24, C12N 1/21

(54) **Cytotoxic lymphocyte maturation factor 35kD subunit and monoclonal antibodies directed thereto**
Zytotoxischer Lymphozyten-Reifefaktor 35kD Untereinheit und monoklonale Antikörper spezifisch dafür
Sous-unité de 35kD du facteur de maturation de lymphocyte cytotoxique et anticorps monoclonaux dirigés contre celle-ci

(30) Priority: 22.12.1989 US 455708; 09.05.1990 US 520935; 27.08.1990 US 572284
(43) Date of publication of application: 20.08.1997
(62) Divisional of application: 90123670.3
(73) Proprietor: F. HOFFMANN-LA ROCHE AG, 4070 Basel (CH)
(72) Inventor: Chizzonite, Richard Anthony, South Kent, Connecticut 06785 (US); Gately, Maurice Kent, Morris Planes, N.J. 07950 (US); Gubler, Ulrich Andreas, Glen Ridge, N.J. 07456 (US); Hulmes, Jeffrey David W. Alton Jones Cell Science, New York 12946 (US); Pan, Yu-Ching Eugene, Pine Book, N.J. 07058 (US); Podlanski, Frank John, New City, N.J. 10956 (US); Stern, Alvin Seth, Passaic Parc, N.J. 07055 (US)
(74) Representative: Schreiner, Siegfried

(56) References cited:
- WO-A-90/05147
- WO-A-92/05256
- THE JOURNAL OF EXPERIMENTAL MEDICINE, vol. 170, no. 3, 1 September 1989, THE ROCKEFELLER UNIVERSITY PRESS, NEW YORK, US, pages 827-845, XP002033285 M. KOBAYASHI ET AL: "Identification and purification of natural killer cell stimulatory factor (NKSF) , a cytokine with multiple biologic effectson human lymphocytes"
- PROCEEDINGS OF THE NATIONAL ACADEMY OF SCIENCES OF USA, vol. 87, 5 September 1990, WASHINGTON US, pages 6808-6812, XP002033286 A.S. STERN ET AL: "Purification to homogeneity and partial characterization of Cytotoxic Lymphocyte Maturation Factor from human B-lymphoblastoid cells"
- GUBLER U. ET AL: 'Coexpression of two distinct genes is required to generate secreted bioactive cytotoxic lymphocyte naturation factor' PROC. NATL. ACAD. SCI. USA vol. 88, May 1991, pages 4143 - 4147

## Description

The present invention relates to the field of cytokines, in particular to those cytokines which synergize with interleukin-2 (IL-2) to activate cytotoxic lymphocytes such as the cytokine Cytotoxic Lymphocyte Maturation Factor (CLMF). The present invention also relates to monoclonal antibodies directed to CLMF.

'Cytokine' is one term for a group of protein cell regulators, variously called lymphokines, monokines, interleukins and interferons, which are produced by a wide variety of cells in the body. These cytokines play an important role in many physiological responses, are involved in the pathophysiology of a range of diseases, and have therapeutic potential. They are a heterogeneous group of proteins having the following characteristics in common. They are low molecular weight (≤80 kDa) secreted proteins which are often glycosylated; they are involved in immunity and inflammation where they regulate the amplitude and duration of a response; and are usually produced transiently and locally, acting in a paracrine or autocrine, rather than endocrine manner. Cytokines are extremely potent, generally acting at picomolar concentrations; and interact with high affinity cell surface receptors specific for each cytokine or cytokine group. Their cell surface binding ultimately leads to a change in the pattern of cellular RNA and protein synthesis, and to altered cell behavior. Individual cytokines have multiple overlapping cell regulatory actions.

The response of a cell to a given cytokine is dependent upon the local concentration of the cytokine, upon the cell type it is acting on and upon other cell regulators to which it is concomitantly exposed. The overlapping regulatory actions of these structurally unrelated proteins which bind to different cell surface receptors is at least partially accounted for by the induction of common proteins which can have common response elements in their DNA. Cytokines interact in a network by: first, inducing each other; second, transmodulating cytokine cell surface receptors and third, by synergistic, additive or antagonistic interactions on cell function. [Immunology Today 10: 299 (1989)].

The potential utility of cytokines in the treatment of neoplasia and as immunoenhancing agents has recently been demonstrated in studies using human recombinant interleukin-2 (rIL-2). Natural interleukin-2 (IL-2) is a lymphokine which is produced and secreted by T-lymphocytes. This glycoprotein molecule is intimately involved in the induction of virtually all immune responses in which T-cells play a role. B cell responses in vitro are also enhanced by the presence of IL-2. IL-2 has also been implicated as a differentiation inducing factor in the control of B and T lymphocyte responses.

Administration of human rIL-2 has been shown in some cases to result in regression of established tumors in both experimental animals [J. Exp. Med. 161:1169-1188 (1985)] and in man [N. Engl. J. Med. 313: 1485-1492 (1985) and N. Engl. J. Med. 316:889-897 (1987)]. The anti-tumor effects of rIL-2 are thought to be mediated by host cytotoxic effector lymphocytes which are activated by rIL-2 in vivo [J. Immunol. 139:285-294 (1987)]. In addition, results from animal models suggest that rIL-2 might also have value in the treatment of certain infectious diseases [J. Immunol. 135:4160-4163 (1985) and J. Virol. 61:2120-2127 (1987)] and in ameliorating chemotherapy-induced immunosuppression [Immunol. Lett. 10:307-314 (1985)].

However, the clinical use of rIL-2 has been complicated by the serious side effects which it may cause [N. Engl. J. Med. 313:1485-1492 (1985) and N. Engl. J. Med. 316:889-897 (1987)]. One approach to improving the efficacy of cytokine therapy while reducing toxicity is to use two or more cytokines in combination. For example, synergistic antitumor activity has been shown to result when rIL-2 is administered to tumor-bearing mice together with recombinant interferon alpha (rIFN alpha) [Cancer Res. 48:260-264 (1988) and Cancer Res. 48:5810-5817 (1988)] or with recombinant tumor necrosis factor alpha (rTNF alpha)[Cancer Res. 47:3948-3953 (1987)]. Since the antitumor effects of IL-2 are thought to be mediated by host cytotoxic effector lymphocytes, it would be of interest to identify and isolate novel cytokines which synergize with rIL-2 to activate cytotoxic lymphocytes in vitro. These novel cytokines would also be useful as antitumor agents when administered in combination with rIL-2 in vivo.

Kobayashi et al. (J. Exp. Med. (1989) 170, 827-845) relates to the identification and purification of natural killer cell stimulatory factor (NKSF), a cytokine with multiple biologic effects on human lymphocytes.

Thus, the present invention provides a 35 kDa subunit of a cytokine protein called Cytotoxic Lymphocyte Maturation Factor (CLMF) which is produced and synthesized by cells capable of secreting CLMF. Examples for such cells are mammalian cells particularly human lymphoblastoid cells. In the presence of low concentrations of IL-2 CLMF synergistically induces the cytolytic activity of Lymphokine Activated Killer (LAK) cells. CLMF is also capable of stimulating T-cell growth.

CLMF can be isolated in a substantially pure form by the/following steps:
a) stimulating B lymphoblastoid cells such as NC-37 cells to produce and secrete cytokines into a supernatant liquid;
b) collecting the supernatant liquid produced by the stimulated cells;
c) separating the supernatant liquid into protein fractions;
d) testing each protein fraction for the presence of CLMF;
e) retaining the protein fractions which are able to stimulate T-cell growth, said fractions containing an active protein which is responsible for the T-cell stimulating activity of the protein fractions;
f) isolating said active protein into a substantially pure form, said protein being Cytolytic Lymphocyte Maturation Factor (CLMF).

The CLMF protein obtained in this way is free from other cytokine proteins. The natural CLMF protein is a 75 kilodalton (kDa) heterodimer comprised of two polypeptide subunits, a 40 kDa subunit and a 35 kDa subunit which are bonded together via one or more disulfide bonds. The present invention also provides the nucleotide sequence of the 35 kDa subunit of the CLMF gene and the amino acid sequence of the 35 kDa subunit of the CLMF protein encoded by the said gene. The present invention relates to a protein which exhibits CLMF activity and contains a biologically active portion of the amino acid sequence of CLMF or which contains an amino acid sequence of CLMF as well as other amino acids or proteins containing analogous sequences to CLMF or its biologically active fragments which proteins exhibit CLMF activity.

The above process steps c) to f) may be used to purify CLMF from any liquid or fluid which contains CLMF together with other proteins. The present invention relates also to protein fractions having CLMF activity and being capable of stimulating T-cell growth, to a substantially purified active CLMF protein, obtained by the above described process, to the isolated cloned gene encoding the 35 kDa subunit, to vectors containing this gene to host cells transformed with the vector containing the said gene and to CLMF proteins prepared in such a transformed host cell. Furthermore the present invention relates to isolated polyclonal or monoclonal antibodies capable of binding to CLMF.

Monoclonal antibodies prepared against a partially purified preparation of CLMF have been identified and characterized by 1: immunoprecipitation of ¹²⁵I-labelled CLMF, 2: immunodepletion of CLMF bioactivity, 3: western blotting of CLMF, 4: inhibition of ¹²⁵I-CLMF binding to its cellular receptor and 5: neutralization of CLMF bioactivity. Twenty hybridomas secreting anti-CLMF antibodies were identified. The antibodies were found to immunoprecipitate ¹²⁵I-labelled CLMF and to immunodeplete CLMF bioactivity as assessed in the T-cell proliferation and LAK cell induction assays. Western blot analysis showed that each antibody binds to the 70 kDa heterodimer and to one of the subunits. Each of the above-mentioned 20 anti-CLMF monoclonal antibodies were specific for CLMF and in particular for the 40 kDa subunit of CLMF. A CLMF receptor binding assay has been developed to evaluate the ability of individual antibodies to inhibit CLMF binding to its cellular receptor. The assay measures the binding of ¹²⁵I-labelled CLMF to PHA activated PBL blast cells in the presence and absence of each antibody. Of the 20 antibodies tested, 12 antibodies were found to inhibit greater than 60% of the ¹²⁵I-labelled CLMF binding to the blast cells. Two inhibitory antibodies, viz. 7B2 and 4A1, neutralize CLMF bioactivity while one non-inhibitory antibody, 8E3, does not neutralize CLMF bioactivity. These data confirm that antibodies which block ¹²⁵I-labelled CLMF binding to its cellular receptor will neutralize CLMF bioactivity as assessed by the T-cell proliferation and LAK cell induction assays. The ability of the antibodies specific for the 40 kDa subunit of CLMF to neutralize CLMF bioactivity indicates that determinants on the 40 kDa subunit are necessary for binding to the CLMF cellular receptor.

The monoclonal anti-CLMF antibodies provide powerful analytical, diagnostic and therapeutic reagents for the immunoaffinity purification of natural and recombinant human CLMF, the development of human CLMF immunoassays, the identification of the active site of the 40 kDa subunit of CLMF and may be used in therapeutic treatments of patients which require selective immunosuppression of cytotoxic T cells, such as in transplantation. Monoclonal antibodies which recognize different epitopes on human CLMF can be used as reagents in a sensitive two-site immunoassay to measure levels of CLMF in biological fluids, cell culture supernatants and human cell extracts.

The monoclonal antibodies against CLMF exhibit a number of utilities including but not limited to:
1. Utilizing the monoclonal antibodies as affinity reagents for the purification of natural and recombinant human CLMF;
2. Utilizing the monoclonal antibodies as reagents to configure enzyme-immunoassays and radioimmunoassays to measure natural and recombinant CLMF in biological fluids, cell culture supernatants, cell extracts and on plasma membranes of human cells and as reagents for a drug screening assay:
3. Utilizing the monoclonal antibodies as reagents to construct sensitive two-site immunoassays to measure CLMF in biological fluids, cell culture supernatants and human cell extracts;
4. Utilizing the monoclonal antibodies as reagents to identify determinants of the 40 kDa subunit which participate in binding to the 35 kDa subunit and which participate in binding to the CLMF cellular receptor;
5. Utilizing the intact IgG molecules, the Fab fragments or the humanized IgG molecules of the inhibitory monoclonal antibodies as therapeutic drugs for the selective blockade of proliferation and activation of cytotoxic T cells, such as in transplantation.

### BRIEF DESCRIPTION OF THE DRAWINGS

Figure 1 is a plot of a supernatant solution obtained from cultured NC37 lymphoblastoid cells applied to a Nu-Gel P-SP column showing the protein fraction containing TGF activity being eluted with a salt gradient.
Figure 2 is a plot of the material containing TGF activity obtained from the separation shown in Figure 1 as it was being eluted with a salt gradient through a Blue-B-Agarose Column.
Figure 3 shows the plot of the material containing TGF activity obtained from the separation shown in Figure 2 as it was being eluted with a NaCl gradient through a Mono Q column.
Figure 4 shows a SDS-polyacrylamide gel electrophoresis (SDS-PAGE) analysis of the fractions 30 to 45, 48 and 50 obtained from the step illustrated in Figure 3. The numbers on the left side, i.e. 44 and 68, refer to the apparent molecular weight of standard proteins of 44 and 68 kDa in lane S.
Figure 5 shows the elution profile through a Vydac Diphenyl column of fraction 38 from the Mono Q Chromatography separation (reversed-phase HPLC) shown in Figure 3.
Figure 6 shows SDS-PAGE analysis of protein purity of the protein fractions 85-90 recovered from the separation process depicted in Figure 5.
Figure 7 shows a SDS-PAGE analysis of fractions 87 and 88 from the reversed-phase HPLC separation under non-reducing (lane A; without β-mercaptoethanol) and reducing (lane B; in the presence of β-mercaptoethanol) conditions showing the 75,000 molecular weight CLMF separated into two subunits of 40 kDa and 35 kDa. The remaining lanes in the gel shown in this Figure contain standard proteins comprising the 44 and 68 kDa marker protein.
Figure 8 shows the elution pattern of the proteins from the supernatant solution from NC-37 cells applied to a Nu-Gel P-SP column and eluted with a salt gradient.
Figure 9 is a Blue-B-Agarose column salt gradient elution profile of the active fractions obtained from the Nu-Gel P-SP column elution shown in Figure 8.
Figure 10 is a Mono-Q column salt gradient elution profile of the active fractions obtained from the elution shown in Figure 9.
Figure 11 is the elution pattern through a Vydac Diphenyl column of active fractions 39 and 40 obtained from the Mono Q Chromatography shown in Figure 10.
Figure 12 shows a SDS-PAGE analysis under reducing conditions of the active fractions obtained from the separation process shown in Figure 11.
Figure 13 is a schematic diagram depicting the separation of the 40 kDa subunit from the 35 kDa subunit of the CLMF cytokine.
Figure 14 is a schematic diagram depicting the determination of the amino acid composition, the N-terminal sequencing, the proteolytic digestion and the complete sequencing of the 40 kDa subunit of the CLMF cytokine.
Figure 15 shows a separation of the tryptic peptides of the digested 40 kDa subunit of the CLMF cytokine.
Figure 16 shows a separation of the proteolytic peptides of the Staphylococcus aureus V8 protease digested 40 kDa subunit CLMF.
Figure 17 is a chart which summarizes the information on the protein structure obtained from the analysis of the proteolytic peptides of the 40 kDa subunit of CLMF. The following abbreviations and symbols are used:
   N-t - N-terminal sequencing on intact protein
   Tr - tryptic peptides from map HP2383 numbered by fraction number
   V8 - V8 protease peptides from map HP2412 numbered by fraction number
   ^ - indicates probable glycosylation site; boxes indicate potential sites
Figure 18 shows the SDS-PAGE analysis of Fraction 39 from the Mono Q FPLC elution profile shown in Figure 3. Lane A: Standardproteins without β-mercaptoethanol; lane B: Fraction 39 without β-mercaptoethanol; lane C: Fraction 39 with β-mercaptoethanol; lane D: Standard proteins with β-mercaptoethanol.
Figure 19 relates to the purification of the 35 kDa subunit by reversed-phase HPLC and depicts the elution pattern through a Vydac C-18 column of fraction 39 of the Mono Q chromatography which was reduced in 5% β-mercaptoethanol.
Figure 20 shows a SDS-PAGE gel analysis under non-reducing conditions of the fractions which were fluorescamine positive from the Vydac C-18 column elution profile shown in Figure 19. S: = protein-standard; F: = flow-through; numbers refer to the fraction number.
Figure 21 depicts the elution pattern of a tryptic digest of fractions 36 and 37 of the Mono Q Chromatography through a YMC ODS column.
Figure 22 shows the stained PVDF membrane with the smeared bands comprising the CNBr cleaved CLMF before (Fig. 22B) and after (Fig. 22A) excising the regions of about 29, 25. 14, 12, and 9 kDa, respectively. The regiones contain the CNBr fragments having the following sequences:
   I (P?)-P-K-N-L-Q-L-K-P-L-K-N-?-V-(Q?)-
      (New sequence from 40 kDa protein)
   II ?-Q-K-A-(R?)-Q-T-L-E-F-Y-P-?-T-
      (New sequence starting at residue no. 30 of 35 kDa protein)
   III V-V-L-T-?-D-T-P-E-E-D-G-I-T-
      (Starts at residue no. 24 of 40 kDa protein)
   IV V-D-A-V-(H?)-K-L-K-Y-E-?-Y-T-?-?-F-F-I-
      (Starts at residue no. 190 of 40 kDa protein)
   note: it is assumed or known that the above sequences
      are preceeded by a Met residue.

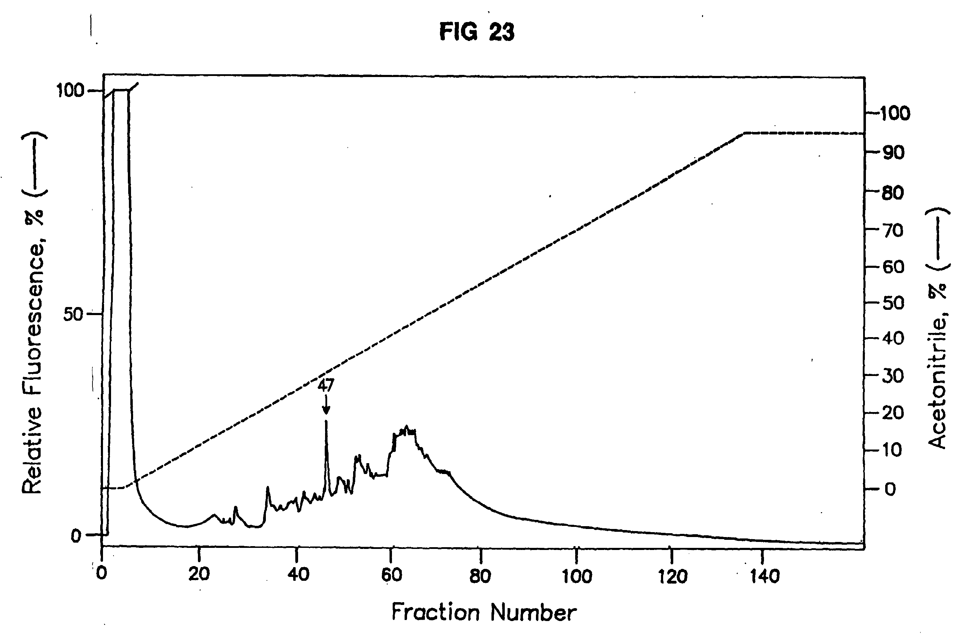

Figure 23 shows a reverse-phase HPLC separation of the peptide fragments obtained by cleaving CLMF with CNBr.

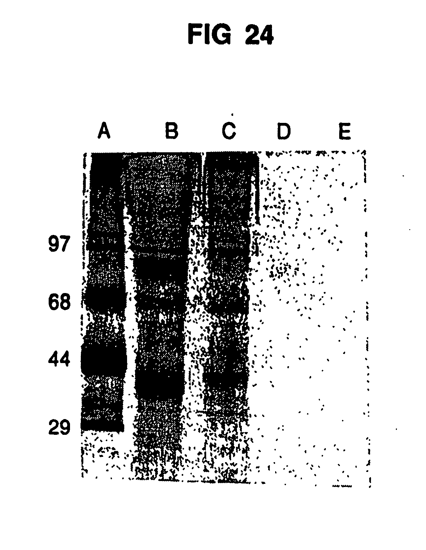

Figure 24 shows an SDS-PAGE of pure CLMF and "free" unassociated 40 kDa subunit of CLMF purified by affinity chromatography using the monoclonal antibody 7B2 covalently attached to an agarose resin. Lane A: molecular weight marker proteins; lane B: starting material; lane C: flow-through; lane D: acid eluate; lane E: potassium thiocyanate eluate.

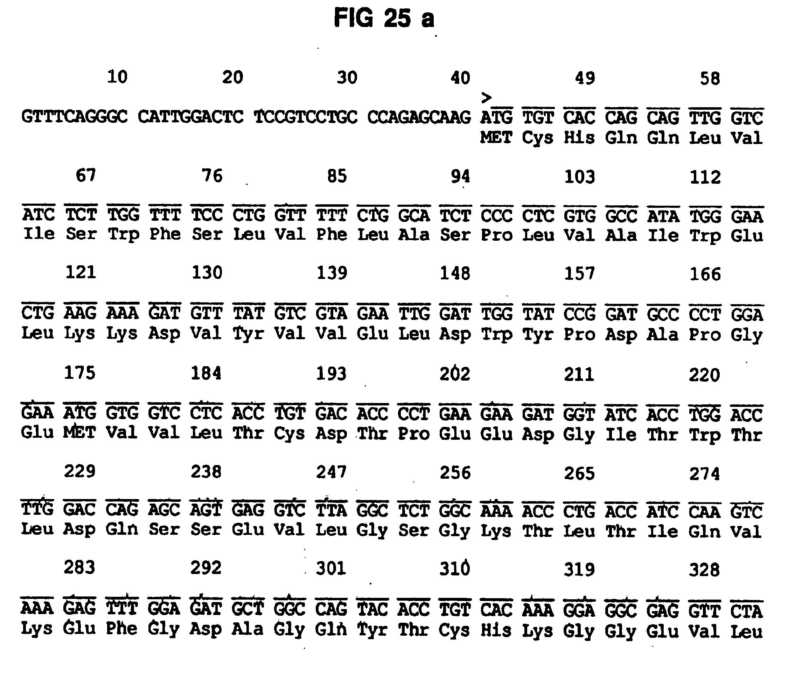

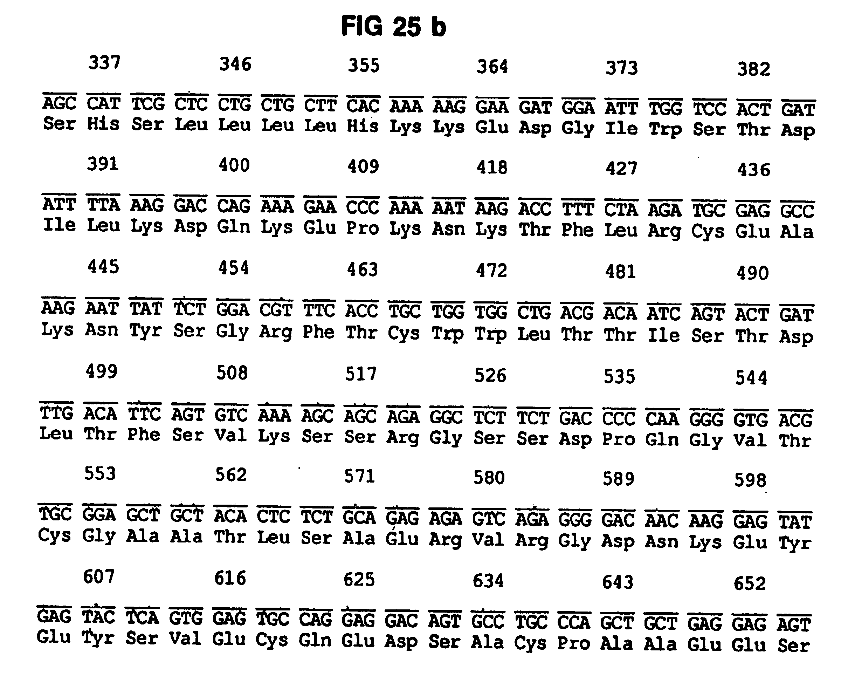

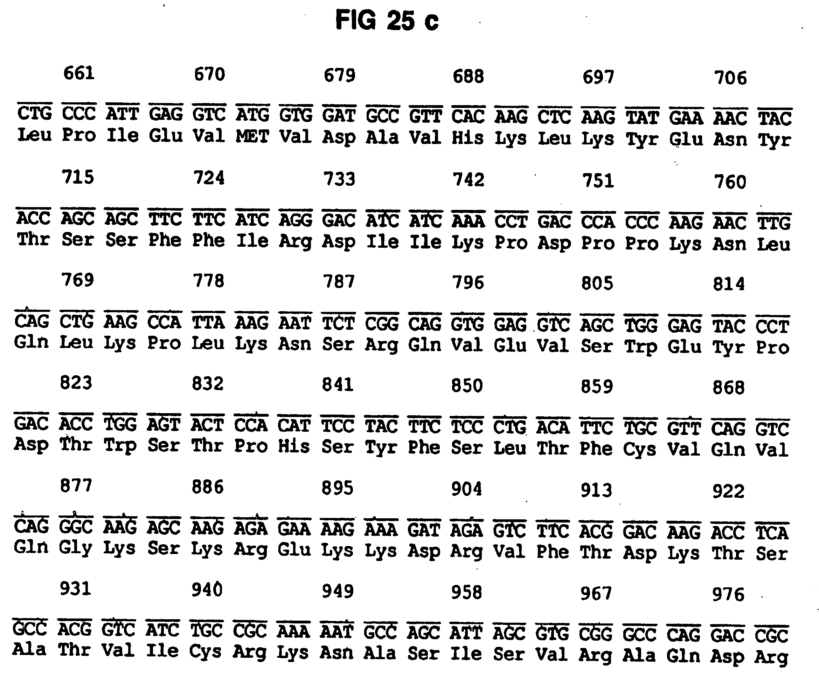

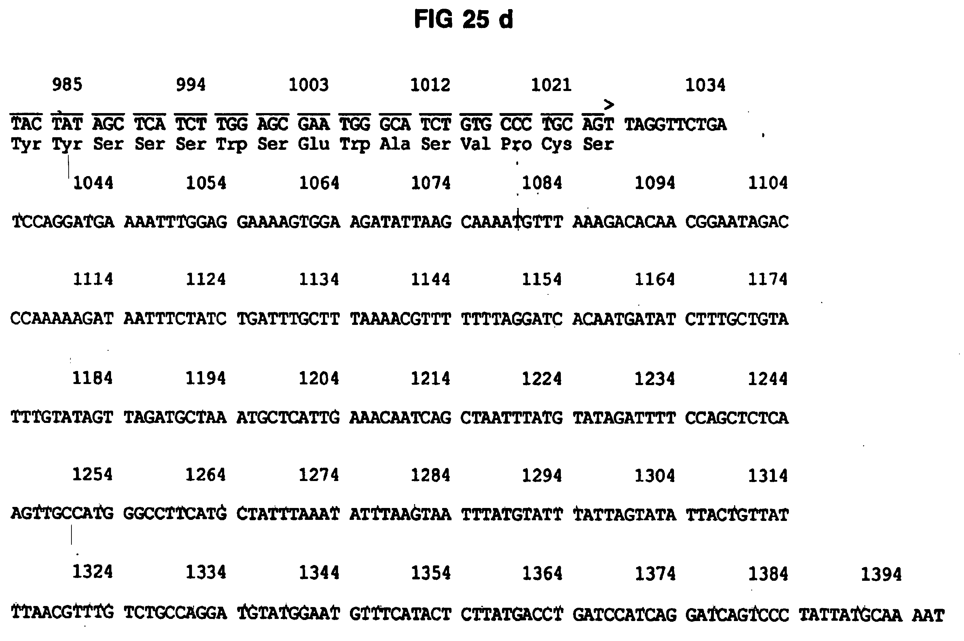

Figure 25 a, b, c and d show the DNA sequence and the deduced amino acid sequence of the 40 kDa subunit of human CLMF.

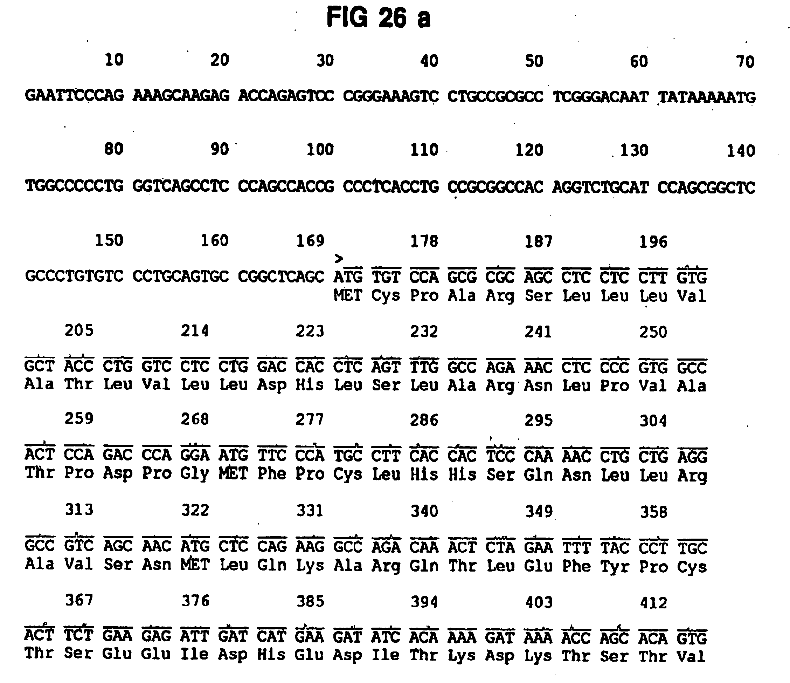

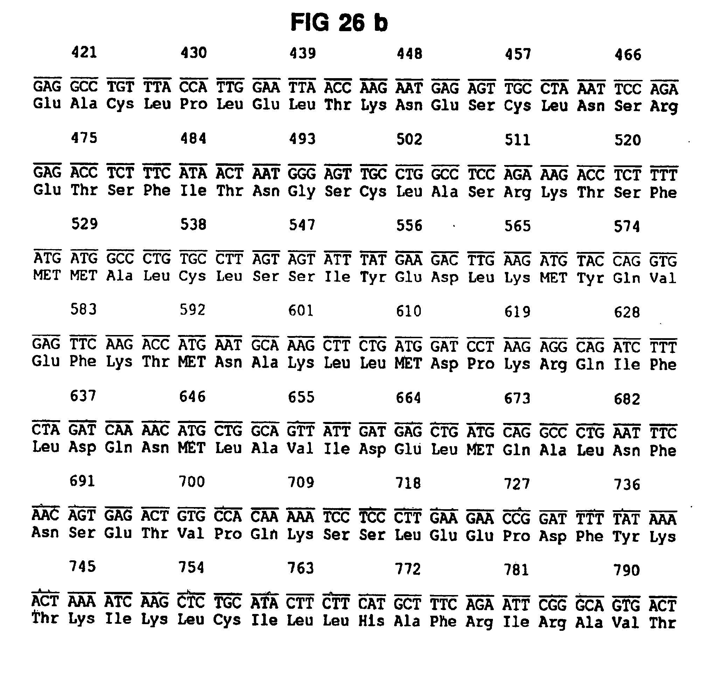

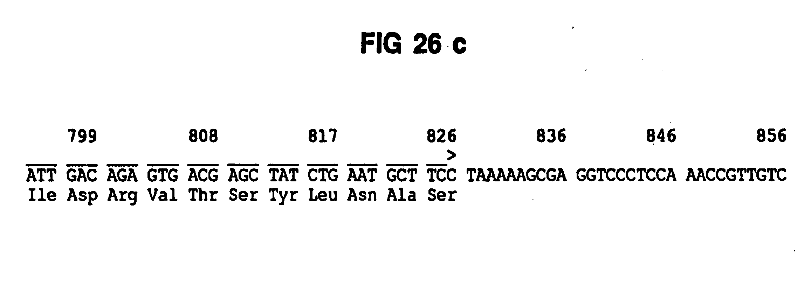

Figure 26 a, b and c show the cDNA sequence and the deduced amino acid sequence of the 35 kDa subunit of CLMF .

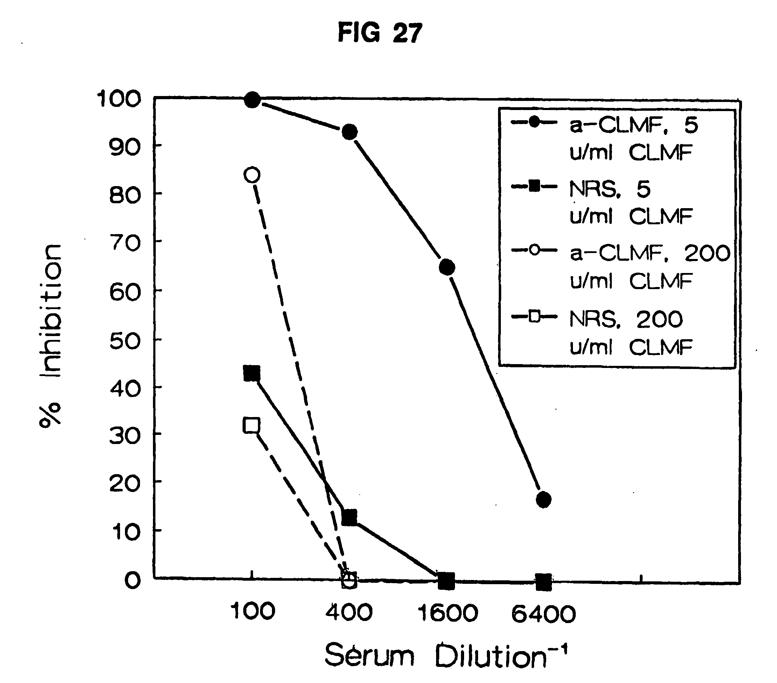

Figure 27 depicts the inhibition of CLMF bioactivity by serum from rats immunized with CLMF and from non-immunized rats (control).

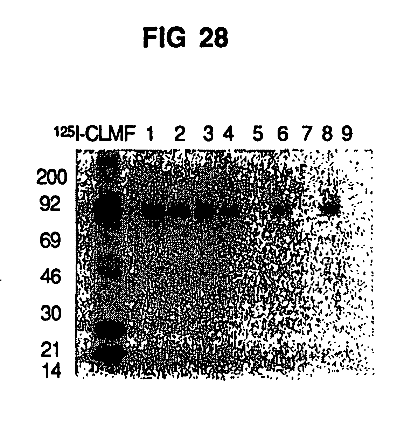

Figure 28 shows a SDS-PAGE analysis of immunoprecipitates of ¹²⁵I-CLMF by monoclonal antibodies 4A1 (lane 1), 4D1 (lane 2), 8E3 (lane 3) and 9C8 (lane 4), by a control antibody (lane 5), by immune rat serum (lanes 6 and 8) and by normal rat serum (lanes 7 and 9). On the left side the molecular weight in kDa is indicated.

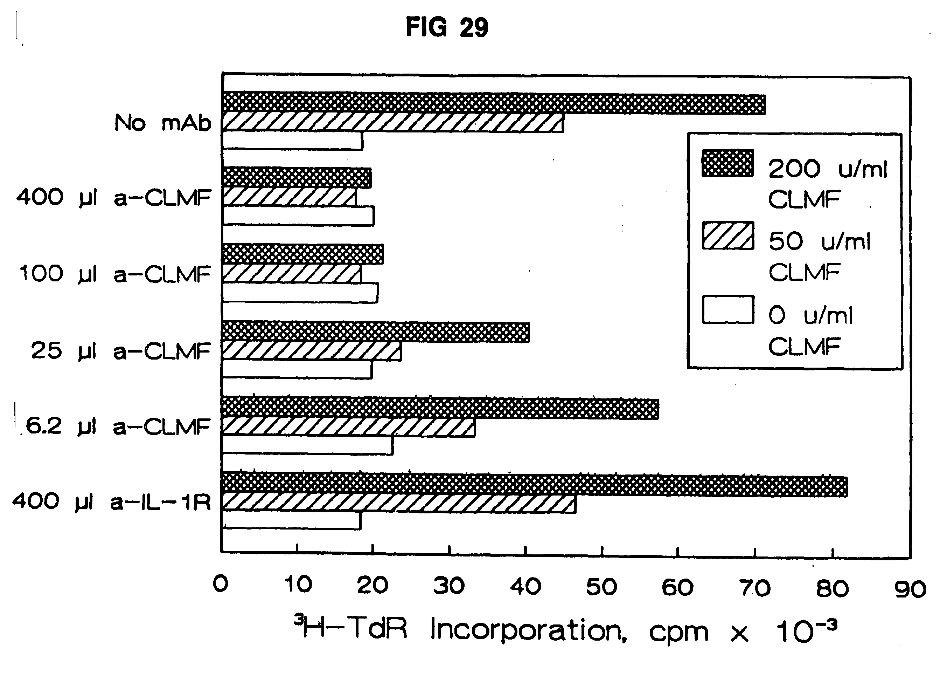

Figure 29 shows the immunodepletion of CLMF bioactivity (TGF activity) by monoclonal anti-CLMF antibodies (a-CLMF).

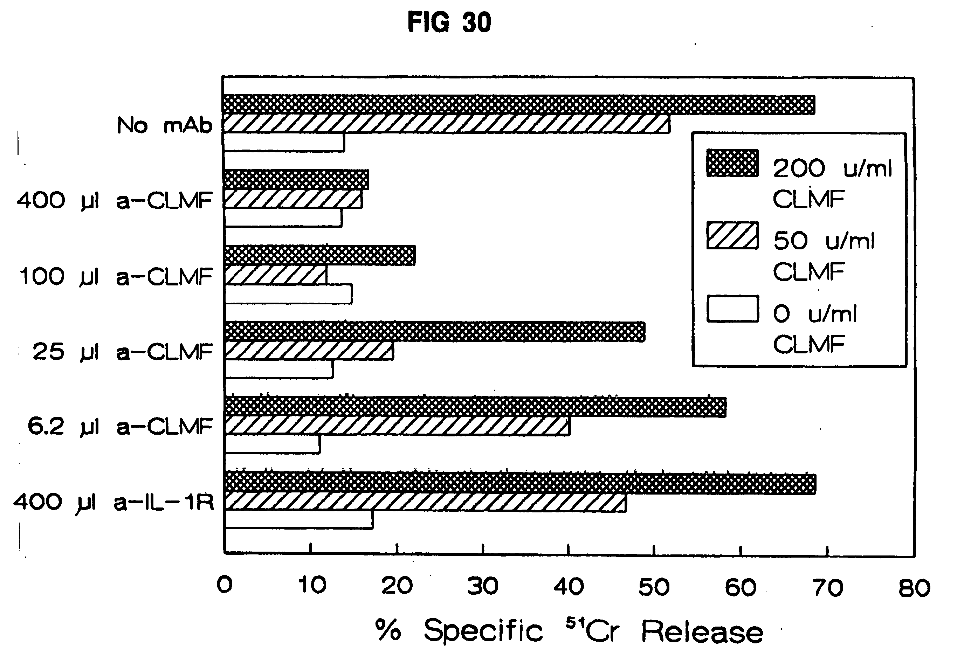

Figure 30 shows the immunodepletion of CLMF bioactivity (LAK induction activity) by monoclonal anti-CLMF antibodies (a-CLMF).

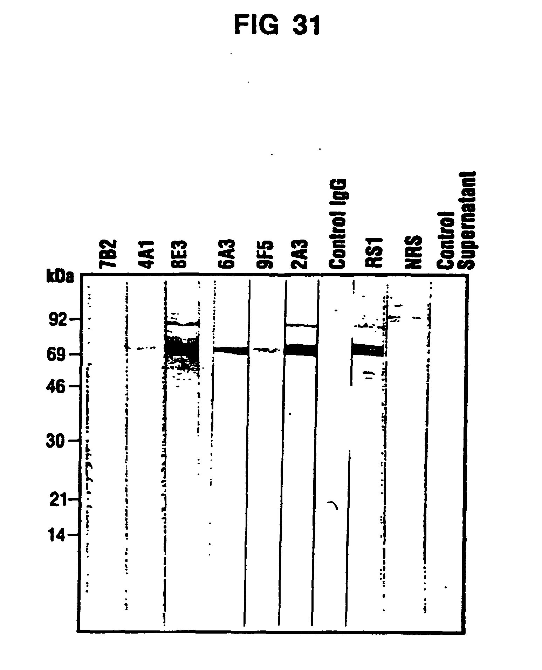

Figure 31 shows a Western blot analysis of the reactivity of the monoclonal antibodies (mAbs) 7B2, 4A1, 8E3, 6A3, 9F5 and 2A3 and of rat polyclonal anti-CLMF antibodies (RS1) with the CLMF 75 kDa heterodimer. NRS: = normal rat serum.

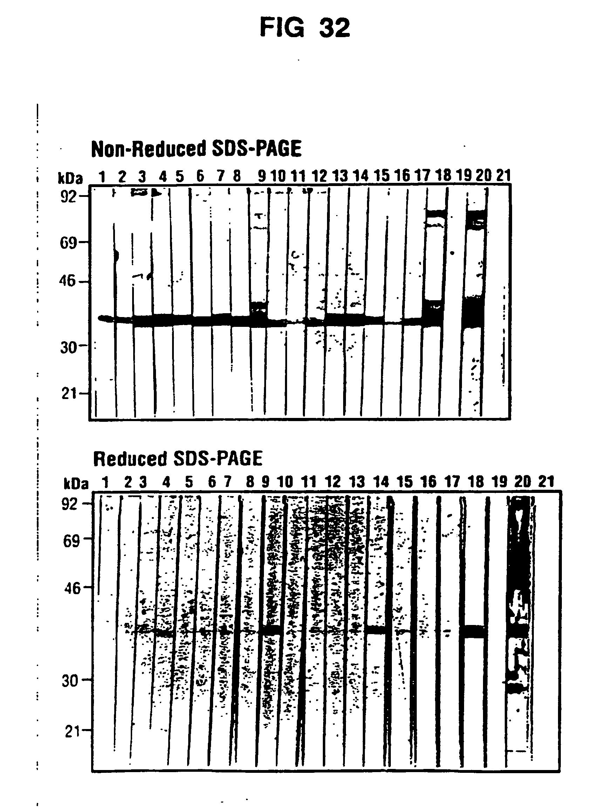

Figure 32 shows a Western blot analysis of the reactivity of monoclonal and rat polyclonal anti-CLMF antibodies with the CLMF 40 kDa subunit. In lanes 1 to 18 the following mAbs were used: 4A1, 4D1, 7B2, 7A1, 2A3, 1C1, 8E4, 8A2, 8E3, 1B8, 4A6, 6A2, 8C4, 9F5, 6A3, 9C8, 8A1 and 22E7, respectively. In lane 19 a control antibody, in lane 20 a fusion rat serum and in lane 21 a normal rat serum was used.

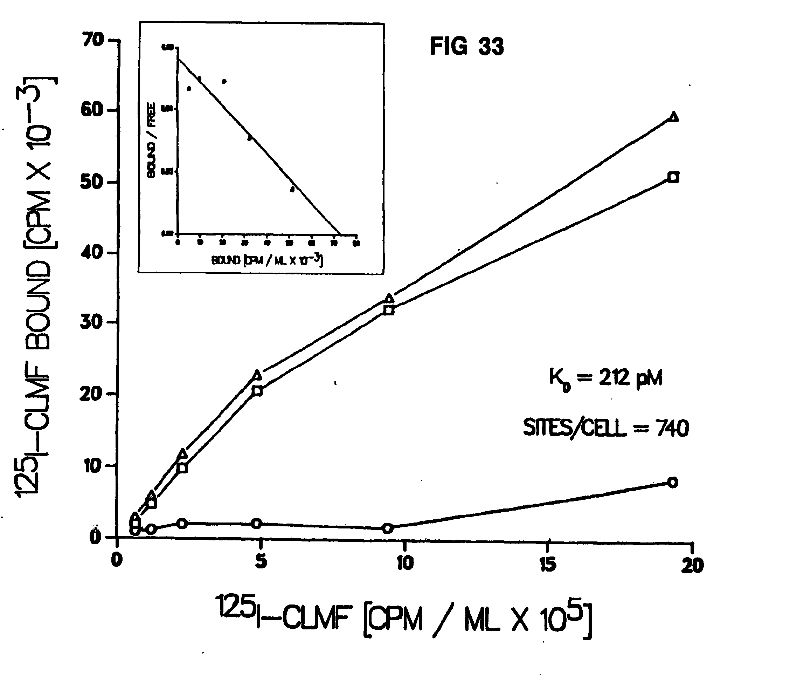

Figure 33 shows the binding of ¹²⁵I-CLMF to PHA-activated peripheral blood lymphocyte (PBL) lymphoblasts.

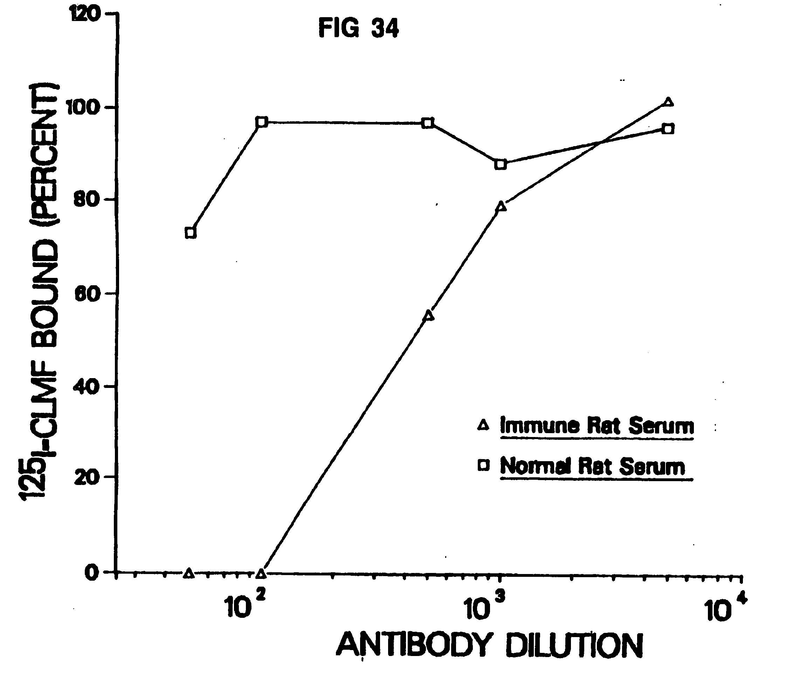

Figure 34 shows the inhibition of ¹²⁵I-CLMF binding to PHA-activated PBL blast cells by rat anti-CLMF serum. The data are expressed as amount (% bound) of ¹²⁵I-CLMF binding to the cells in the presence of the indicated concentrations of serum when compared to the total specific binding in the absence of serum.

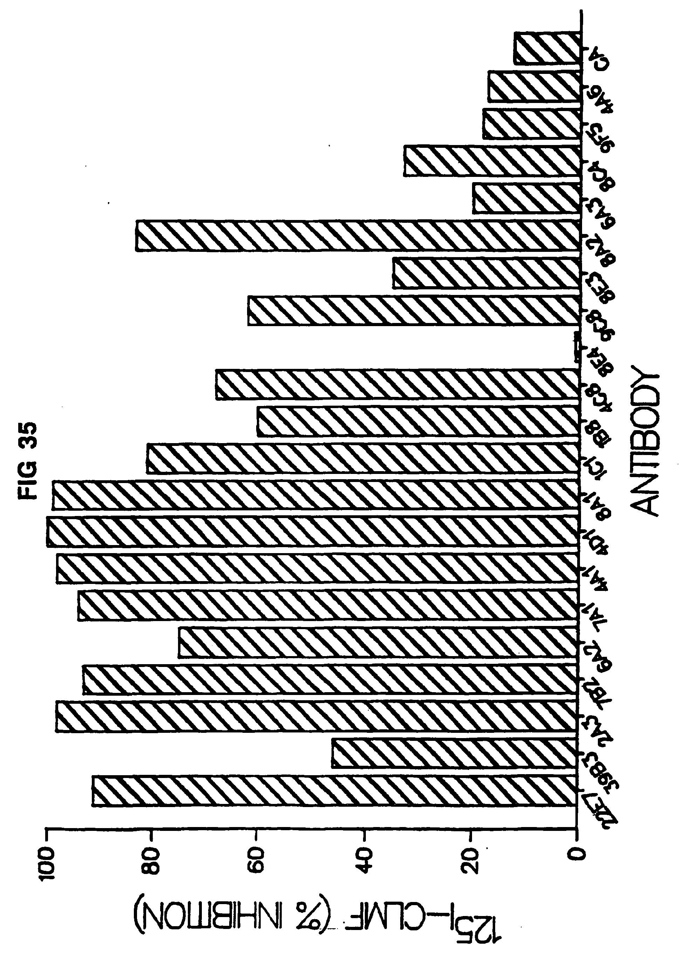

Figure 35 shows the inhibition of the binding of ¹²⁵I-CLMF to PHA-activated PBL blast cells by monoclonal antibody supernatants. The data are expressed as % inhibition of the binding of ¹²⁵I-CLMF to the cells in the presence of a 1:1 dilution of supernatant when compared to the total specific binding in the absence of antibody supernatant.

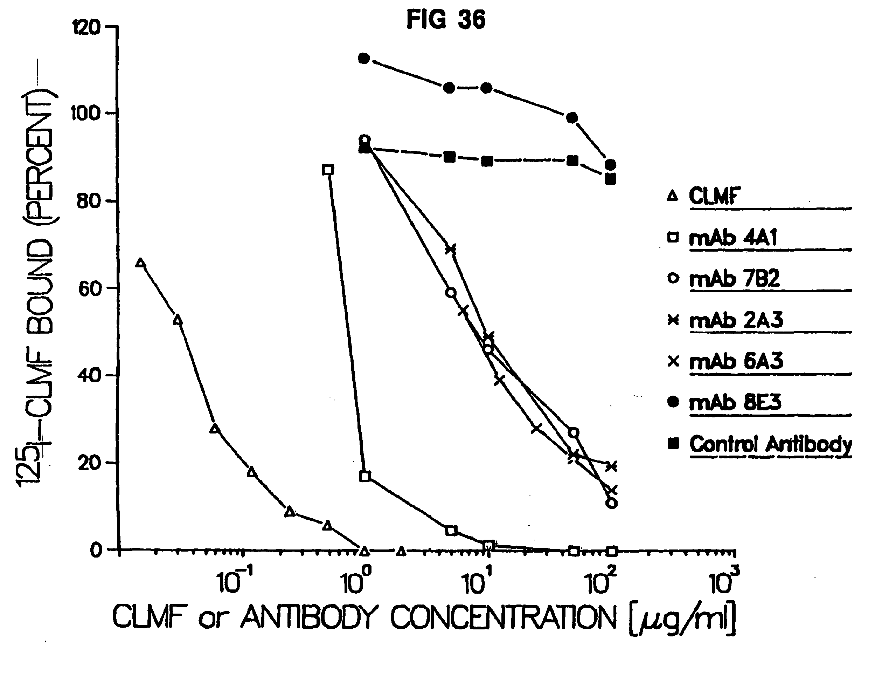

Figure 36 shows the inhibition of the binding of ¹²⁵I-CLMF to PHA-activated PBL blast cells by various concentrations of purified monoclonal antibodies. The data are expressed as the amount (% cpm bound) of ¹²⁵I-CLMF bound to the cells in the presence of the indicated concentrations of antibody when compared to the total specific binding in the absence of antibody.

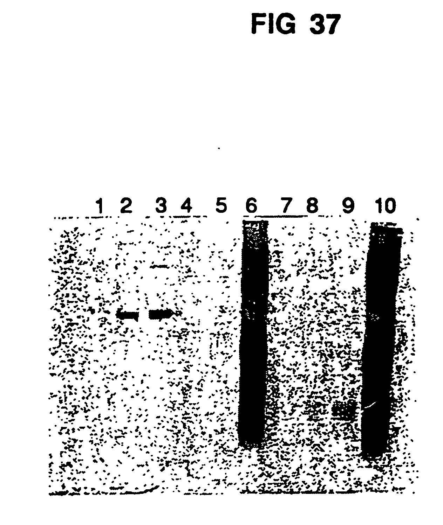

Figure 37 shows a Western blot analysis of the reactivity of a rabbit polyclonal anti-CLMF antibody with the 75 kDa CLMF (nonreduced) and with the 35 kDa CLMF subunit (reduced). The antibody was prepared against a synthetic peptide fragment of the 35 kDa CLMF subunit. Lanes 1 to 5 are without β-mercaptoethanol; lanes 6 to 10 with β-mercaptoethanol.

### Lane

1 1 µl CLMF
2 3 µl CLMF
3 6 µl CLMF
4 Blank
5 Blank
6 5 µl prestained molecular weight standards
7 1 µl CLMF
8 3 µl CLMF
9 6 µl CLMF
10 10 µl prestained molecular weight standards

The CLMF biological activity of all of the proteins of the present invention including the fragments and analogues may be determined by using a standard T-cell growth factor assay.

In accordance with the present invention, natural CLMF is obtained in pure form. The amino acid sequences of the 35 kDa subunit and the 40 kDa subunit of the CLMF protein is depicted in Figures 25 and 26. Based on these sequences,

Thus, the present invention relates to a protein having Cytotoxic Lymphocyte Maturation Factor (CLMF) activity in a substantially pure form, such as the CLMF protein per se, or to a 35 kDa subunit the said protein which exhibits CLMF activity if combined with the 40 kDa subunit and comprises at least a part of the amino acid sequence of the natural form of CLMF.

The present invention also relates to cloned genes coding for a 35 kDa subunit of CLMF and to isolated polynucleotides encoding a subunit as defined above, which polynucleotide contains a sequence corresponding to the cDNA encoding a 35 kDa subunit of CLMF, to recombinant vectors comprising a polynucleotide encoding a 35 kDa subunit of the CLMF protein, to microorganisms transformed with the said recombinant vectors, to antibodies directed to the said subunits as well as to processes for the preparation of the said subunits vectors and antibodies.

The practice of the present invention will employ, unless otherwise indicated, conventional techniques of molecular biology, microbiology, recombinant DNA and immunology, which are within the skills of an artisan in the field. Such techniques are explained fully in the literature. See e.g., Maniatis, Fitsch & Sambrook, MOLECULAR CLONING; A LABORATORY MANUAL (1982); DNA CLONING, VOLUMES I AND II (D.N Glover ed., 1985); OLIGONUCLEOTIDE SYNTHESIS (M.J. Gait ed., 1984); NUCLEIC ACID HYBRIDIZATION (B.D. Hames & S.J. Higgins eds., 1984); TRANSCRIPTION AND TRANSLATION (B.D. Harnes & S.J. Higgins eds., 1984); ANIMAL CELL CULTURE (R.I. Freshney ed., 1986); IMMOBILIZED CELLS AND ENZYMES (IRL Press, 1986); B. Perbal, A PRACTICAL GUIDE TO MOLECULAR CLONING (1984); the series, METHODS IN ENZYMOLOGY (Academic Press, Inc.); GENE TRANSFER VECTORS FOR MAMMALIAN CELLS (J.H. Miller and M.P. Calos eds., 1987, Cold Spring Harbor Laboratory), Methods in Enzymology Vol. 154 and Vol. 155 (Wu and Grossman, and Wu, eds., respectively); IMMUNOCHEMICAL METHODS IN CELL AND MOLECULAR BIOLOGY (Mayer and Walker, eds., 1987, Academic Press, London), Scopes, PROTEIN PURIFICATION: PRINCIPLES AND PRACTICE, second Edition (1987, Springer-Verlag, N.Y.), and HANDBOOK OF EXPERIMENTAL IMMUNOLOGY, VOLUMES I-IV (D.M. Weir and C.C. Blackwell eds., 1986).

The DNA sequences and DNA molecules of the present invention may be expressed using a wide variety of host/vector combinations. For example, useful vectors may consist of segments of chromosomal, non-chromosomal and synthetic DNA sequences. Examples of such vectors are viral vectors, such as the various known derivatives of SV40, bacterial vectors, such as plasmids from E. coli including pCR1, pBR322, pMB9 and RP4, phage DNAs, such as the numerous derivatives of phage λ, M13 and other filamentous single-stranded DNA phages, as well as vectors useful in yeasts, such as the 2µ plasmid, vectors useful in eukaryotic cells more preferably vectors useful in animal cells, such as those containing SV40, adenovirus and/or retrovirus derived DNA sequences. Useful vectors may be also derived from combinations of plasmids and phage DNA's, such as plasmids which have been modified to comprise phage DNA or other derivatives thereof.

Expression vectors which may be used for the preparation of recombinant 35 kDa CLMF subunits are characterized by comprising at least one expression control sequence which is operatively linked to the 35 kDa CLMF subunit DNA sequence inserted in the vector in order to control and to regulate the expression of the cloned 35 kDa CLMF subunit DNA sequence. Examples of useful expression control sequences are the lac system, the trp system, the tac system, the trc system, major operator and promoter regions of phage λ, the control region of fd coat protein, the glycolytic promoters of yeast, e.g., the promoter for 3-phosphoglycerate kinase, the promoters of yeast acid phosphatase, e.g., Pho 5, the promoters of the yeast α-mating factors, and promoters derived from polyoma virus, adenovirus, retrovirus, and simian virus, e.g., the early and late promoters or SV40, and other sequences known to control the expression of genes of prokaryotic or eukaryotic cells and of their viruses as well as combinations of the said promoter/operator sequences.

Among such useful expression vectors are known vectors that enable the expression of the cloned CLMF-related DNA sequences in eukaryotic hosts, such as in animal and human cells [e.g., P. J. Southern and P. Berg, J. Mol. Appl. Genet. 1: 327-41 (1982); S. Subramani et al., Mol. Cell. Biol. 1: 854-64 (1981); R. J. Kaufmann and P. A. Sharp, Mol. Cell. Biol. 159: 601-64 (1982); S. I. Scahill et al., "Expression and Characterization of The Product Of A Human Immune Interferon DNA Gene in Chinese Hamster Ovary Cells", Proc. Natl. Acad. Sci. U.S.A. 80: 4654-59 (1983); G. Urlaub and L. A. Chasin, Proc. Natl. Acad. Sci. USA 77: 4216-20 (1989)].

Furthermore, within each specific expression vector, various sites may be selected for insertion of the CLMF-related DNA sequences of the present invention. These sites are usually designated by the restriction endonuclease which cut them. They are well recognized by those of skill in the art. It is, of course to be understood that an expression vector useful in this invention need not have a restriction endonuclease site for insertion of the chosen DNA fragment. Instead, the vector could be joined to the fragment by alternative means. The site chosen in the expression vector for the insertion of a selected DNA fragment and the operative linking of the DNA fragment to an expression control sequence is determined by a variety of factors, such as the number of sites susceptible to a particular restriction enzyme, the location of start and stop codons relative to the vector sequence and the desired selection method for the host transformed with the recombinant vector. The choice of a vector and an insertion site for a DNA sequence is determined by a balance of these factors, not all selections being equally effective for a given case.

The host cell used for the expression of the CLMF-related DNA sequence may be selected from a variety of known hosts. Examples for such hosts are prokaryotic or eukaryotic cells. A large number of such hosts ace available from various depositories such as the American Type Culture Collection (ATCC) or the Deutsche Sammlung für Mikroorganismen (DSM). Examples for prokaryotic cellular hosts are bacterial strains such as E.coli, B.subtilis and others. Preferred hosts are mammalian cells such as the SV40 transformed African Green monkey kidney cell line COS.

Not all host/expression vector combinations function with equal efficiency in expressing a given DNA sequence. However, a particular selection of a host/expression vector combination may be made by those of skill in the art after due consideration of the principles set forth herein without departing from the scope of this invention. For example, the selection should be based on a balancing of a number of factors, These include, for example, compatibility of the host and vector, susceptibility of the protein to proteolytic degradation by host cell enzymes, possible contamination of the protein to be expressed by host cell proteins difficult to remove during purification, toxicity of the proteins encoded by the DNA sequence to the host, ease of recovery of the desired protein, expression characteristics of the DNA sequence and the expression control sequence operatively linked to them, biosafety, costs and the folding, form or any other necessary post-expression modifications of the desired protein.

The host organisms which contain the expression vector comprising the 35 kDa CLMF subunit DNA are usually grown up under conditions which are optimal for the growth of the host organism. Towards the end of the exponential growth, when the increase in the number of cells per unit time decreases, the expression of the CLMF subunit is induced, i.e. the DNA coding for the subunit is transcribed and the transcribed mRNA is translated. The induction can be effected by adding an inducer or a derepressor to the growth medium or by altering a physical parameter, e.g. by a temperature change.

The CLMF subunit produced in the host organism can be secreted by the cell by special transport mechanisms or can be isolated by breaking open the cell. The cell can be broken open by mechanical means [Charm et al., Meth. Enzmol. 22: 476-556 (1971)], by enzymatic treatment (e.g. lysozyme treatment) or by chemical means (e.g. detergent treatment, urea or guanidine·HCl treatment, etc.) or by a combination thereof.

In eukaryotes, polypeptides which are secreted from the cell are synthesized in the form of a precursor molecule. The mature polypeptide results by cleaving off the so-called signal peptide. As prokaryotic host organisms are not capable of cleaving eukaryotic signal peptides from precursor molecules, eukaryotic polypeptides must be expressed directly in their mature form in prokaryotic host organisms. The translation start signal AUG, which corresponds to the codon ATG on the level of the DNA, causes that all polypeptides are synthesized in a prokaryotic host organism with a methionine residue at the N-terminus. In certain cases, depending on the expression system used and possibly depending on the polypeptide to be expressed this N-terminal methionine residue is cleaved off.

The 35 kDa CLMF subunit produced by fermentation of the prokaryotic and eukaryotic hosts transformed with the DNA sequences of this invention can then be purified to essential homogeneity by known methods such as, for example, by centrifugation at different velocities, by precipitation with ammonium sulphate, by dialysis (at normal pressure or at reduced pressure), by preparative isoelectric focusing, by preparative gel electrophoresis or by various chromatographic methods such as gel filtration, high performance liquid chromatography (HPLC), ion exchange chromatography, reverse phase chromatography and affinity chromatography (e.g. on Sepharose^{™} Blue CL-6B or on carrier-bound monoclonal antibodies directed against CLMF).

The purified CLMF subunit of the present invention can be employed for the preparation of LAK cell and T cell activator and antitumor compositions and in methods for stimulating LAK cell, T-cells or Natural Killer Cells.

The 35 kDa CLMF subunit of the present invention can also be analyzed to determine the active sites for CLMF activity. The information from this analysis may be used to predict and produce fragments or peptides, including synthetic peptides, having the activity of CLMF. Among the known techniques for determining such active sites are x-ray crystallography, nuclear magnetic resonance, circular dichroism, UV spectroscopy and site specific mutagenesis. Accordingly, the fragments obtained in this way may be employed in methods for stimulating T-cells or LAK cells.

The CLMF subunits prepared in accordance with this invention or pharmaceutical compositions comprising the 35 kDa CLMF subunit may be administered to warm blooded mammals for the clinical uses indicated above. The administration may be by any conventional modes of administration of agents which exhibit antitumor activity auch as by intralesional or parenteral application either intravenously, subcutaneously or intramuscularly. Obviously, the required dosage will vary with the particular condition being treated, the severity of the condition, the duration of the treatment and the method for administration. A suitable dosage form for pharmaceutical use may be obtained from sterile filtered, lyophilized protein reconstituted prior to use in a conventional manner. It is also within the skill of the artisan in the field to prepare pharmaceutical compositions comprising 35 kDa CLMF subunit of the present invention by mixing the said CLMF subunit with compatible pharmaceutically acceptable carrier materials such as buffers, stabilizers, bacteriostats and other excipients and additives conventionally employed in pharmaceutical parenteral dosage forms. The present invention also relates to such pharmaceutical compositions.

The preferred form of administration depends on the intended mode of administration and therapeutic application. The pharmaceutical compositions comprising a CLMF protein or peptide derivative of the present invention also will preferably include conventional pharmaceutically acceptable carriers and may include other medicinal agents (e.g. interleukin-2), carriers, adjuvants, excipients, etc., e.g., human serum albumin or plasma preparations. Preferably, the compositions of the invention are in the form of a unit dose and will usually be administered one or more times a day. The unit dose is preferably packed in 1 ml vials containing an effective amount of the 35 kDa CLMF subunit and if desired of interleukin-2 in lyophilized form. The vials containing the CLMF subunit and if desired the interleukin-2 are preferably packed in a container together with written instructions describing the correct use of the pharmaceutical composition. The present invention relates also to such a unit dose packed in a container, preferably together with a separate unit dose of interleukin-2, most preferably together with the appropriate instructions. Furthermore the present invention relates to a process for the preparation of the said unit dose.

In order that our invention herein described may be more fully understood, the following examples are set forth. It should be understood that these examples are for illustrative purposes only and should not be construed as limiting this invention in any way to the specific embodiments recited therein. It has to be noted that the specific product names and suppliers mentioned below are not meant to be mandatory. The person skilled in the art is in a position to select alternative products from other suppliers.

### EXAMPLE

### PURIFICATION AND CHARACTERIZATION OF CYTOTOXIC LYMPHOCYTE MATURATION FACTOR (CLMF)

### Production of Supernatant Liquid Containing CLMF.

Human NC-37 B lymphoblastoid cells (ATCC CCL 214, American Type Culture Collection, Rockville, MD) were used for production of CLMF. These cells were maintained by serial passage in RPMI 1640 medium supplemented with 5% heat-inactivated (56°C, 30 min.) fetal bovine serum, 2 mM L-glutamine, 100 units/ml penicillin, and 100 µg/ml streptomycin (all cell culture media were from GIBCO Laboratories, Grand Island, NY).

Higher producer sublines of NC-37 cells were derived by limiting dilution cloning in liquid microcultures. Each well of three Costar 3596 microplates (Costar Co., Cambridge. MA) received 100 µl of a cell suspension containing five NC-37 cells/ml. The medium used for the cloning was a 1:1 mixture of fresh passage medium and filtered, conditioned medium from stock cultures of the parent NC-37 cells. One week and two weeks after culture initiation each of the microcultures was fed with 50 µl of the 1:1 mix of fresh and conditioned medium. Between 3 and 4 weeks after culture initiation the contents of wells containing clones of NC-37 cells were harvested and passed into larger cultures.

When the number of cells in a given subline exceeded 1.4 x 10⁶, one million cells were stimulated to produce CLMF in 1 ml cultures containing 3 ng/ml phorbol 12-myristate 13-acetate (PMA) (Sigma Chemical Co., St. Louis, MO) and 100 ng/ml calcium ionophore A23187 (Sigma). Supernatants were harvested from the cultures after 2 days, dialyzed against about 50 volumes of Dulbecco's phosphate buffered saline (Gibco) using e.g. SPECTROPOR^{®} #1 tubing (Fisher Scientific) overnight with one change of buffer and then for 4 hours against 50 volumes of RPMI 1640 medium with 50 µg/ml of gentamicin (both from Gibco) and tested for CLMF by means of the T cell growth factor assay (see below). Three sublines, NC-37.89, NC-37.98, and NC-37.102, were identified which routinely produced CLMF at titers ≥ 4 times the titers produced by the parental NC-37 cell line. Since cells from these three sublines produced CLMF at similar titers (≥ 800 units/ml), culture supernatants derived from the three sublines were pooled for use as starting material for the purification of CLMF.

Bulk production of CLMF was carried out in roller bottle cultures on a roller apparatus set at about 38 rpms (Wheaton Cell Production Roller Apparatus Model II, Wheaton Instruments, Millville, NJ). Cell suspensions were prepared containing 1-1.5 x 10⁶ NC-37.89, NC-37.98 or NC-37.102 cells/ml in RPMI 1640 medium supplemented with 1% Nutridoma-SP (Boehringer Mannheim Biochemicals, Indianapolis, IN), 2 mM L-glutamine, 100 units/ml penicillin, 100 µg/ml streptomycin, 10 ng/ml PMA and 20-25 ng/ml calcium ionophore A23187. Two hundred fifty to three hundred fifty ml aliquots of the cell suspensions were added to Falcon 3027 tissue culture roller bottles (Becton Dickinson, Lincoln Park, NJ) which had been gashed with a mixture of 5% CO₂, 95% air. The roller bottles were then capped tightly and incubated at 37°C with continuous rolling for three days. At the end of this time, the culture supernatants were harvested. EDTA and phenylmethylsulfonyl fluoride (both from Boehringer Mannheim) were added to the culture supernatants at final concentrations of 1 mM and 0.1 mM, respectively, to retard proteolytic degradation. The supernatants were stored at 4°C.

### Lympokine Activated Killer (LAK) Cell Induction (LCI) Assay.

Culture supernatants and chromatographic fractions were tested for their ability to synergize with rIL-2 to induce the generation of cytolytic LAK cells as follows. Human peripheral blood mononuclear cells (PBMC) were isolated by the following method. Blood from normal volunteer donors was drawn into syringes containing sufficient sterile preservative-free heparin (Sigma) to give a final concentration of approximately 5 units/ml. The blood was diluted 1:1 with Hanks' balanced salt solution (HBSS) without calcium or magnesium (GIBCO). The diluted blood was then layered over 15 ml aliquots of Ficoll/sodium diatrizoate solution (Lymphocyte Separation Medium, Organon Teknika Corp., Durham, NC) in 50 ml Falcon 2098 centrifuge tubes. The tubes were centrifuged for 30 minutes at room temperature at 500 x g. Following centrifugation, the cells floating on the Ficoll/sodium diatrizoate layer were collected and diluted by mixing with ≥ 2 volumes of HBSS without calcium or magnesium. The resulting cell suspension was then layered over 15 ml aliquots of 20% sucrose (Fisher) in RPMI 1640 medium with 1% human AB serum (Irvine Scientific, Santa Ana, CA) in Falcon 2098 centrifuge tubes. The tubes were centrifuged for 10 minutes at room temperature at 500 x g, and the supernatant fluids were discarded. The cell pellets were resuspended in 5 ml of HBSS without calcium or magnesium, repelleted by centrifugation, and finally resuspended in the appropriate culture medium. Accessory cells were removed from the PBMC by treatment with 5 mM L-glutamic acid dimethyl ester (Sigma) using the same conditions as described by Thiele et al. J. Immunol. 131:2282-2290 (1983) for accessory cell depletion by L-leucine methyl ester except that the glutamic acid ester was substituted for the leucine ester.

The accessory cell-depleted PBMC were further fractionated by centrifugation on a discontinuous Percoll density gradient (Pharmacia, Piscataway, NJ) as described by Wong et al., Cell Immunol. 111:39-54 (1988). Mononuclear cells recovered from the 38. 41, 45, and 58% Percoll layers were pooled and used as a source of LAK cell precursors in the assay. The cells recovered from the Percoll gradient were washed and suspended in tissue culture medium (TCM) composed of a 1:1 mixture of RPMI 1640 and Dulbecco's modified Eagle's medium, supplemented with 0.1 mM nonessential amino acids, 60 µg/ml arginine HCl, 10 mM HEPES buffer, 2 mM L-glutamine, 100 units/ml penicillin, 100 µg/ml streptomycin (all available from GIBCO), 5 x 10⁻⁵ M 2-mercaptoethanol (Fisher Scientific, Fair Lawn, NJ), 1 mg/ml dextrose (Fisher), and 5% human AB serum (Irvine Scientific, Santa Ana, CA). These cells were incubated in 24-well tissue culture plates (Costar, Cambridge, MA) in 1 ml cultures (7.5 x 10⁵ cells/culture) to which 10⁻⁴ M hydrocortisone sodium succinate (Sigma) was added to minimize endogenous cytokine production. Some cultures also received human rIL-2 (supplied by Hoffmann-La Roche, Inc., Nutley, NJ) at a final concentration of 5 units/ml and/or supernatants to be assayed for CLMF activity. All cultures were incubated for 3-4 days at 37°C in a humidified atmosphere of 5% CO₂, 95% air.

At the end of this incubation, the contents of each culture were harvested, and the cells were pelleted by centrifugation and resuspended in 0.5 ml of fresh TCM. One tenth ml aliquots of these cell suspensions were mixed with 0.1 ml aliquots of ⁵¹Cr-labelled K562 or Raji cells (both cell lines may be obtained from the ATCC) and tested for their lytic activity in 5 hour ⁵¹Cr release assays. The method for labelling target cells with ⁵¹Cr and performing the cytolytic assays have been described by Gately et al., [JNCI 69:1245-1254 (1982)]. The percent specific ⁵¹Cr release was calculated as [(e - c)/(100 - c)] X 100. where e is the percentage of ⁵¹Cr released from target cells incubated with lymphocytes and c is the percentage of ⁵¹Cr released spontaneously from target cells incubated alone. The total releasable ⁵¹Cr was determined by lysis of the target cells with 2% sodium dodecyl sulfate; see Gately et al., JNCI 69:1245-1254 (1982). All lymphocyte populations were assayed in quadruplicate for lytic activity.

LAK Cell Induction Microassay. The microassay for measuring synergy between rIL-2 and CLMF-containing solutions in the induction of human LAK cells was similar to the LAK cell induction assay described above but with the following modifications. Human peripheral blood mononuclear cells which had been depleted of accessory cells and fractionated by Percoll gradient centrifugation as described above were added to the wells of Costar 3596 microplates (5 x 10⁴ cells/well). Some of the wells also received rIL-2 (5 units/ml final concentration) and/or purified CLMF or immunodepleted CLMF-containing solutions. All cultures contained 10⁻⁴ M hydrocortisone sodium succinate (Sigma) and were brought to a total volume of 0.1 ml by addition of TCM with 5% human AB serum. The cultures were incubated for 3 days at 37°C, after which 0.1 ml of ⁵¹Cr-labelled K562 cells (5 x 10⁴ cells/ml in TCM with 5% human AB serum) were added to each well. The cultures were then incubated overnight at 37°C. Following this, the cultures were centrifuged for 5 minutes at 500 x g, and the supernatant solutions were harvested by use of a Skatron supernatant collection system (Skatron, Sterling, VA). The amount of ⁵¹Cr released into each supernatant solution was measured with a gamma counter (Packard, Downer's Grove, IL), and the % specific ⁵¹Cr release was calculated as described above. All samples were assayed in quadruplicate.

### Cytolytic T Lymphocyte (CTL) Generation Assay.

Methods used for generating and measuring the lytic activity of human CTL have been described in detail by Gately et al. in J. Immunol. 136: 1274-1282 (1986) and by Wong et al. in Cell. Immunol. 111: 39-54 (1988). Human peripheral blood mononuclear cells were isolated from the blood of normal volunteer donors, depleted of accessory cells by treatment with L-glutamic acid dimethyl ester, and fractioned by Percoll gradient centrifugation as described above. High density lymphocytes recovered from the interface between the 45% and 58% Percoll layers were used as responder lymphocytes in mixed lymphocyte-tumor cultures (MLTC). CTL were generated in MLTC in 24-well tissue culture plates (Costar #3424) by incubation of Percoll gradient-derived high density lymphocytes (7.5 x 10⁵ culture) together with 1 x 10⁵ uv-irradiated melanoma cells e.g. HT144 (obtainable from ATCC) or with 5 x 10⁴ gamma-irradiated melanoma cells e.g. HT144 in TCM with 5% human AB serum (1.2 ml/culture). For uv-irradiation, HT144 cells were suspended at a density of 1-1.5 x 10⁶ cells/ml in Hanks' balanced salt solution without phenol red (GIBCO) containing 1% human AB serum. One ml aliquots of the cell suspension were added to 35 x 10 mm plastic tissue culture dishes (Falcon #3001), and the cells were then irradiated (960 µW/cm² for 5 min) by use of a 254 nm uv light (model UVG-54 MINERALIGHT® lamp, Ultra-violet Products, Inc., San Gabriel, CA). For gamma irradiation, HT144 cells were suspended at a density of 1-5 x 10⁶ cells/ml in TCM with 5% human AB serum and irradiated (10,000 rad) by use of a cesium source irradiator (model 143, J.L. Shepherd and Associates, San Fernando, CA). Uv- or gamma-irradiated HT144 were centrifuged and resuspended in TCM with 5% human AB serum at the desired cell density for addition to the MLTC. In addition to lymphocytes and melanoma cells, some MLTC received human rIL-2 and/or purified human CLMF at the concentrations indicated. Hydrocortisone sodium succinate (Sigma) was added to the MLTC at a final concentration of 10⁻⁴ M (cultures containing uv-irradiated melanoma cells) or 10⁻⁵ M (cultures containing gamma-irradiated melanoma cells) to supress endogenous cytokine production [S. Gillis et al., J. Immunol. 123: 1624-1631 (1979)] and to reduce the generation of nonspecific LAK cells in the cultures [L.M. Muul and M.K. Gately, J. Immunol. 132: 1202-1207 (1984)]. The cultures were incubated at 37°C in a humidified atmosphere of 5% CO₂ in air for 6 days. At the end of this time, lymphocytes from replicate cultures were pooled, centrifuged, resuspended in 1.2 ml TCM containing 5% human AB serum, and tested for their ability to lyse HT144 melanoma cells, and, as a specificity control, K562 erythroleukemia cells (obtainable from ATCC) in overnight ⁵¹Cr release assays.

Melanoma cells and K562 cells were labeled with ⁵¹Cr sodium chromate as described by Gately et al. [JNCI 69: 1245-1254 (1982)]. Likewise, measurement of lympocyte-mediated lysis of ⁵¹Cr-labeled melanoma cells was performed in a manner identical to that described by Gately et al. (ibid.) for quantitating lysis of glioma target cells. For assaying the lysis of ⁵¹Cr-labeled K562 cells, 0.1 ml aliquots of lymphocyte suspensions were mixed with 25 µl aliquots of ⁵¹Cr-labeled K562 (2 x 10⁵ cells/ml in TCM with 5% human AB serum) in the wells of Costar 3696 "half-area" microtest plates. After overnight incubation at 37°C, the plates were centrifuged for 5 min at 1400 x g, and 50 µl of culture medium was aspirated from each well. The amount of ⁵¹Cr in each sample was measured with a gamma counter (Packard), and the % specific ⁵¹Cr release was calculated as described above. All assays were performed in quadruplicate, and values in the table (see below) represent the means ± 1 S.E.M. of replicate samples.

### T cell growth factor (TGF) assay.

The ability of culture supernatants and chromatographic fractions to stimulate the proliferation of PHA-activated human T lymphoblasts was measured as follows. Human PBMC were isolated by centrifugation over discontinuous Ficoll and sucrose gradients as described above for the LCI assay. The PBMC (5 x 10⁵ cells/ml) were cultured at 37°C in TCM containing 0.1% phytohemagglutinin-P (PHA-P) (Difco Laboratories, Detroit, MI). After 3 days, the cultures were split 1:1 with fresh TCM, and human rIL-2 was added to each culture to give a final concentration of 50 units/ml. The cultures were then incubated for an additional 1 to 2 days, at which time the cells were harvested, washed, and resuspended in TCM at 4 x 10⁵ cells/ml. To this cell suspension was added heat-inactivated goat anti-human rIL-2 antiserum (final dilution: 1/200) to block any potential IL-2-induced cell proliferation in the assay. This antiserum may be prepared using methods well-known in the art or may be obtained from Genzyme Co., Boston, MA. The antiserum used was shown to cause 50% neutralization of 2 units/ml rIL-2 at a serum dilution of 1/20,000.

Fifty µl aliquots of the cell suspension containing anti-IL-2 antiserum were mixed with 50 µl aliquots of serial dilutions of culture supernatants or chromatographic fractions in the wells of Costar 3596 microplates. The cultures were incubated for 1 day at 37°C in a humidified atmosphere of 5% CO₂ in air, and 50 µl of ³H-thymidine (New England Nuclear, Boston, MA), 10 µCi/ml in TCM, were then added to each well. The cultures were further incubated overnight. Subsequently, the culture contents were harvested onto glass fiber filters by means of a cell harvester (Cambridge Technology Inc., Cambridge, MA), and ³H-thymidine incorporation into cellular DNA was measured by liquid scintillation counting. All samples were assayed in triplicate.

In purifying CLMF it was necessary to define units of activity in order to construct chromatographic elution profiles and to calculate the percent recovery of activity and the specific activity of the purified material. To do this, a partially purified preparation of human cytokines produced by coculturing PHA-activated human PBMC with NC-37 cells was used as a standard. The preparation was assigned an arbitrary titer of 2000 units/ml. Several dilutions of this preparation were included in each TGF or LAK induction assay. The results obtained for the standard preparation were used to construct a dose-response curve from which could be interpolated units/ml of activity in each unknown sample at the dilution tested. Multiplication of this value by the dilution factor yielded the activity of the original sample expressed in units/ml.

For antibody neutralization studies, the TGF assay was modified as follows. Twenty-five µl aliquots of CLMF-containing medium were mixed with 50 µl aliquots of serial dilutions of antiserum or antibody solutions in the wells of COSTAR 3596® microplates. The mixtures were incubated for 30 minutes at 37°C, and 25 µl aliquots of a suspension of PHA-activated lymphoblasts (8 x 10⁵/ml in TCM plus 1:100 anti-rIL-2) were then added to each well. The cultures were further incubated, pulsed with ³H-thymidine, harvested, and analyzed for ³H-thymidine incorporation as described above.

### Natural killer (NK) cell activation assay.

Purified CLMF was tested for its ability to activate NK cells when added alone or in combination with rIL-2 as follows. Human PBMC were isolated by centrifugation over discontinuous Ficoll and sucrose gradients as described above and were suspended in RPMI 1640 medium supplemented with 10% heat-inactivated fetal bovine serum, 100 units/ml penicillin, 100 µg/ml streptomycin, and 2 mM L-glutamine. The PBMC were incubated overnight at 37°C in 1 ml cultures (5 x 10⁶ cells/culture) together with rIL-2 and/or purified CLMF at various concentrations. After 18-20 hours, the contents of the cultures were harvested and centrifuged, and the cells were resuspended in the same medium used for the overnight cultures. The cytolytic activity of the cultured PBMC was then assessed in ⁵¹Cr release assays as described above.

### Concentration of cell supernatant solutions

Stored, frozen crude human CLMF supernatant solutions totaling 60 liters prepared from several batches of induced NC-37 cells were pooled and concentrated 30-fold using the Pellicon Cassette System (30,000 NMWL PTTK00005; Millipore Corp., Bedford, MA). After concentrating to the desired volume of approximately 1.9 liters, a buffer exchange was performed with 10 mM MES, pH adjusted to 6.0 with 10 N NaOH. The concentrate was centrifuged at 10.000 x g for 10 minutes at 4°C and the precipitate discarded.

### Ion-Exchange Chromatography on NuGel P-SP Column

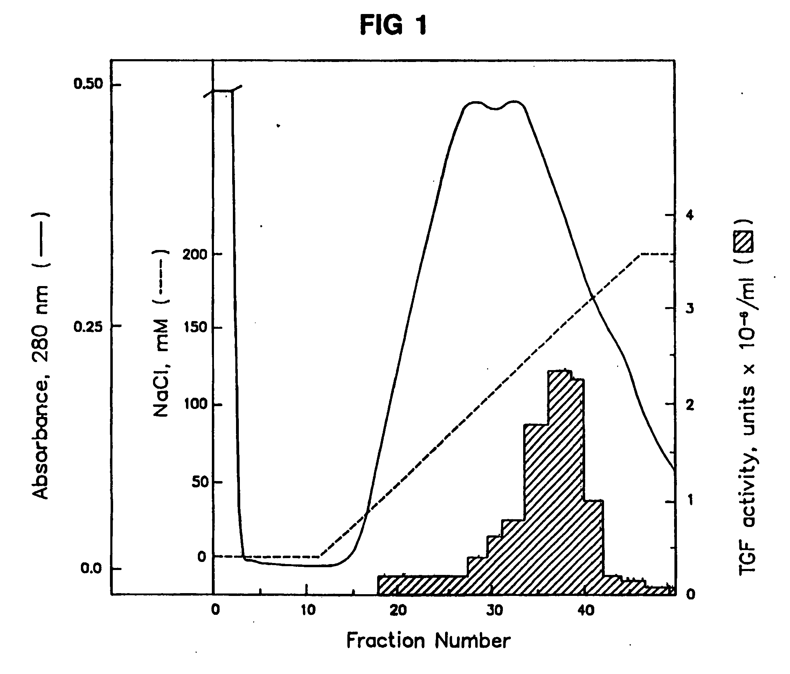

The concentrated supernatant solution was applied at a flow rate of 120 ml/hr to a Nu-Gel P-SP (Separation Industries, Metuchen, NJ) column (5 x 5 cm), equilibrated in 10mM MES, pH 6.0. The column was washed until baseline absorbance monitoring at 280 nm was obtained. Absorbed proteins were then eluted with a 500 ml salt gradient from 0 to 0.5 M NaCl/10 mM MES, pH 6.0 at a flow rate of 2 ml/min (Fig. 1). Aliquots of fractions were assayed for T cell growth factor (TGF) activity. Fractions containing TGF activity were pooled and dialyzed (Spectra/Por 7, Fisher Scientific) against 50 volumes 20 mM Tris/HCl, pH 7.5 in order to reduce the salt concentration of the preparation by 50-fold.

### Dye-Affinity Chromatography on Blue B-Agarose Column

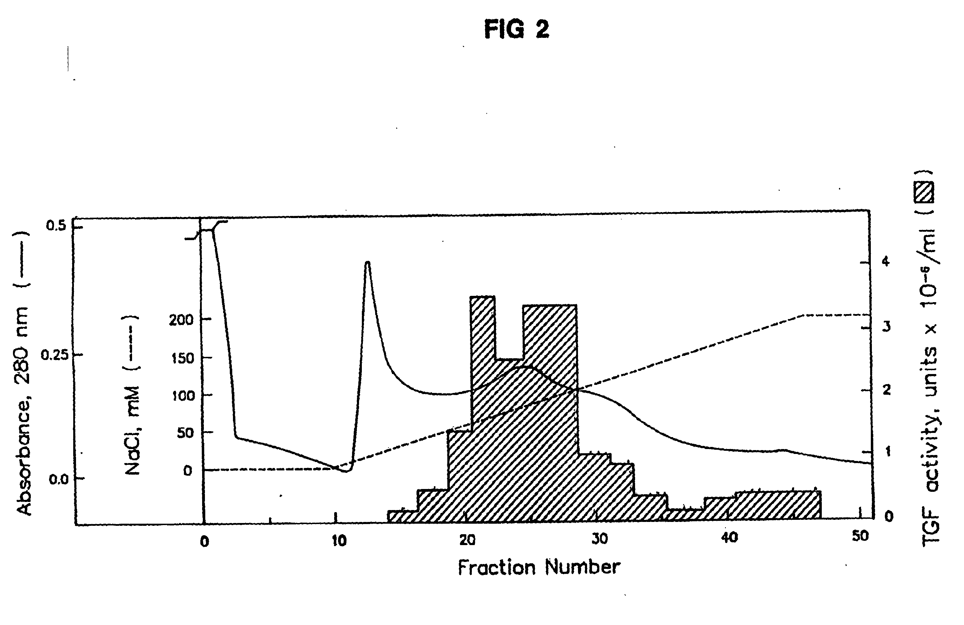

The dialyzed sample was centrifuged at 10.000 x g for 10 minutes at 4°C and the precipitate discarded. The supernatant solution was applied at a flow rate of 20 ml/hr to a Blue B-Agarose (Amicon, Danvers, MA) column (2.5 x 10 cm) equilibrated in 20 mM Tris/HCl, pH 7.5. The column was washed with this same buffer until baseline absorbance monitoring at 280 nm was obtained. Absorbed proteins were then eluted with a 500 ml salt gradient from 0 to 0.5 M NaCl/20 mM Tris/HCl, pH 7.5 at a flow rate of 15 ml/hr (Fig. 2). Aliquots of fractions were assayed for TGF activity. Fractions containing TGF activity were pooled and dialyzed (Spectra/Por 7, Fisher Scientific) against 100 volumes 20 mM Tris/HCl, pH 7.5 in order to reduce the salt concentration of the preparation by 100-fold.

### Ion-Exchange Chromatography on Mono Q Chromatography

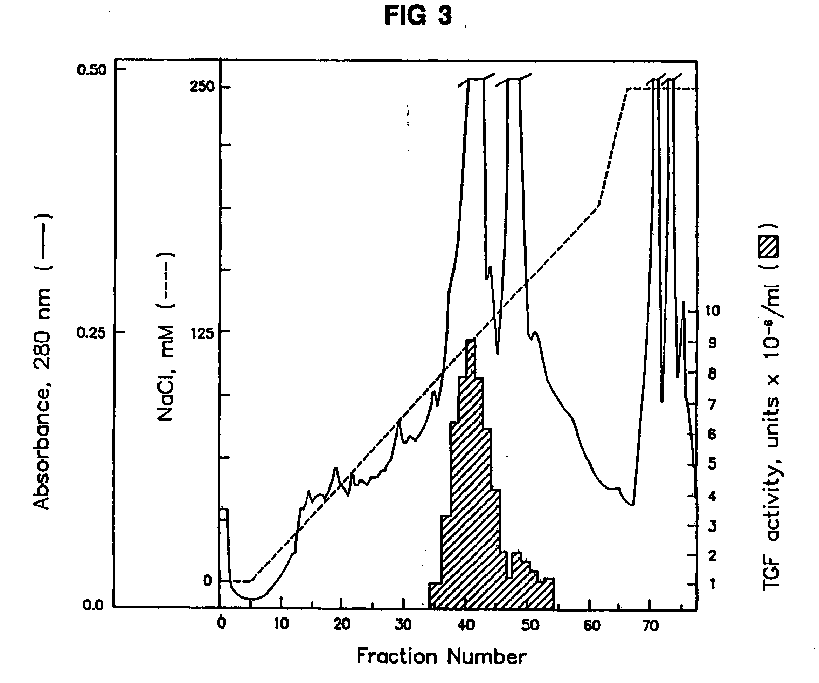

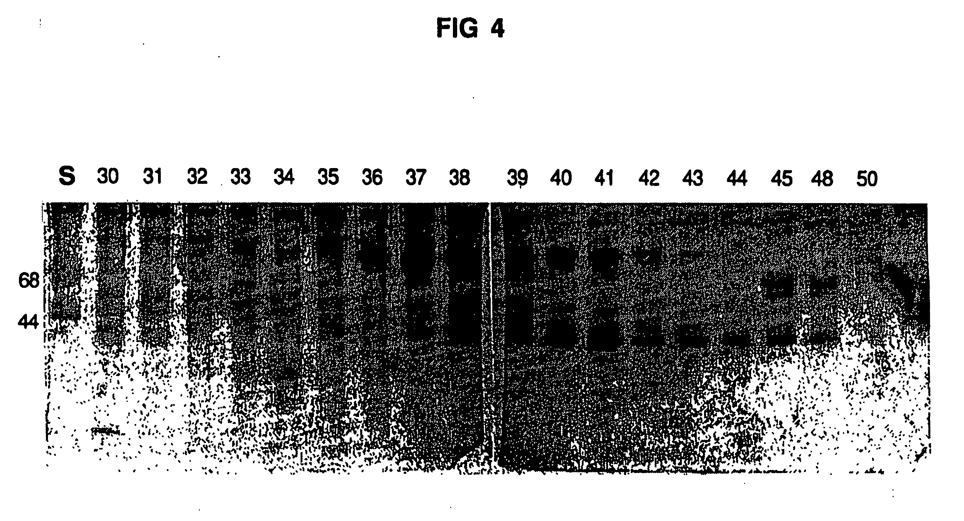

The dialyzed sample was filtered through a 0.45 µm cellulose acetate filter (Nalgene Co., Rochester, NY) and the filtrate applied at a flow rate of 60 ml/hr to a Mono Q HR 5/5 (Pharmacia LKB Biotechnology, Inc., Piscataway, NJ) column (5 x 50mm) equilibrated in 20mM Tris/HCl, pH 7.5. The column was washed with this same buffer until baseline absorbance monitoring at 280 nm was obtained. Absorbed proteins were then eluted with a 1 hr linear salt gradient from 0 to 0.25 M NaCl/20 mM Tris/HCl, pH 7.5 at a flow rate of 60 ml/hr (Fig. 3). Aliquots of fractions were assayed for TGF activity and protein purity was assessed without reduction by SDS-PAGE [Laemmli, Nature (London) 227:680-685 (1970)] using 12% slab gels. Gels were silver stained [Morrissey, Anal. Biochem. 117:307-310 (1981)] to visualize protein (Fig. 4). Fractions 36 and 37 were of greater than 95% purity and revealed a major band at 75,000 molecular weight. Fractions 38 through 41 containing TGF activity, revealed the 75 kDa protein by SDS-PAGE with major contaminants at 55,000 and 40,000 molecular weight.

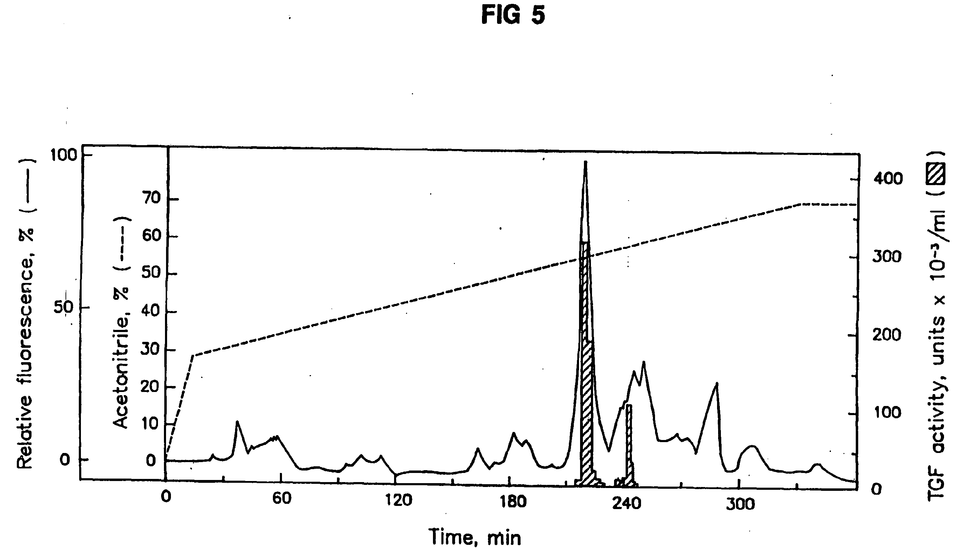

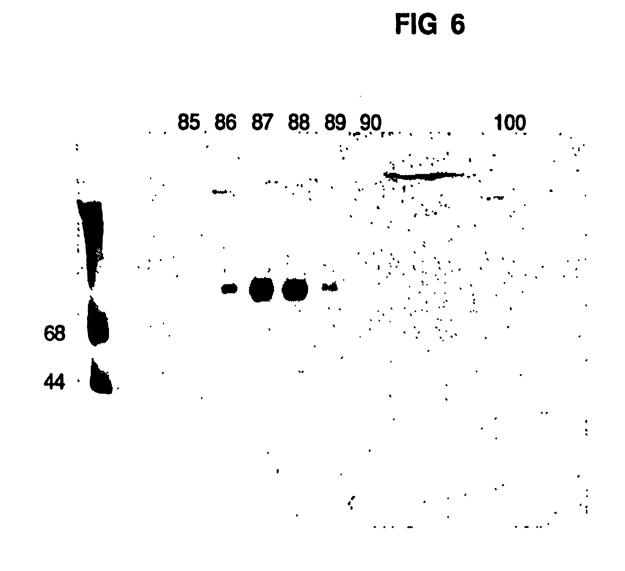

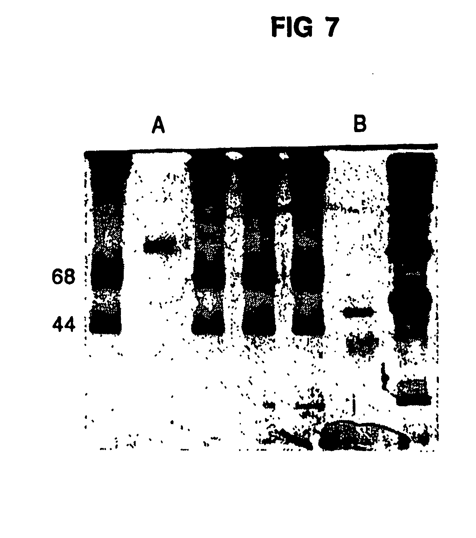

Therefore, to eliminate these contaminating proteins, fraction 38 of the previous Mono Q chromatography was diluted 1:1 vol/vol with 8 M urea and pumped onto a Vydac diphenyl column using a reversed-phase HPLC enrichment technique. The column was then washed with 5 ml of 0.1% trifluoroacetic acid. Elution of the proteins was accomplished with a gradient of 0-70% acetonitrile over 7 hrs in 0.1% trifluoroacetic acid (Fig. 5). Aliquots of fractions were assayed for TGF activity. Protein purity of the fractions containing TGF activity was assessed by SDS-PAGE under non-reducing conditions using a 10% slab gel. The gel was silver stained to visualize protein (Fig. 6). Fractions 86 through 90 were of greater than 95% purity and revealed protein of 75,000 molecular weight. Fractions 87 and 88 were pooled and aliquots were analyzed by SDS-PAGE under reducing (in the presence of β-mercaptoethanol) and non-reducing conditions (in the absence of β-mercaptoethanol). Under the reducing conditions, the 75,000 molecular weight CLMF was separated into two subunits of 40,000 and 35,000 daltons (Fig. 7). Thus it was concluded that CLMF is a 75 kDa heterodimer composed of disulfide--bonded 40 kDa and 35 kDa subunits.

The overall purification of CLMF that was achieved is shown in Table 1. The protein content of the Mono Q- and Vydac diphenyl-purified material was calculated on the basis

**TABLE 1**

| Step | Volume (ml) | Pooled Activity (U/ml) | Total Units (U) | Pooled Protein (mg/ml) | Total Protein (mg) | Specific Activity (U/mg) |
|---|---|---|---|---|---|---|
| Pooled Cell Supernatants | 60,000 | 2.58 x 10³ | 1.6 x 10⁸ | ND | ND | ND |
| | | | | | | |
| Ultrafiltered Concentrate | 1,940 | 1.57 x 10⁵ | 3.0 x 10⁸ | 1.83 | 3550 | 8.5 x 10⁴ |
| | | | | | | |
| NuGel P-SP | 90 | 2.00 x 10⁶ | 1.8 x 10⁸ | 0.70 | 63 | 2.8 x 10⁶ |
| | | | | | | |
| Blue-B-Agarose | 45 | 3.11 x 10⁶ | 1.4 x 10⁸ | 0.24 | 11 | 1.3 x 10⁷ |
| | | | | | | |
| Mono Q Fraction 37 | 1 | 6.40 x 10⁶ | 6.4 x 10⁶ | 0.075 | 0.075 | 8.5 x 10⁷ |
| | | | | | | |
| mono Q Fraction 38->42 | 5 | 6.90 x 10⁶ | 3.45 x 10⁷ | 0.081 | 0.405 | 8.5 x 10⁷ |
| | | | | | | |
| Diphenyl Fraction 87+88 | 1.1 | 5.74 x 10⁵ | 5.22 x 10⁵ | 0.008 | 0.010 | 5.2 x 10⁷ |

of amino acid analysis. A specific activity of 8.5 x 10⁷ units/mg and 5.2 x 10⁷ units/mg for Mono Q- and Vydac diphenyl-purified material respectively, was obtained. The fact that the diphenyl-purified protein has a slightly lower specific activity than the Mono Q-purified material may be due to inactivation or denaturation of some of the molecules of CLMF in the HPLC elution solvents (i.e., acetonitrile in 0.1% trifluoroacetic acid).

### Chemical Characterization

The ability to prepare homogeneous CLMF allowed for the first time the determination of the amino acid composition and a partial sequence analysis of the naturally occurring CLMF protein. Between 10 and 20 picomoles of Mono-Q-purified CLMF was subjected to hydrolysis, and its amino acid composition was determined (Table 2). Proline, cysteine and tryptophan were not determined (ND). Quantitation of histidine was not possible due to a large artifact peak, associated with Tris, coeluting with His (*)

Between 5 and 30 picomoles of diphenyl-purified CLMF was subjected to hydrolysis with and without pre-treatment with performic acid. Complete amino acid composition was thus obtained (Table 3) with the exception of tryptophan.

Amino-terminal sequence determination was attempted by automated Edman degradation on 100 pmol of the Mono Q-purified CLMF. Data from the first 22 cycles indicated two sequences present, as would be expected from the heterodimeric structure of CLMF. These results may be summarized as follows:

| Cycle | 1 | 2 | 3 | 4 | 5 | 6 | 7 | 8 |
|---|---|---|---|---|---|---|---|---|
| Amino Acid | I/? | W/? | E/L | L/P | K/V | K/A - | D/T | V/P |
| Cycle | 9 | 10 | 11 | 12 | 13 | 14 | 15 | 16 |
| Amino Acid | Y/D | V/P | V/G | E/M | L/F | D/P | W/? | Y/L |
| Cycle | 17 | 18 | 19 | 20 | | 21 | | 22 |
| Amino Acid | P/H | D/H | A/S | P/Q | | G/? | | E/? |

**TABLE 2**

| Amino Acid | mol % |
|---|---|
| Aspartic acid or asparagine | 11.8 |
| Threonine | 7.8 |
| Serine | 8.4 |
| Glutamic acid or glutamine | 14.9 |
| Proline | ND |
| Glycine | 6.2 |
| Alanine | 7.6 |
| Cysteine | ND |
| Valine | 6.9 |
| Methionine | 2.0 |
| Isoleucine | 4.6 |
| Leucine | 9.0 |
| Tyrosine | 3.7 |
| Phenylalanine | 4.0 |
| Histidine | * |
| Lysine | 9.3 |
| Arginine | 5.4 |
| Tryptophan | ND |

**TABLE 3**

| Amino acid | mol % |
|---|---|
| Aspartic acid or asparagine | 10.8 |
| Threonine | 7.2 |
| Serine | 8.9 |
| Glutamic acid or glutamine | 13.1 |
| Proline | 3.8 |
| Glycine | 4.7 |
| Alanine | 5.9 |
| Cysteine | 2.9 |
| Valine | 6.2 |
| Methionine | 1.9 |
| Isoleucine | 4.2 |
| Leucine | 9.4 |
| Tyrosine | 3.6 |
| Phenylalanine | 3.7 |
| Histidine | 1.8 |
| Lysine | 7.7 |
| Arginine | 4.4 |
| Tryptophan | ND |

### Reversed-Phase HPLC

The chromatographic system has been described previously by Stern, A.S. and Lewis. R.V. (1985) in Research Methods in Neurochemistry, Eds. Marks, N. and Rodnight, R. (Plenum. New York) Vol. 6. 153-193. An automated fluorescence detection system using fluorescamine (Polysciences, Inc., Warrington, PA) monitored the protein in the column effluents [Stein. S. and Moschera, J. (1981) Methods Enzymol. 78:435-447]. Reversed-phase HPLC was carried out using Vydac C18 or diphenyl columns (4.6 x 20mm. The Sep/a/ra/tions Group, Hesperia, CA). Proteins were eluted with an acetonitrile gradient in 0.1% TFA.

### Protein Analysis

Amino acid analysis was performed on an instrument which used post-column reaction with fluorescamine for detection [Pan. Y.-C.E., and Stein, S. (1986) in Methods of Protein Microcharacterization (Shively, J.E., Ed.). pp. 105-119. Humana Press, Clifton. NJ].

Sequence analysis was performed using an Applied Biosystems Inc. Model 470A gas phase sequencer (Foster City, CA) [Hewick, R.M., Hunkapillar, M.W., Hood, L.E., and Dreyer. W.J.. J. Biol. Chem. 256:7990-7997 (1981)]. Phenylthiohydantoin (PTH) amino acid derivatives were identified "on-line" with an ABI Model 120A PTH analyzer.

### DETERMINATION OF PARTIAL AMINO ACID SEQUENCES OF THE SUBUNITS OF CLMF

### Purification of the 40 kDa subunit of CLMF

Stored supernatant solutions from NC-37 cells totaling 39.1 liters were pooled and concentrated to approximately 2.4 liters using the Pellicon Cassette System and stored at -20°C. To clarify this concentrate after thawing, the preparation was centrifuged and the precipitate discarded.

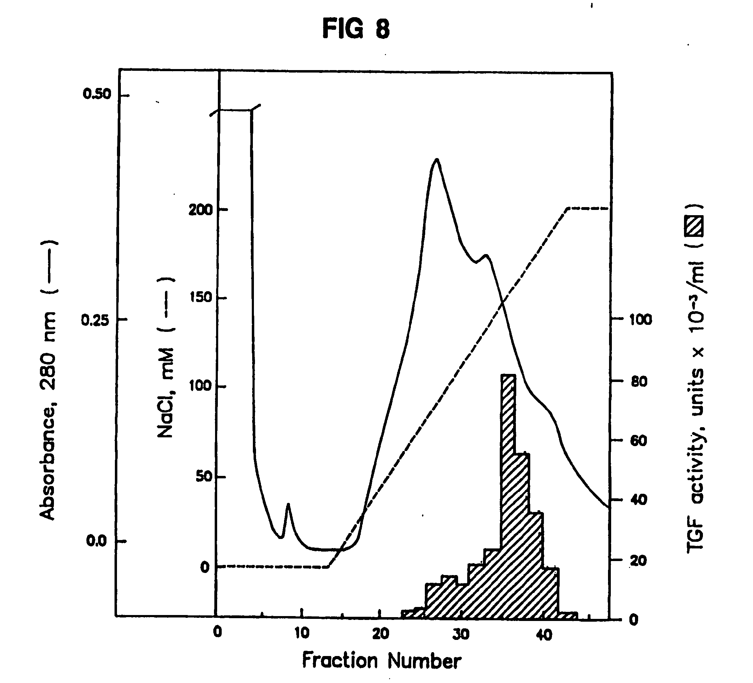

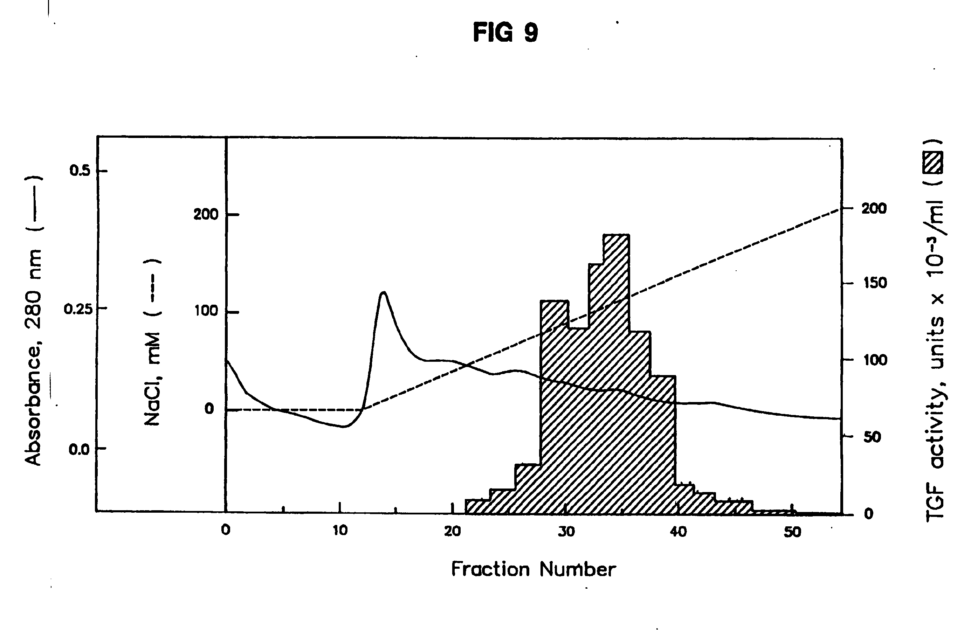

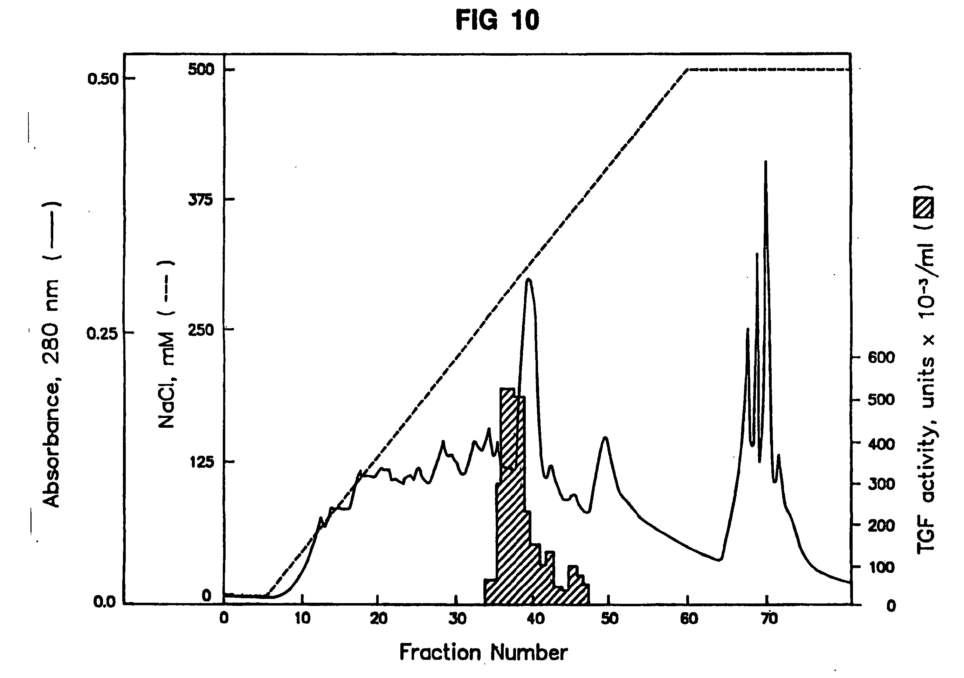

The supernatant solution was applied to a Nu-Gel P-SP column and protein was eluted with a salt gradient (Fig. 8). Peak TGF activity was determined and the active fractions were pooled and dialyzed in order to reduce the salt concentration of the preparation by 50-fold. This material, after centrifugation to remove particulates, was applied to a Blue-B-Agarose column. Protein was eluted with a salt gradient (Fig. 9). Peak TGF activity was determined and the active fractions were pooled and dialyzed in order to reduce the salt concentration of the preparation by 100-fold. This material, after filtration, was applied to a Mono Q column. Protein was eluted with a salt gradient (Fig. 10). Aliquots of fractions were assayed for TGF activity.

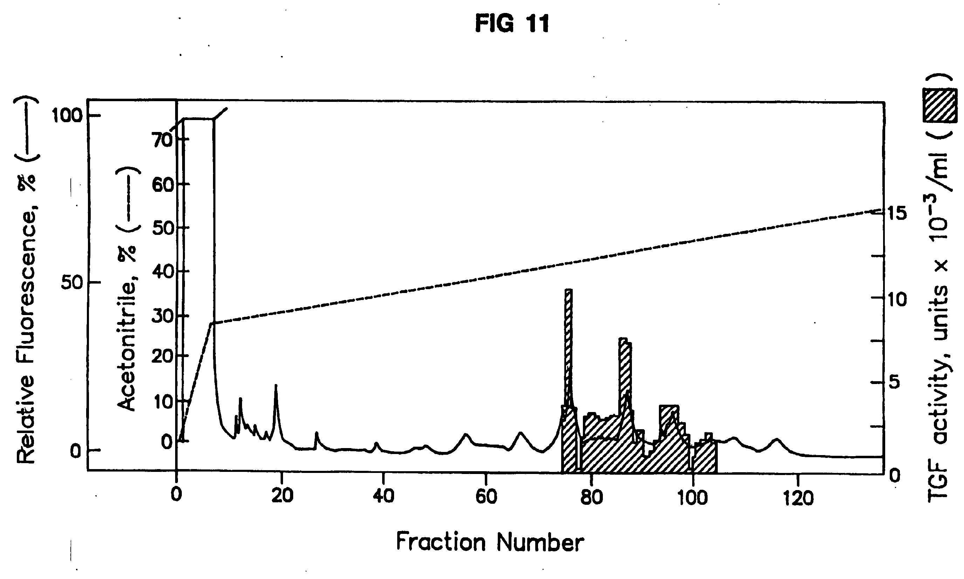

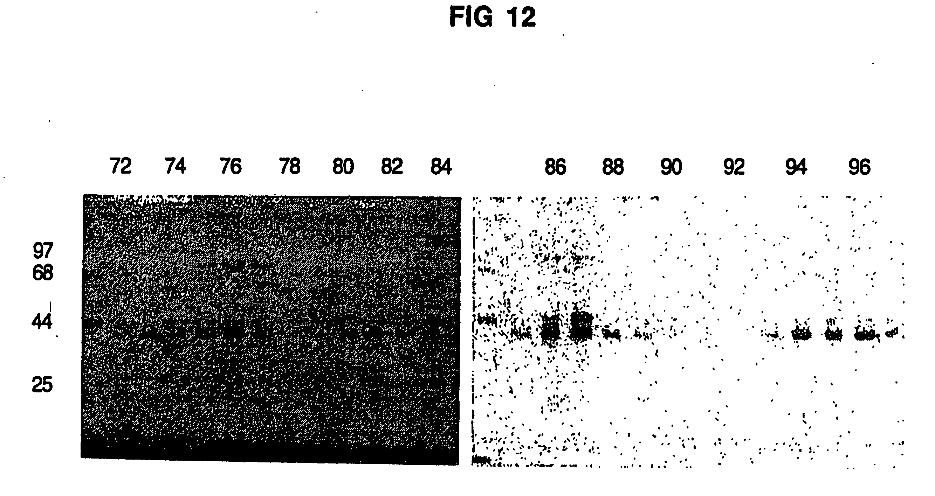

Fractions 39 and 40 of the previous Mono Q chromatography were pooled and diluted 1:1 vol/vol with 8M urea and pumped onto a Vydac diphenyl column using an enrichment technique. The column was then washed with 5 ml of 0.1% trifluoroacetic acid. Elution of the proteins was accomplished with a gradient of 0-70% acetonitrile over 7 hrs in 0.1% trifluoroacetic acid (Fig. 11). Aliquots of fractions were assayed for TGF activity. Protein purity of the fractions containing TGF activity was assessed by SDS-PAGE under reducing conditions, i.e. in the presence of β-mercaptoethanol (Fig. 12). Fractions 94 through 97 contained the 40,000 dalton subunit >90% pure.

### Determination of the amino-terminal sequences of the subunits of CLMF

The ability to prepare a highly enriched preparation of the 40,000 dalton subunit of CLMF allowed for its partial sequence analysis.

Amino terminal sequence determination was attempted by automated Edman degradation on 20 pmol of the diphenyl-purified 40,000 dalton subunit. The results may be summarized as follows:

| Cycle | 1 | 2 | 3 | 4 | 5 | 6 | 7 |
|---|---|---|---|---|---|---|---|
| Amino Acid | I | W | E | L | K | K | D |
| Cycle | 8 | 9 | 10 | 11 | 12 | 13 | 14 |
| Amino Acid | V | Y | V | V | E | L | D |
| Cycle | 15 | 16 | 17 | 18 | 19 | 20 | 21 |
| Amino Acid | W | Y | P | D | A | P | G |
| Cycle | 22 | 23 | | | | | |
| Amino Acid | E | M | | | | | |

With regard to the sequence analysis of 75,000 dalton CLMF and the sequence analysis of the 40,000 dalton subunit of CLMF, one can deduce the amino terminal sequence of the 35,000 dalton subunit of CLMF. The amino terminal sequences of the 35,000 dalton subunit and the 40,000 dalton subunit can be summarized as follows:
35,000 dalton subunit: 
                    5                              10
NH -?-?-Leu-Pro-Val-Ala-Thr(?)-Pro-Asp-Pro-Gly-
  2
              15                    20
Met-Phe-Pro-?-Leu-His-His-Ser(?)-Gln-
40,000 dalton subunit: 
1                    5                         10
NH -Ile-Trp-Glu-Leu-Lys-Lys-Asp-Val-Tyr-Val-Val-Glu
  2
        15                    20          23
Leu-Asp-Trp-Tyr-Pro-Asp-Ala-Pro-Gly-Glu-Met-
where ? represents an undetermined or "best-guessed" residue.

### Determination of internal amino acid sequence segments of the 40 kDa subunit of CLMF

CLMF was purified as described above. The 40,000 dalton subunit was separated and purified from the 35,000 dalton subunit by the method described by Matsudaira [J. Biol. Chem. 262: 10035-10038 (1987)]. Fifty micrograms of CLMF (in 500 µl of 20 mM Tris, pH 7.5; 0.15 M NaCl) was diluted with 200 µl of a 2 x concentrate of sample buffer [Laemmli, Nature 227: 680-685 (1970)]. The sample was concentrated to 400 µl and disulfide bonds broken by the addition of 18 µl β-mercaptoethanol followed by exposure to 105°C for 6 minutes.

The sample was loaded onto a minigel (1.0 mm thick) containing 12% polyacrylamide and electrophoresed according to Laemmli (supra). After electrophoresis, the gels were soaked in transfer buffer (10 mM 3-cyclohexylamino-1-propanesulfonic acid, 10% methanol, pH 11.0) for 5 minutes to reduce the amount of Tris and glycine. During this time, a polyvinylidene difluoride (PVDF) membrane (Immobilon; Millipore; Bedford, MA) was rinsed with 100% methanol and stored in transfer buffer. The gel, backed with two sheets of PVDF membrane and several sheets of blotting paper, was assembled into a blotting apparatus and electroeluted for 30 min at 0.5 Amps in transfer buffer. The PVDF membrane was washed in deionized H₂0 for 5 minutes. The edge of the blot was excised from the PVDF membrane and stained with 0.1% Coomassie Blue R-250 in 50% methanol for 5 minutes, and then destained in 50% methanol, 10% acetic acid for 5-10 minutes at room temperature. The 40,000 dalton stained band was then matched to the corresponding region of the unstained blot and the 40,000 subunit was cut from the unstained PVDF.

The N-termines of the Coomassie Blue-stained 40,000 dalton subunit was sequenced to confirm that the N-terminus matched the one previously determined (see above). By this method, the 40,000 dalton protein was identified as the 40,000 subunit of CLMF.

Five percent of the PVDF bound 40,000 dalton subunit was analyzed for its amino acid composition (Table 4). The remaining 95% of the blotted 40,000 dalton subunit was fragmented with trypsin according to the procedure of Bauw, et al. [Proc. Natl. Acad. Sci. USA 86: 7701-7705 (1989)]. The membrane carrying the protein was cut into pieces of approximately 3 by 3 mm, and collected in an Eppendorf tube. They were then immersed in 300 µl of a 2% polyvinylpyrrolidone (40,000 dalton) solution in methanol. After 30 minutes, the quenching mixture was diluted with an equal volume of distilled water and further incubated for 5-10 minutes. The supernatant solution was then discarded and the membrane pieces were washed four times with 300 µl water and once with 300 µl 100 mM Tris HCI (pH 8.5). Two hundred microliters of this buffer containing 2 µg of trypsin was added. The sample was shaken and incubated for 4 hours at 37°C. The supernatant solution was then transferred into a second Eppendorf tube and the membrane pieces were further washed once with 100 µl of 88% (vol/vol) formic acid and three times with 100 µl of deionized water. All washing solutions were added to the digestion mixture in the second Eppendorf tube. The resultant peptides contained in the pooled digest were separated by narrow bore HPLC (HP1090A, Hewlett Packard) on a YMC C-18 column (2.6 x 50 mm; Morris Plains, NJ).

**TABLE 4**

| Amino Acid | Residue No. | |
|---|---|---|
| Aspartic acid or asparagine | 27.9 | (28) |
| Threonine | 20.7 | (23) |
| Serine | 24.6 | (34) |
| Glutamic acid or glutamine | 44.6 | (35) |
| Proline | ND | (14) |
| Glycine | 16.3 | (15) |
| Alanine | 16.2 | (14) |
| Cysteine | ND | (10) |
| Valine | 20.9 | (23) |
| Methionine | 2.5 | (2) |
| Isoleucine | 10.3 | (12) |
| Leucine | 22.9 | (22) |
| Tyrosine | 12.9 | (12) |
| Phenylalanine | 9.9 | (9) |
| Histidine | 5.2 | (5) |
| Lysine | 24.5 | (26) |
| Arginine | 12.5 | (12) |
| Tryptophan | ND | (10) |

Note: The results represent the mean of two analyses. Proline, cysteine, and tryptophan were not determined (ND). Values in parentheses represent the theoretical amino acid composition of the 40,000 dalton subunit based upon the primary structure of the protein deduced from sequence analysis of cloned 40.000 dalton subunit.

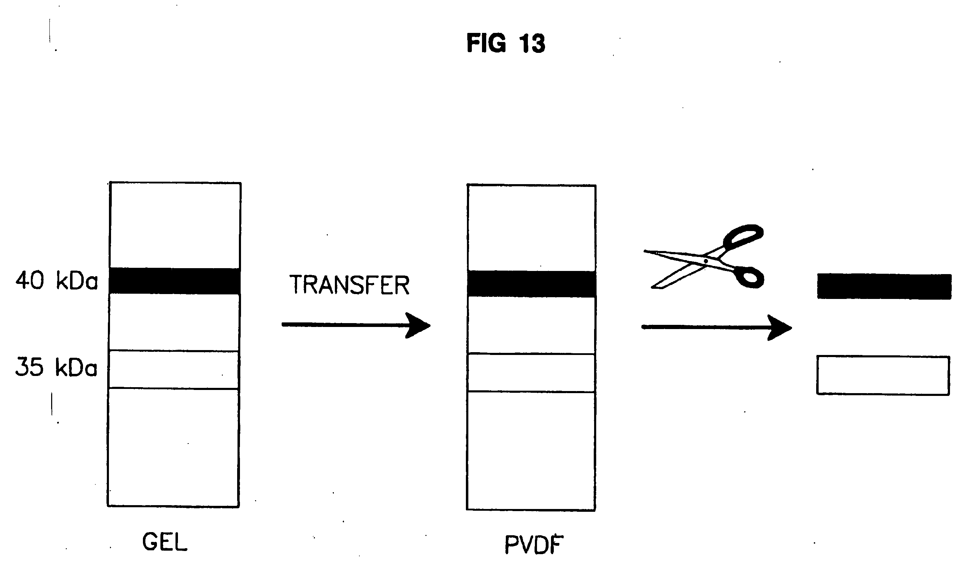

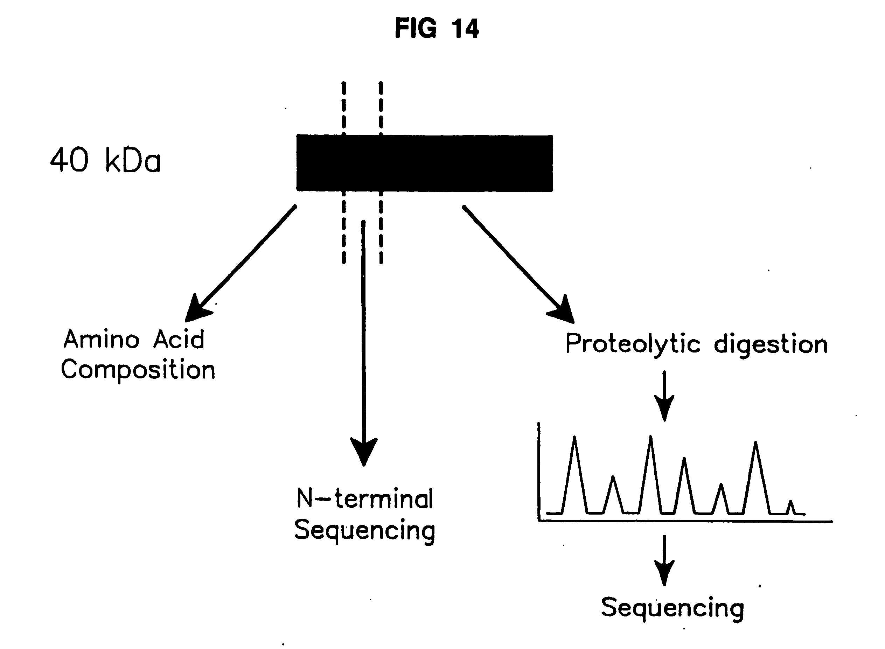

The above described procedure is shown schematically in Figures 13 and 14.

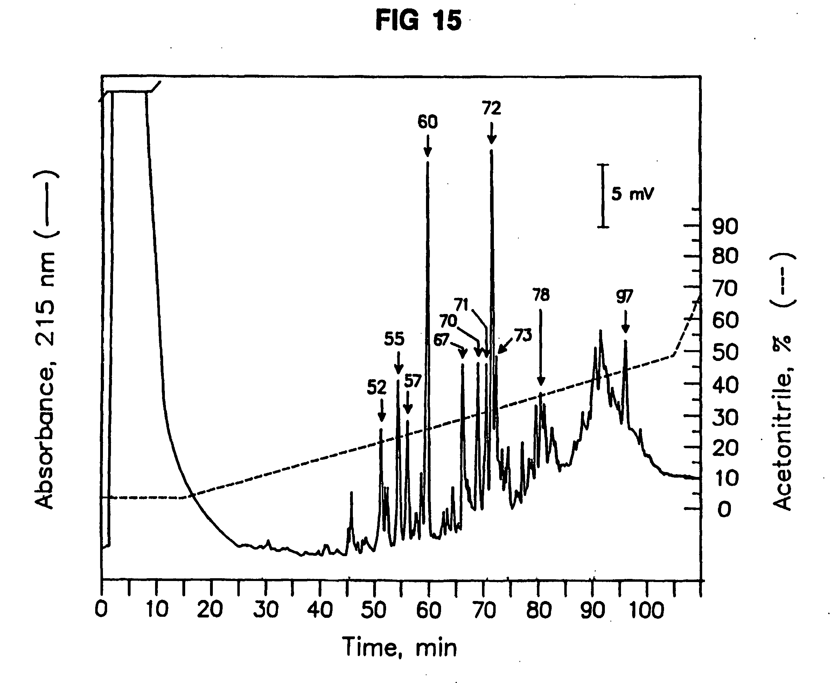

The tryptic peptide map of the digested 40,000 dalton subunit is shown in Figure 15. Peptides were eluted with a linear gradient of acetonitrile. The peaks which were sequenced are numbered according to their fraction number. The amino acid sequence of these peptides is shown in Table 5.

Many tryptic peptides were recovered from all regions of the intact 40,000 dalton subunit (Table 5). The N-terminal hexapeptide (fraction no. 60) was recovered in high yield. The carboxy-terminal peptide (fraction no. 72) was recovered and is the full length of the predicted C-terminal peptide although the last two amino acids were not positively confirmed by sequencing. This is probably due to the fact that Cys and Ser residues are not detected well, especially when they occur at the end of a peptide. Four potential Asn-linked carbohydrate sites may be predicted from the cDNA sequence. Two peptides containing two of these sites were sequenced. When peptide 196-208 (fraction no. 70) was sequenced, no peak was detected at residue 200 indicating that this Asn (predicted by the cDNA) is indeed glycosylated. Peptide 103-108 (fraction no. 52) yielded Asn at residue 103. Therefore, this site is not glycosylated.

An unknown peak seen in the phenylisothiocyanate (PTH) sequence analysis [Hewick et al., J. Biol. Chem. 256: 7990 (1981)] of fraction no. 55 was detected at the position corresponding to residue no. 148. The site is predicted to be a Cys residue which is normally not detected by sequence analysis unless it is modified.

The above PVDF transfer procedure was repeated on a second 50 µg aliquot of CLMF (see Figure 13 and 14 for

**TABLE 5**

| Tryptic 40kDa CLMF peptides off PVDF | | |
|---|---|---|
| fraction no. | residue no. | N-terminal sequence |
| 52 | 103-108 | N-K-T-F-L-R |
| 55 | 139-157 | G-S-S-D-P-Q-G-V-T-*-G-A-A-T-L-S-A-E-R |
| 55 & 57 | 267-279(?) | V-F-T-D-K-T-S-A-T-V-I-?-R |
| 57 | 52-58 | T-L-T-I-Q-V-K |
| 57 | 218-228 | N-L-Q-L-K-P-L-K-N-S-R |
| 60 | 1-6 | I-W-E-L-K-K |
| 67 | 288-? | A-Q-D-R-Y-Y-S-S- |
| 67 | 85-102(?) | K-E-D-G-I-W-S-T-D-I-L-K-D-Q-K-E-P- |
| 70 | 196-208 | L-K-Y-E-?-Y-T-S-S-F-F-I-(R?) |
| 71 | 85-96(?) | K-E-D-G-I-?-S-T-D-I-L-K |
| 72 | 288-306(?) | A-Q-D-R-Y-Y-S-S-S-W-E-?-A-S-V-P-?-? |
| 78 | 71-85 | (G?)-G-E-V-L-S-H-S-L-L-L-(L?)-H-K-K |

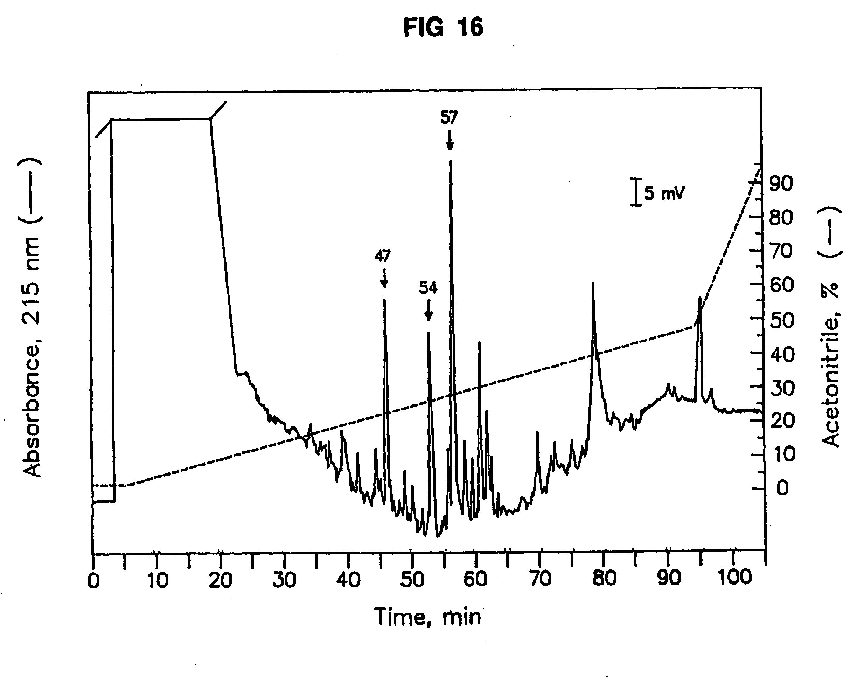

procedure outline). However, the blotted 40,000 dalton subunit was fragmented with the proteolytic enzyme, Staphylococcus aureus V8 protease (Endoproteinase Glu-C, Boehringer Mannheim, Indianapolis, IN). Membrane pieces were digested for 6 hours at 37°C with 20 µg of V8. The peptides were extracted with 88% (vol/vol) formic acid and separated on a Phase Separations column (2 x 150 mm, C8 S3, Queensferry, England, UK) (Figure 16). Peptides were eluted with a linear gradient of acetonitrile. The peaks which were sequenced are numbered according to their fraction number. The amino acid sequence of these peptides is shown in Table 6.

**TABLE 6**

| V8 (Glu-C) 40kDa peptides off PVDF | | |
|---|---|---|
| fraction no. | residue no. | N-terminal sequence |
| 47 | 1-3 | I-W-E |
| 54 | 4-12 | L-K-K-D-V-Y-V-V-E |
| 57 | 13-22 | L-D-W-Y-P-D-A-P-G-E |
| 57 | 45-59 | V-L-G-S-G-K-T-L-T-I-Q-V-K-(E?) |

Three major peaks of peptide (fraction nos. 47, 54 and 57) containing four peptides were sequenced. All four peptides were from the amino-terminal region of the 40 kDa subunit indicating that the N-terminus of the protein is most susceptible to V8-digestion.

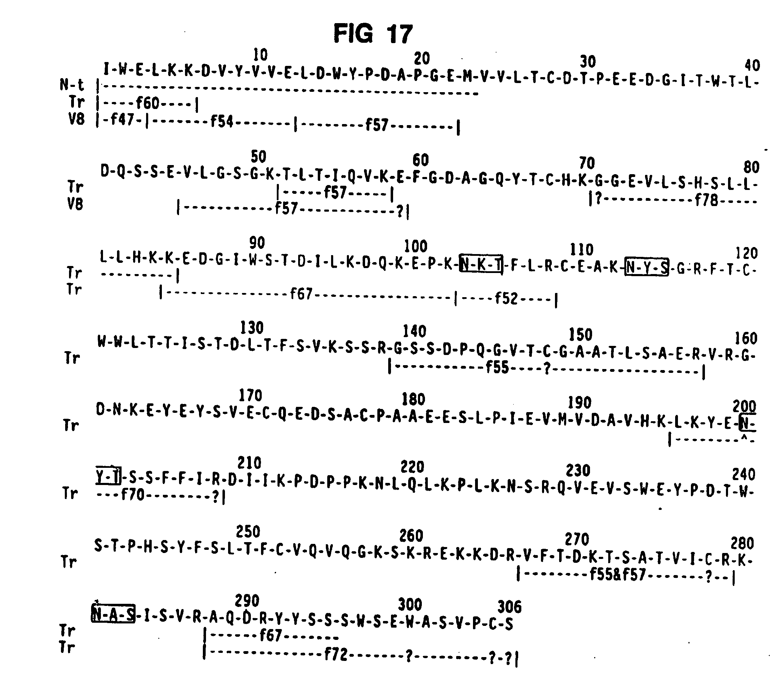

Figure 17 summarizes the protein structural determination of the 40,000 dalton subunit of CLMF.

### Direct determination of the amino-terminal sequence of the 35,000 dalton subunit of CLMF

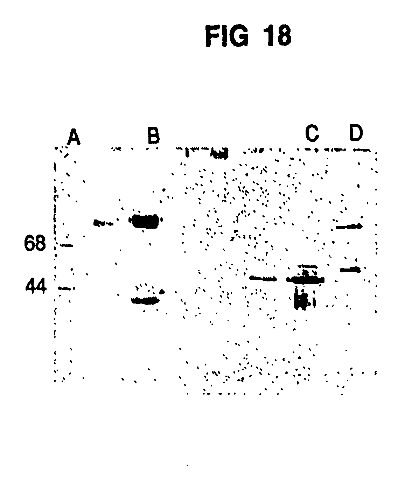

SDS-PAGE analysis of the Mono Q fraction 39 (see Fig. 3) under reducing (in the presence of β-mercaptoethanol) and non-reducing (in the absence of β-mercaptoethanol) conditions (Fig. 18) demonstrated that the 40,000 dalton molecular weight "contaminant" is "free" 40,000 dalton CLMF subunit (i.e. unassociated with the 35,000 dalton subunit). The evidence which points to this deduction is that without reduction (lane B, Fig. 18) mainly 75,000 dalton CLMF is present with some 40,000 dalton protein. After reduction (lane C, Fig. 18), the 75,000 dalton CLMF is gone yielding the 35,000 dalton subunit and an enriched 40,000 dalton band.

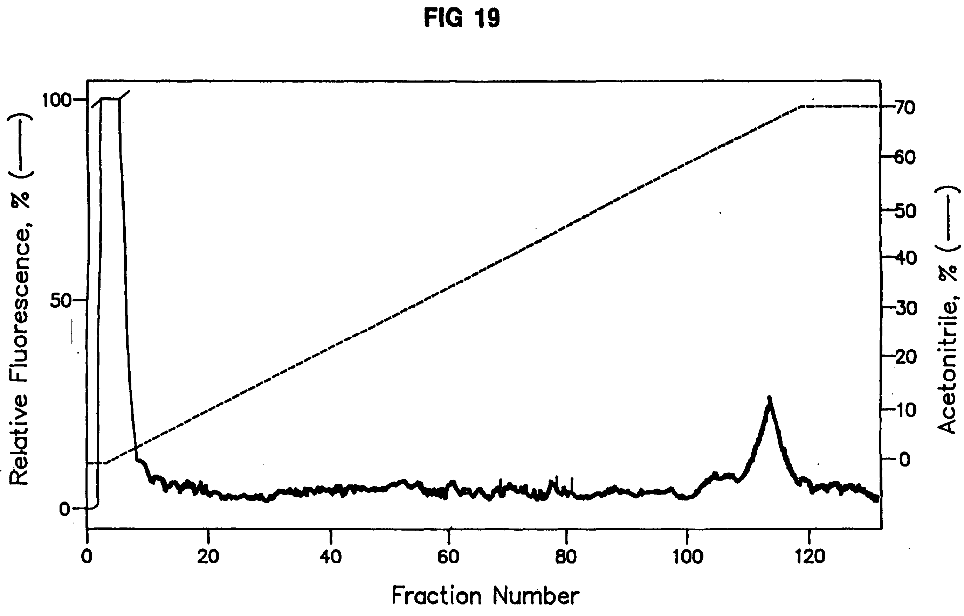

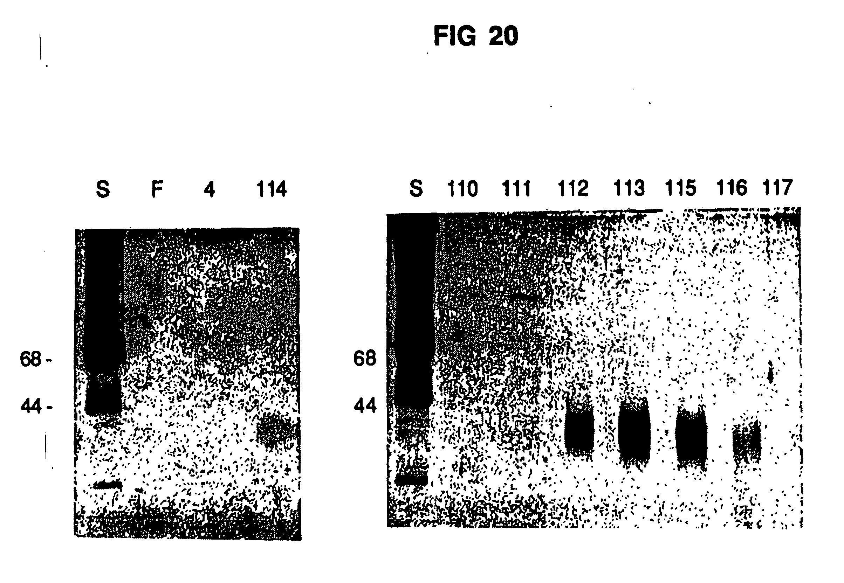

Fraction 39 of the previous Mono Q chromatography was reduced in 5% β-mercaptoethanol in the presence of 4 M urea and heated for 5 minute at 95°C. The sample was pumped onto a Vydac C-18 column using an enrichment technique and the column was then washed with 5 ml of 0.1% trifluoroacetic acid. Elution of the proteins was accomplished with a gradient of 0-70% acetonitrile over 5 hrs in 0.1% trifluoroacetic acid (Fig. 19). Protein purity of the fractions which were fluorescamine positive was assessed by SDS-PAGE under non-reducing conditions using a 10% slab gel. The gel was silver stained to visualize protein (Fig. 20). Fractions 112 through 117 revealed a diffuse band at 35,000 molecular weight which was greater than 95% pure. The 40,000 dalton subunit and any other proteins present in fraction 39 remained bound to the C-18 column. These proteins (including the 40,000 dalton subunit) were finally eluted with a solution of 42% formic acid/40% 1-propanol.

The ability to prepare homogeneous 35,000 subunit allowed for the determination of the amino acid composition and partial sequence analysis of the lower molecular weight subunit of the CLMF protein. Approximately 1 µg of 35 kDa subunit was subjected to hydrolysis, and its amino acid composition was determined (Table 7). Proline, cysteine and tryptophan were not determined (ND).

**TABLE 7**

| Amino Acid | Mol % |
|---|---|
| Aspartic acid or asparagine | 10.9 |
| Threonine | 6.7 |
| Serine | 8.3 |
| Glutamic acid or glutamine | 14.9 |
| Proline | ND |
| Glycine | 6.1 |
| Alanine | 7.7 |
| Cysteine | ND |
| Valine | 6.3 |
| Methionine | 2.9 |
| Isoleucine | 4.5 |
| Leucine | 10.9 |
| Tyrosine | 3.2 |
| Phenylalanine | 4.4 |
| Histidine | 2.3 |
| Lysine | 5.6 |
| Arginine | 5.5 |
| Tryptophan | ND |

Amino-terminal sequence determination was attempted by automated Edman degradation on 100 pmol of the C-18 purified 35 kDa subunit. Data from the first 20 cycles confirmed the sequence obtained by deduction as described above. Furthermore, the second amino acid was obtained in addition to amino acids 21 through 26. These results may be summarized as follows:

| Cycle | 1 | 2 | 3 | 4 | 5 | 6 | 7 |
|---|---|---|---|---|---|---|---|
| Amino Acid | ? | N | L | P | V | A | T |
| Cycle | 8 | 9 | 10 | 11 | 12 | 13 | 14 |
| Amino Acid | P | D | P | G | M | F | P |
| Cycle | 15 | 16 | 17 | 18 | 19 | 20 | 21 |
| Amino Acid | ? | L | H | H | S | Q | N |
| Cycle | 22 | 23 | 24 | 25 | 26 | | |
| Amino Acid | L | L | R | A | V | | |

Therefore, the amino terminal sequence of the 35,000 dalton subunit can be summarized as follows: 35,000 dalton subunit: 
5                    10
NH2-?-Asn-Leu-Pro-Val-Ala-Thr-Pro-Asp-Pro-Gly-Met-
15                20                    25  26
Phe-Pro-?-Leu-His-His-Ser-Gln-Asn-Leu-Leu-Arg-Ala-Val
 where ? represents an undetermined residue.

### Determination of the sequence of a tryptic fragment of CLMF

Mono Q fractions 36 and 37 from the initial purification of CLMF were pooled (approximately 100 pmol/1.7 ml). A 30 µl sample was removed and the volume of the rest was reduced to 200 µl under a stream of helium. One hundred microliters of 0.1 M ammonium bicarbonate was added. Trypsin (Worthington Biochemical Corp., Freehold, NJ) cleavage was performed at a substrate-to-enzyme ratio of 2:1 (w/w) at 37°C for 20 hours. The resultant peptide fragments were reduced and carboxymethylated. This was accomplished by addition of 160 µl of 0.1 M Tris-HCl, pH 8.5/6 M guanidine-HCl. The volume was reduced to 200 µl under a stream of helium, and 4 µl of dithiothreitol (50 mg/ml) was added. The mixture was incubated at 37°C for 4 hrs. After reductive cleavage of the disulfide bonds, [¹⁴C]iodoacetic acid (4 µmol) was added and the resulting solution was incubated in the dark at room temperature for 10 minutes.

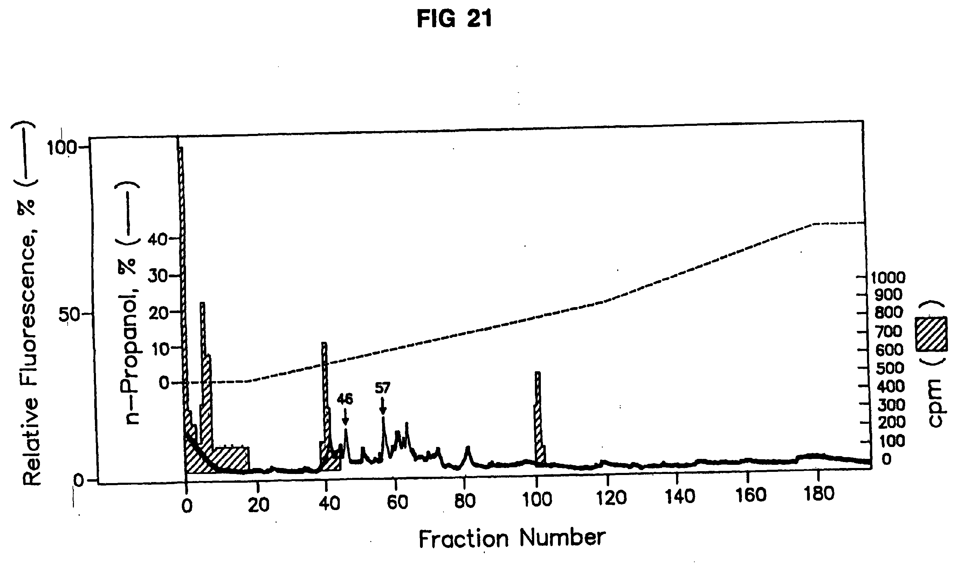

The resultant peptide fragments were isolated by reversed-phase HPLC (Fig. 21) on an S-5 120 Ångstrom ODS column (2.6 x 50 mm, YMC, Inc., Morris Plains, NJ). Peptides were eluted with a 1-propanol gradient in 0.9 M acetic acid, pH adjusted to 4.0 with pyridine. The amino acid sequence of the peptide found in fraction 46 was found to be: Asp-Ile-Ile-Lys-Pro-Asp-Pro-Pro-Lys (determined by automated Edman degradation).

### Determination of internal amino acid sequence segments of CLMF

CLMF was purified as previously described. Approximately 80 µg of protein was precipitated with 10% trichloroacetic acid. The precipitate was dissolved in 70% (v/v) aqueous formic acid at room temperature. An approximately 50-fold molar excess over methionine residues of cyanogen bromide (CNBr) in a small volume of 70% formic acid was added, with stirring, and the mixture was incubated in the dark under oxygen-free helium at room temperature for 48 hrs. The mixture was diluted with 15 volumes of water, divided into two equal portions and dried under a stream of helium. For complete removal of the acid and by-products, the drying was repeated after further addition of water.

One of the portions (approx. 40 µg) of fragmented CLMF was dissolved with 50 µl Laemmli sample buffer [Laemmli, Nature 227: 680-685 (1970)] containing 4% β-mercaptoethanol followed by exposure to 105° C for 6 minutes. The sample was loaded into 3 wells of a minigel (1.0 mm thick) containing 17.5% polyacrylamide and electrophoresed according to Laemmli (supra).

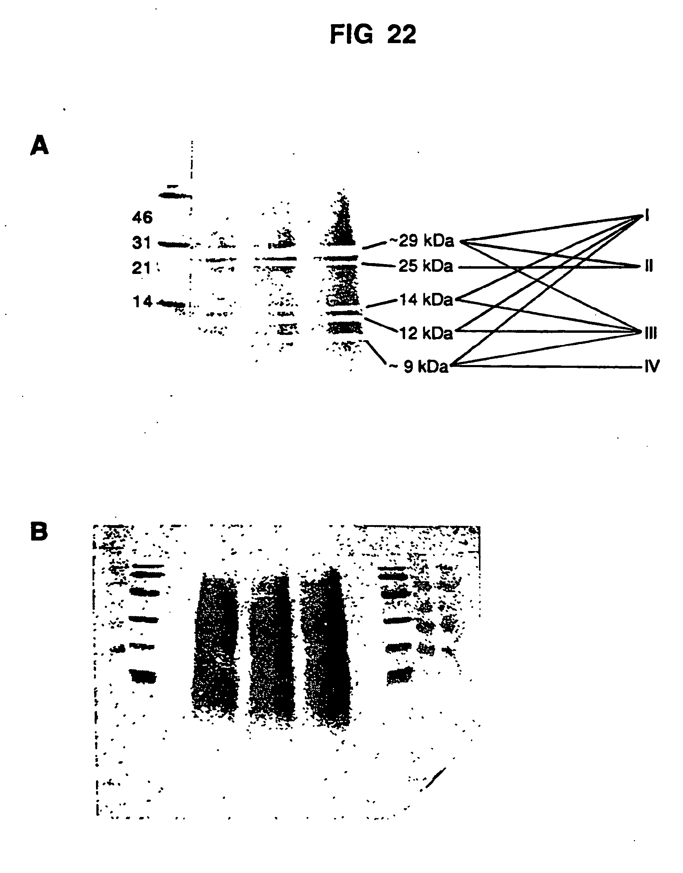

After electrophoresis, the gels were soaked in transfer buffer (10 mM 3-cyclohexylamino-1-propanesulfonic acid, 10% methanol, pH 11.0) for 30 min. During this time, a polyvinylidene difluoride (PVDF) membrane (Immobilon; Millipore; Bedford, MA) was rinsed with 100% methanol and stored in transfer buffer. The gel, backed with two sheets of PVDF membrane and sandwiched with blotting paper, was assembled into a blotting apparatus and electroeluted for 30 min. at 0.5 Amps in transfer buffer. The PVDF membrane was washed in deionized H₂O for 5 min and stained with 0.1% Coomassie Blue R-250 in 50% methanol for 5 min, and then destained in 50% methanol, 10% acetic acid for 5-10 min at room temperature. A number of smeared bands were observed (see Fig. 22B).

Five regions of the membrane were excised across the three last lanes containing the CLMF CNBr digest. These regions were sequenced. A summary of the sequences obtained from the CNBr fragments of CLMF is shown on Fig. 22A.

The second portion (approx. 40 µg) of fragment CLMF was dissolved in approx. 400-500 µl 88% formic acid containing 6 M guanidine HCl, 0.1 M Tris/HCl, 0.5 M NaOH, pH 8.0. The sample was pH adjusted to pH 4.0 with formic acid. The peptide fragments were isolated by reversed-phase HPLC (Fig. 23) on a Vydac C₄ column (4.6 x 20 mm, The Sep/a/ra/tions Group, Hesperia, CA). Peptides were eluted with a 4.5 hours linear gradient of acetonitrile in 0.1% TFA. One of these peaks was sequenced and the amino acid sequence of this peptide was:

| Fraction No. | N-Terminal Sequence |
|---|---|
| 47 | V-D-A-V-H-K-L-K-Y-E-?-Y-T-S-(S?) -F-F-I-R-D-I-I-K-P- |
| | (Starts at residue number 190 of 40 kDa subunit) |

It is assumed or known that the above sequence is preceded by a Met residue. The residue marked with "?" represents a "best-guessed" residue.

### PURIFICATION OF CLMF AND THE 40,000 DALTON SUBUNIT THEREOF USING AFFINITY CHROMATOGRAPHY

An affinity chromatography resin was prepared by covalently attaching the monoclonal antibody 7B2, the preparation of which is described below, to activated agarose. Similarly, the below outlined purification could also be carried out by covalently coupling the antibody to silica or thin microporous membranes. The activated agarose was prepared as follows:
1. 100 ml Sepharose CL-6B was washed three times with 100 ml H₂O.
2. 100 ml of 1% sodium meta-periodate in H₂O was added to the resin and the suspension shaken at room temperature for 60 min.
3. The resin was washed with cold H₂O thoroughly.

The covalent attachment of 7B2 to the activated agarose was carried out as follows:
1. 9 ml of the activated agarose prepared as described above was suspended in 7 ml of 7B2 (approx. 3.9 mg/ml) in phosphate buffered saline, pH 7.4.
2. 50.2 mg of cyanoborohydride was added to the gel suspension which was shaken overnight at 4°C.
3. The gel suspension was filtered and added to 7 ml of 1.0 M ethanolamine, pH 7.0 containing 50.2 mg of cyanoborohydride.

One milliliter of the above described resin (approx. 2.6 mg IgG/ml gel) was packed in a column and washed extensively with phosphate buffered saline. Fractions from the Mono Q chromatography containing the 75 kDa CLMF protein and additional major contaminating proteins were pooled (approx. 3.5 x 10⁶ U TGF activity) and dialyzed extensively against PBS. This preparation was applied to the 7B2-Sepharose column at a rate of 5 ml/hr at room temperature. The column was washed with phosphate buffered saline (pH 7.4) until baseline absorbance monitoring at 280 nm was obtained. Adsorbed proteins were then eluted with 0.2 N acetic acid, 0.15 M NaCl, at approx. pH 3. Aliquots of fractions were assayed for TGF activity. Approximately 76% of the starting activity was recovered in the acid eluate.

Protein purity was assessed without reduction by SDS-PAGE [Laemmli, Nature 227: 680-685 (1970)] using a 10% slab gel. Gels were silver stained [Morrissey, Anal. Biochem. 117:307-310 (1981)] to visualize protein. The acid eluant contained pure CLMF and the "free" unassociated 40 kDa subunit of CLMF (Fig. 24).

### DETERMINATION OF THE pI OF CLMF

Thirty microliters of the pooled Mono Q fractions 36 and 37 (see Fig. 3) were spotted onto a precast ampholine PAGplate gel, pH 3.5-9.5 (Pharmacia LKB Biotechnology) to determine the pI of CLMF. Based on pI standard markers, a major band was observed at pI 4.8 and a minor band at pI 5.2. Based on pH determination, the pI of these bands are 4.2 and 4.6 respectively.

### BIOLOGIC ACTIVITIES OF PURIFIED CLMF

Purified CLMF stimulated the proliferation of human PHA-activated lymphoblasts in the T cell growth factor assay (Table 8). The T cell growth factor activity of the purified CLMF recovered from the Mono Q column was compared to that of a standard preparation of human lymphokines in five separate experiments, and the specific activity of the purified CLMF was found to be 8.5 ± 0.9 X 10⁷ units/mg protein. In one experiment in which purified CLMF obtained from diphenyl HPLC was compared to the standard lymphokine preparation in the TGF assay, a specific activity of 5.2 X 10⁷ units/mg protein was observed. When suboptimal concentrations of purified CLMF and human rIL-2 were tested in combination in the TGF assay, additive proliferation was observed (Table 8), up to the maximum proliferation caused by rIL-2 alone. However, proliferation caused by rIL-2 could be distinguished from proliferation due to CLMF in that the former was totally inhibited in the presence of a neutralizing goat anti-human IL-2 antiserum but the latter was not affected.

The ability of purified CLMF to activate cytotoxic effector cells was examined both in a 4-day LAK cell induction assay and in an overnight NK cell activation assay. In the LCI assay, purified CLMF at concentrations as high as 800 units/ml had little activity in the absence of IL-2 (Table 9). However, CLMF synergized with low concentrations of human rIL-2 in causing LAK cell induction in as much as the lytic activity generated in the presence of both cytokines was significantly greater than the sum of the lytic activities observed in cultures containing either cytokine alone (Table 9). In the presence of rIL-2, purified CLMF was active at concentrations as low as 3 units/ml.

**TABLE 8**

| Purified Human CLMF Stimulates the Proliferation of Human PHA-Activated Lymphoblasts | | | |
|---|---|---|---|
| | Cytokine Added: | | ³H-Thymidine Incorporated by PHR-Activated Lymphoblasts (mean cpm + l S.E.M.) |
| Expt. | Human CLMF^{c} (u/ml) | Human rIL-2 (u/ml) | |
| 1^{a} | 0 | 0 | 10,607 ± 596 |
| | 500 | 0 | 70,058 ± 1,630 |
| | 100 | 0 | 60,377 ± 1,927 |
| | 20 | 0 | 36,018 ± 321 |
| | 4 | 0 | 24,996 ± 669 |
| | 0.8 | 0 | 17,765 ± 790 |
| | | | |
| 2^{b} | 0 | 0 | 9.976 ± 374 |
| | 200 | 0 | 60.980 ± 1,713 |
| | 50 | 0 | 38.817 ± 884 |
| | 12.5 | 0 | 18,885 ± 2,132 |
| | 3.1 | 0 | 13,648 ± 731 |
| | | | |
| | 0 | 16 | 80,041 ± 5,835 |
| | 0 | 4 | 21.282 ± 1,145 |
| | 0 | 1 | 11,241 ± 898 |
| | | | |
| | 50 | 4 | 62,050 ± 2,408 |
| | 12.5 | 4 | 40,628 ± 2,196 |
| | 3.1 | 4 | 31,144 ± 3,754 |

| | | | |
|---|---|---|---|
| ^{a} All cultures in experiment 1 contained goat anti-human rIL-2. ^{b} None of the cultures in experiment 2 contained goat anti-human rIL-2. ^{c} Purified human CLMF from Mono Q FPLC. | | | |

**TABLE 9**

| Purified Human CLMF Synergizes with Human rIL-2 in the Generation of Lymphokine-Activated Killer (LAK) Cells in 4-Day Cultures | | | |
|---|---|---|---|
| Cytokine Added: | | % Specific ⁵¹Cr Release^{a} from: | |
| Human CLMF^{b} (u/ml) | Human rIL-2 (u/ml) | K562 | Raji |
| | | | |
| 0 | 0 | 3 ± 1.7 | -1 ± 0.5 |
| 800 | 0 | 7 ± 0.3 | 1 ± 0.1 |
| 200 | 0 | 5 ± 1.1 | 1 ± 0.4 |
| 50 | 0 | 4 ± 3.0 | 0 ± 0.9 |
| | | | |
| 0 | 5 | 10 ± 2.4 | 2 ± 0.8 |
| 800 | 5 | 41 ± 4.0 | 11 ± 0.8 |
| 200 | 5 | 42 ± 1.9 | 11 ± 0.3 |
| 50 | 5 | 36 ± 2.7 | 9 ± 0.8 |
| 12.5 | 5 | 28 ± 2.1 | 7 ± 0.7 |
| 3.1 | 5 | 19 ± 0.8 | 5 ± 0.3 |
| 0.8 | 5 | 14 ± 1.2 | 3 ± 0.8 |

| | | | |
|---|---|---|---|
| ^{a} Values represent the means ± 1 S.E.M. of quadruplicate determination. The spontaneous ⁵¹Cr release values for K562 and Raji were 16% and 14%, respectively. ^{b} Purified human CLMF from Mono Q FPLC. | | | |

In contrast to the results in the 4-day LAK induction assay, purified CLMF was effective by itself in activating human NK cells in an overnight assay (Table 10). In this assay, CLMF was active at concentrations as low as 1.6 units/ml. When CLMF was tested in combination with human rIL-2, the two cytokines together had, at best, additive effects in enhancing NK activity (Table 10).

**TABLE 10**

| Purified Human CLMF Causes Activation of Natural Killer (NK) Cells in Overnight Cultures | | | |
|---|---|---|---|
| Cytokine Added: | | % Specific ⁵¹Cr Release^{a} from | |
| | | Raji Cells at Effector/Target Ratio=: | |
| Human CLMF^{b} (u/ml) | Human rIL-2 (u/ml) | 20/l | 5/l |
| 0 | 0 | 10 ± 0.6 | 5 ± 0.4 |
| 40 | 0 | 31 ± 0.4 | 14 ± 0.5 |
| 8 | 0 | 23 ± 2.1 | 12 ± 0.4 |
| 1.6 | 0 | 15 ± 0.3 | 10 ± 0.6 |
| 0.3 | 0 | 12 ± 1.2 | 9 ± 0.2 |
| | | | |
| 0 | 1 | 13 ± 0.4 | 6 ± 0.5 |
| 40 | 1 | 33 ± 2.0 | 17 ± 0.5 |
| 8 | 1 | 26 ± 0.8 | 13 ± 1.9 |
| 1.6 | 1 | 19 ± 1.1 | 11 + 2.1 |
| 0.3 | 1 | 16 ± 1.0 | 10 ± 1.5 |
| | | | |
| 0 | 5 | 20 ± 1.3 | 13 ± 0.6 |
| 40 | 5 | 23 ± 2.0 | 12 ± 1.5 |
| 8 | 5 | 29 ± 1.1 | 16 ± 0.7 |
| 1.6 | 5 | 27 ± 1.2 | 13 ± 0.8 |
| 0.3 | 5 | 24 ± 1.8 | 13 ± 1.2 |
| | | | |
| 0 | 25 | 38 ± 1.4 | 19 ± 0.7 |

| | | | |
|---|---|---|---|
| ^{a} Each value represents the mean ± 1 S.E.M. of quadruplicate determination. The spontaneous ⁵¹Cr release was 9%. ^{b} Purified human CLMF from Mono Q FPLC. | | | |

In addition to its ability to enhance the lytic activity of nonspecific NK/LAK cells, CLMF also facilitated specific human cytolytic T lymphocyte (CTL) responses in vitro. CLMF increased the specific allogeneic CTL response to weakly immunogenic, gamma-irradiated HT144 melanoma cells (Table 11). In combination with a low concentration of rIL-2, CLMF also facilitated specific allogeneic human CTL responses to uv-irradiated HT144 melanoma cells, which did not elicit any detectable CTL response in the absence of added cytokines (Table 11). The specificity of the cytolytic effector cells generated in these studies was demonstrated by their ability to cause substantial lysis of ⁵¹Cr-labeled HT 144 melanoma cells but little or no lysis of K562 cells. In contrast, LAK cells which were generated in the same experiments by incubating low density lymphocytes with rIL-2 in the absence of hydrocortisone lysed the K562 cells to a much greater extent than HT144 melanoma cells. For further discussion of the specificity and identity of the cytolytic effector cells generated in assays such a those shown in Table 11 [see Gately et al., J. Immunol. 136: 1274-1282 (1986)].

**TABLE 11**

| **PURIFIED HUMAN, CLMF ENHANCES SPECIFIC HUMAN CYTOLYTIC T LYMPHOCYTE RESPONSES TO ALLOGENEIC MELANOMA CELLS IN VITRO** | | | | | | | | |
|---|---|---|---|---|---|---|---|---|
| Contents of Cultures: | | | | | % Specific ⁵¹Cr Release from ^{a} | | | |
| Lymphocytes | Hydrocortisone | Melanoma | rIL-2 | CLMF | Expt. 1. | | Expt. 2 | |
| (Percoll) fraction^{b}) | (M) | Cells^{c} | (u/ml) | (u/ml) | HT144 | K562 | HT144 | K562 |
| 4 | 10⁻⁴ | | | | 6 ± 3 | -4 ± 1 | -2 ± 2 | 3 ± 4 |
| 4 | 10⁻⁴ | | 7.5 | 10 | -3 ± 1 | -1 ± 1 | -5 ± 1 | 4 ± 1 |
| | | | | | | | | |
| 4 | 10⁻⁴ | HTᵤᵥ | | | 0 ± 2 | -3 ± 1 | -2 ± 2 | 2 ± 1 |
| 4 | 10⁻⁴ | HTᵤᵥ | | 10 | 7 ± 2 | -1 ± 1 | 13 ± 3 | -4 ± 1 |
| 4 | 10⁻⁴ | HTᵤᵥ | 7.5 | | 5 ± 1 | 3 ± 1 | 1 ± 3 | 3 ± 3 |
| 4 | 10⁻⁴ | IITᵤᵥ | 7.5 | 10 | 20 ± 3 | 10 ± 1 | 27 ± 9 | -6 ± 1 |
| | | | | | | | | |
| 4 | 10⁻⁵ | HT_{γ} | | | 17 ± 3 | -4 ± 1 | 10 ± 1 | 3 ± 1 |
| 4 | 10⁻⁵ | HR_{γ} | | 10 | 39 ± 3 | -2 ± 2 | 33 ± 6 | 5 ± 3 |
| | | | | | | | | |
| 1 + 2 | | | 15 | | 33 ± 4 | 67 ± 3 | 19 ± 1 | 47 ± 1 |

| | | | | | | | | |
|---|---|---|---|---|---|---|---|---|
| ^{a}In both experiments the contents of duplicate cultures were pooled, washed, resuspended in 1.2 ml TCM, and assayed for lytic activity undiluted and at 1:5 dilution. In experiment 1, the data shown were obtained using the 1:5 dilution of lymphocytes in the cytolytic assay, corresponding to a lymphocyte: target ratio of approximately 4:1. In experiment 2, significant lysis was seen only when lymphocytes were added undiluted to the lytic assay, and these data are shown in the table. The spontaneous ⁵¹Cr release from HT144 cells was 25% and 31% in experiments and 2, respectively, and for K562 was 18% and 27% in experiments and 2, respectfully. ^{b}Percoll Fraction 4 contained high density lymphocytes recovered froth the interface between the 45% and 58% Percoll layers, whereas Percoll fraction 1 + 2 contained lower density lymphocytes harvested from the interfaces between the 35% and 38% and between the 38% and 41% Percoll layers. Percoll fraction 4 contained CTL precursors but few LAK precursors; on the other hand, fraction 1 + 2 was rich in LAK cell precursors. ^{c}IITᵤᵥ and HT_{γ} represent HT144 melanoma cells which had been uv-irradiated or gamma-irradiated, respectively. | | | | | | | | |

Our results demonstrate that purified human CLMF by itself caused proliferation of activated human T lymphocytes, enhanced the cytolytic activity of human NK cells, and augmented human CTL responses. These activities of CLMF, which are similar to those of IL-2, suggest that CLMF, like IL-2, should have immunoenhancing and antitumor effects when used as a single therapeutic agent in vivo. Clearly, CLMF may also have utility in stimulating the growth in vitro of NK/LAK cells and of activated T cells, such as may be derived from tumor infiltrating lymphocytes [Topalian et al., J. Immunol. 142: 3714-3725 (1989)]. In addition, purified CLMF synergized with low concentrations of rIL-2 in causing the generation of human LAK cells in culture and acted additively or synergistically with rIL-2 in facilitating specific CTL responses in vitro. These results suggest that the use of CLMF in combination with rIL-2 might constitute a more optimal antitumor therapy.

### CLONING OF A cDNA CODING FOR THE 40 kDa SUBUNIT OF HUMAN CLMF

### 1) Cell culture and isolation of polyA+ RNA

NC 37 cells (subclone 98) were grown in roller bottles as described above and induced with PMA and calcium ionophore for 15.5 hours. The cells were harvested, resulting in a frozen cell pellet of 1.11 grams comprising about 5.25 x 10⁸ cells. A portion of the culture was continued for 3 days, at which point the bioassay titer for CLMF activity read 2200 units/ml, indicating that the cells harvested for isolation of RNA had indeed produced the CLMF activity. Total RNA was isolated from the frozen cells by standard procedures and polyA+ RNA was obtained by affinity chromatography. The yield of polyA+ RNA was 2.5% (w/w) relative to the total amount of RNA input.

### 2) Establishment of a cDNA library

2 µg of the above polyA+ RNA were reverse transcribed into cDNA using 150 ng random hexamers as primers. A library in lambda gt10 was established, and 1.5 x 10⁵ clones were amplified for the screening.

### 3) Use of PCR to Generate a DNA Probe Specific for the 40 kDa CLMF Subunit cDNA

The partial N-terminal sequence of the purified 40 kDa protein is IWELKKDVYVVELDWYPDAP.... Two primers for use in mixed primer PCR were designed and synthesized by standard procedures. The forward primer was designed as the coding strand corresponding to amino acids ELKKD in the above sequence, containing all possible codons for that sequence and having an extension at its 5' end including an EcoR1 site and three additional bases adding stability. The sequence of the forward primer is thus 5' ctc gaa ttc gaa/g c/ttn aaa/g aaa/g ga, i.e. a 23mer with 64 different sequences. The reverse primer was designed in the same manner, to represent the antisense strand corresponding to the amino acid sequence YPDAP in the partial N-terminal 40 kDa sequence. The reverse primer thus has the sequence 5' ctc gaa ttc ngg ngc a/gtc ngg a/gta and is a 24 mer containing 256 different sequences. The symbol n stands for any one of the four possible bases a,g,c or t. The primers thus define an amplicon of 72 basepairs in length. After cutting with EcoRl for generating cohesive ends for subcloning, the amplicon size drops to 64 basepairs. Single-stranded cDNA was generated for use in the PCR as described in section 2 above, using polyA+ RNA from induced and, as a control, uninduced cells. 40 ng of either one of those cDNAs were amplified with forward and reverse primers in 100 µl of 10 mM Tris-HCl pH 8.3/50 mM KCl/1.5 mM MgCl₂/0.01 % gelatine/200 µM each of the four nucleotides/10 units Taq-polymerase/250 pmoles of each primer. The PCR parameters were as follows: initial denaturation was at 95°C for 7 minutes. Low stringency annealing was performed by cooling to 37°C over 2 minutes, incubating 2 minutes at 37°C, heating to 72°C over 2.5 minutes, extending at 72°C for 1.5 minutes, heating to 95°C over 1 minute and denaturing at 95°C for 1 minute; this low stringency annealing cycle was repeated once. Afterwards, 30 standard cycles were run as follows: 95°C for 1 minute, 55°C for 2 minutes, 72° C for 2 minutes. A final extension was performed at 72°C for 10 minutes. 10% of the total samples were run on a 4% agarose gel, stained and analyzed. The amplicon of the expected size was only detectable in the sample where induced cDNA had been amplified. The remainder of the sample was extracted with phenol, concentrated by precipitation with ethanol and redissolved in 42 µl of water. The sample was digested with 60 units of the restriction enzyme EcoR1 in 50 µl at 37°C for 2 hours. The sample was subsequently run on a 6% polyacrylamide gel and the 64 bp amplicon was cut out of the gel and eluted by standard procedures. The DNA amplicon was subcloned into the EcoR1 site of the bluescript SK+ plasmid by standard procedures (Stratagene, La Jolla, CA). Colonies obtained from the transformation of the E.coli strain DH5 (obtainable from ATCC) were picked and analyzed for the presence of the 64 bp insert (other E.coli strain compatible with bluescript SK+ plasmids may be used). Two positive candidates were sequenced to determine the sequence of the cloned amplicon. It is clear from this analysis that the correct fragment was amplified, since the deduced amino acid sequence matches exactly the partial amino terminal amino acid sequence from the purified 40 kDa protein. This information was subsequently used to design a 54 bp long oligonucleotide probe that could be used for screening of the cDNA library. Two oligos were designed, with the following sequence: 5' gag cta aag aaa gat gtt tat gtc gta gaa ttc gat and 5' agg ggc atc cgg ata cca atc caa ttc tac gac ata. These two oligos are partially complementary to form the following structure: 
5' gagctaaagaaagatgtttatgtcgtagaattggat 3'
3' atacagcatcttaacctaaccataggcctacgggga 5'
 Such a structure can be labelled by using Klenow fragment and labelled nucleotides such that a high specific activity probe results for the screening of cDNA libraries.

### 4) Screening of cDNA Libraries

A total of 3 x 10⁵ clones from the amplified library were screened on 6 duplicate filters under the following conditions: 50 ml of 5 x SSC/10 x Denhardts/100 µg/ml denatured calf thymus DNA/20% formamide/0.1% SDS/1.5 x 10⁶ cpm of labelled 54 mer at 37°C for 16 hours. The filters were subsequently washed in 2 x SSC at 42°C for 30 minutes, dried and exposed to X-ray film. After overnight exposure with an intensifying screen, 16 possible positives were picked and further analyzed by a second screening round. 10 rehybridizing phage were isolated and their DNA prepared. 8 of those 10 isolates looked identical, upon EcoR1 cutting releasing two fragments of 0.8 kb and 0.6 kb length, indicating a possible internal EcoR1 site. Upon blotting and hybridization with the screening probe, only the 0.6 kb fragment showed hybridization. The two fragments were subcloned separately into the EcoR1 site of the bluescript SK+ plasmid as described above and were completely sequenced. This analysis showed that both fragments align in one contiguous cDNA of about 1.4 kb in length with a naturally occurring internal EcoR1 site, since both fragments upon translation showed the presence of reading frames coding for tryptic peptides that had actually been isolated from purified 40 kDa protein. The complete sequence of the 40 kDa subunit as deduced from the cDNA is shown in Figure 25. The cDNA codes for one open reading frame of 328 amino acids. The protein starts with the initiating Met, followed by another 21 amino acids that make up a classical hydrophobic signal peptide. The N-terminus of mature purified 40 kDa subunit, i.e. IWELKKD..., follows immediately after the signal sequence. The mature protein thus consists of 306 amino acids. The deduced protein sequence contains 4 possible N-linked glycosylation sites, two of which are present in isolated and sequenced tryptic peptides. One of these two sites is used in vivo for the attachment of a carbohydrate side chain. The calculated molecular weight of the mature unglycosylated protein is 34699, the pI is 5.24. The corresponding mRNA is 2.4 kb in length and is detectable in a northern blot in steady state RNA only from induced cells.

### CLONING OF A cDNA CODING FOR THE 35 kDa SUBUNIT OF HUMAN CLMF

Cell culture, isolation of mRNA and establishment of a cDNA library were carried out as described earlier for the cloning of the 40 kDa subunit.

### Use of mixed primer PCR to generate a DNA probe specific for the 35 kDa subunit cDNA

The partial N-terminal sequence of the purified 35 kDa subunit is ?NLPVATPDPGMFP?LHHSQNLLRAV.... Two primers for use in mixed primer PCR were generated by standard procedures. The forward primer was designed as the coding strand corresponding to the amino acids DPGMF in the above sequence, containing all possible codons for that sequence and having an extension at its 5' end including an EcoR1 site and three additional bases adding stability. The sequence of this forward primer was thus 5' CTC GAA TTC GAT/C CCN GGN ATG TT -3', i.e. a 23 mer with 32 different sequences. The reverse primer was designed in the same manner, to represent the antisense strand corresponding to the amino acids NLLRA in the partial N-terminal sequence. The reverse primer had the sequence 5' CTC GAA TTC NGC NGG/T NAA/G NAA/G A/GTT, i.e a 24mer with 4096 different sequences. In both primer sequences, N stands for all 4 bases.The two primers thus defined an amplicon 69 bases long. After cutting with EcoR1 for generating cohesive ends for subcloning, the amplicon size drops to 61 bases. About 3 µg of human genomic DNA were amplified with forward and reverse primers in 50 µl of 10 mM Tris-HCl pH 8.3 / 50 mM KCl / 1.5 mM MgCl₂ / 0.01% gelatine / 200 µM each of the four nucleotides / 2.5 units of Taq polymerase / 64 pmoles of forward and 2048 pmoles of reverse primer (to compensate for the greatly differing complexities of the two primers). The PCR cycling parameters were as follows: initial denaturation was at 95°C for 7 minutes. Low stringency annealing was performed by cooling to 37°C over 2 minutes, incubating at 37°C for 2 minutes, heating to 72°C over 2.5 minutes, extending at 72°C for 1.5 minutes, heating to 95°C over 1 minute and denaturing at 95°C for 1 minute; this low stringency annealing cycle was repeated once. Afterwards, 40 standard cycles were run as follows: 95°C for 1 minute, 55°C for 2 minutes and 72°C for 3 minutes. A final extension was performed at 72°C for 10 minutes. About 20% of the samples were run on a 6% polyacrylamide gel and an amplicon of the expected size was detected after staining the gel with ethidium bromide. The remainder of the sample was extracted with phenol, concentrated by precipitation with ethanol and redissolved in 17 µl of water. The sample was digested with 20 units of EcoR1 enzyme in 20 µl for 60 minutes at 37°C . The sample was subsequently fractionated on an 8% polyacrylamide gel and the 61 basepair amplicon was cut out of the gel and eluted by standard procedures. The DNA amplicon was subcloned into the EcoR1 site of the Bluescript plasmid SK+ (Stratagene, La Jolla, CA) by standard procedures. Colonies obtained from the transformation of the E.coli strain DH5 were analyzed for the presence of the 61 basepair insert. Two candidates were sequenced to determine the sequence of the subcloned amplicon. One of the two clones contained the correct sequence, since translation of that sequence resulted in the amino acid sequence expected from the purified protein. Based on this information, two synthetic oligonucelotides were designed, with the following sequences: 
5'gatccgggaatgttcccatgccttcaccactccc 3'
3'gtacggaagtggtgagggttttggaggatgcccga 5'
 Such a structure can be labelled using radiolabelled nucleotides with the Klenow fragment to very high specific activities for library screening.

### Screening of a cDNA library:

A total of 10⁶ clones from the amplified 16 hours library were screened on 40 duplicate filters with the above probe under the following conditions: 400 ml of 5 X SSC / 20% formamide / 10 X Denhardts / 100 µg/ml denatured calf thymus DNA / 0.1% SDS / 3.8 x 10⁷ cpm labelled probe at 37°C overnight. The filters were subsequently washed in 2 X SSC at 40°C and exposed to X-ray film with a screen overnight. Six potential positives were picked from this first round of screening and analyzed by a second round of plaque hybridization, as above. One clone was picked for the final analysis. Upon preparing phage DNA, the clone was found to contain two EcoR1 fragments of about 0.8 kb and 0.3 kb in size. The two fragments were subcloned separately into the Bluescript SK+ plasmid and sequenced. This analysis showed that the two fragments align into one contigous sequence of about 1.1 kb total length with a naturally occurring internal EcoR1 site. The complete sequence of the cDNA and the deduced amino acid sequence for the 35 kDa CLMF subunit are shown in Figure 26. The cDNA codes for an open reading frame of 219 amino acids, starting with the initiating Met at position 1. The following 21 amino acids constitute a classical hydrophobic signal sequence. Immediately following the signal peptide, the N-terminus of the mature 35 kDa protein starts with the sequence RNLPVAT.... Purified 35 kDa protein had yielded the sequence ?NLPVAT.... The mature 35 kDa protein thus consists of 197 amino acids, containing three possible N-linked glycosylation sites and 7 Cys-residues. The calculated molecular weight of mature unglycosylated protein is 22513, and the pI is 6.09. The corresponding mRNA is 1.4 kb in length and is only detectable in RNA from cells that had been induced for CLMF for at least 6 hours.

### EXPRESSION OF BIOLOGICALLY ACTIVE RECOMBINANT CLMF IN

### COS CELLS

The two subunits for CLMF were engineered for expression in mammalian cells as follows:

### 40 kDa subunit

The two EcoR1 fragments constituting the full length cDNA for the 40 kDa CLMF subunit were ligated to an expression vector similar to pBC12 [see B. Cullen, Meth. Enzymology 152: 684-703 (1987)], except that the cDNA expression is driven off the SV40 early promoter/enhancer. Clones containing the two inserts in the proper orientation to each other were selected by colony hybridization with a synthetic oligonucleotide that spans the internal EcoR1 site in the 40 kDa cDNA. This oligonucleotide has the following sequence: 5' CTG AAG CCA TTA AAG AAT TCT CGG CAG GTG 3'. It was labelled by kinasing using standard procedures. Clones were subsequently analyzed for proper orientation of insert to vector by the polymerase chain reaction procedure, using as forward primer a primer specific for sequences in the SV40 early promoter and as reverse primer an oligonucleotide corresponding to the 40 kDa cDNA sequence positions no. 851-868. Clones with the correct orientation will give a PCR amplicon of 885 bp. Eight out of 20 clones tested gave the predicted fragment, and one was chosen for further study.

### 35 kDa subunit

The full length cDNA for the 35 kDa subunit was amplified out of the original lambda phage by PCR, using primers situated to the left and right of the EcoR1 site in lambda gt10 (primers were New England Biolab Articles No. 1231 and 1232). The resulting PCR amplicon was blunt-end ligated into the EcoRV site of the bluescript plasmid SK+ and the DNA propagated. DNA sequencing showed that the orientation of the cDNA insert within the plasmid was such that the end of the cDNA corresponding to the 5' end of the mRNA was close to the ClaI site in the polylinker. The insert was thus released by cutting with ClaI, filling out this end with T4 DNA polymerase and cutting secondarily with Not I. The resulting fragment was gel-purified and subcloned into an expression plasmid based on the bluescript vector and containing the SV40 early promoter to drive expression of inserted cDNAs. The sites in the expression plasmid used were a blunt-ended PstI site at the 5' end and a Not I site at the 3' end of the cDNA. One clone was chosen for further study after ascertaining its structure by PCR as above for the 40 kDa construct.

### Expression of the two cDNAs in COS-cells

The DNAs for the expression constructs of the 40 kDa and 35 kDa subunits were introduced into COS cells (obtainable from ATCC) on 6 cm diameter plates by the DEAE Dextran transfection procedure (7 x 10⁵ cells/dish plated; 1 µg DNA per dish) described by Cullen [Meth. Enzymology 152: 684 (1987)]. 24 hours after the transfection, the cells were fed with standard tissue-culture medium containing 1% Nutridoma instead of the fetal bovine serum and the supernatants were collected after 40 hours and filtered through a 0.45 µ filter.

Supernatant fluids from cultures of COS cells which had been transfected with cDNA encoding the 35 kDa CLMF subunit or the 40 kDa CLMF subunit or with both cDNAs were tested for CLMF activity in the T cell growth factor assay (Table 13). As shown in the table, COS cells which had been transfected with only one of the subunit cDNAs did not release biologically active CLMF into the culture fluid. However, COS cells which had been transfected with both subunit cDNAs produced biologically active CLMF. By comparing the amount of lymphoblast proliferation induced by the culture fluid from doubly transfected COS cells to the amount of proliferation induced by purified NC-37-derived CLMF, the concentration of CLMF activity in the culture fluid was estimated to be 374 units/ml. Assuming a specific activity of 8 x 10⁷ units/mg CLMF protein, this result suggests that the fluid from cultures of doubly transfected COS cells contained approximately 4.7 ng/ml of recombinant CLMF.

**TABLE 13**

| T Cell Growth Factor Activity of Recombinant CLMF Expressed in COS cells | | |
|---|---|---|
| Cytokine added: | Concentration: | ³H-Thymidine Incorporated by PHA-Activated Lymphoblasts (mean cpm ± 1 S.E.M.) |
| None | | 14,581 ± 343 |
| | | |
| Natural CLMF* | 200 units/ml | 79,848 ± 854 |
| Natural CLMF | 40 units/ml | 59,093 ± 2029 |
| Natural CLMF | 8 units/ml | 39,180 ± 545 |
| Natural CLMF | 1.6 units/ml | 25,996 ± 763 |

| Supernatant fluid from cultures of COS cells transfected with: | | |
|---|---|---|
| 35 kDa CLMF subunit cDNA | 1/5 dilution | 15,332 ± 797 |
| 35 kDa CLMF subunit cDNA | 1/25 dilution | 12,149 ± 379 |
| | | |
| 40 kDa CLMF subunit cDNA | 1/5 dilution | 14,883 ± 1039 |
| 40 kDa CLMF subunit cOMA | 1/25 dilution | 13,889 ± 110 |
| | | |
| 35 kDa + 40 kDa CLMF subunit cDNAs | 1/5 dilution | 66,228 ± 166 |
| 35 kDa + 40 kDa CLMF subunit cDNAs | 1/25 dilution | 47,873 ± 275 |
| | | |
| Mock transfected | 1/5 dilution | 14,368 ± 628 |
| Mock transfected | 1/25 dilution | 14,426 ± 173 |

| | | |
|---|---|---|
| *Purified NC-37-derived CLMF from Mono Q FPLC. | | |

### ANTI-CLMF HYBRIDOMAS AND ANTIBODIES

### Preparation, Characterization and Purification of anti-CLMF Hybridomas

Lewis rats (Charles River Laboratories, Wilmington, MA) were initially immunized by the intraperitoneal route (i.p.) with partially purified CLMF mixed with an equal volume of Freund's complete adjuvant (Gibco). The rats were injected i.p. with booster immunization of CLMF mixed with Freund's incomplete adjuvant (Gibco) according to the schedule in Table 14. For preparation of activated spleen cells, one rat was injected i.v. with partially purified CLMF on two successive days starting 4 days prior to the cell fusion (Table 14). Spleen cells were isolated from this rat and fused with NSO cells [Calfre et al., Meth. Enzymol. 73:3-46 (1981)] at a ratio of 1:1 (spleen cells : NSO cells) with 35% polyethylene glycol (PEG 4000, E. Merck). Other cells suitable as fusion partners in hybridoma cell fusion may be used instead of NSO cells. The fused cells were plated at a density of 5 x 10⁴ cells/well/ml in 48 well plates in IMDM [Iscove et al., J. Exp. Med. 147:923-933 (1978)] supplemented with 15% fetal bovine serum (FBS), glutamine (2 mM), beta-mercaptoethanol (0.1 mM), gentamycin (50 µg/ml). HEPES (10 mM) and 15% P388D1 cell supernatant (P388D1 cells are obtainable from ATCC). Hybridoma supernatants were screened for specific CLMF antibodies in 4 assays: 1) immunoprecipitation of ¹²⁵I-labelled CLMF, 2) immunodepletion of CLMF bioactivity, 3) western blotting with CLMF and 4) inhibition of ¹²⁵I-CLMF binding to PHA-activated PBL blast cells. Hybridoma cell lines secreting anti-CLMF antibodies were cloned by limiting dilution. Antibodies were purified from large scale hybridoma cultures or ascites fluids by affinity chromatography on protein G bound to cross-linked agarose according to the manufacturer's protocol (Gammabind G, Genex, Gaithersburg, MD).

**TABLE 14**

| Immunization Schedule: | | | | | |
|---|---|---|---|---|---|
| Date | CLMF (10⁸ units/mg) | | Total Protein | Spec. Activity | Purity |
| | units | µg | (µg) | (U/mg) | (%) |
| 3/28/89 | 1x10⁴ | 0.1 µg | 15 | 6.7x10⁵ | 6.7 |
| 4/10/89 | 1.2x10⁴ | 0.1 µg | ? | 6x10⁵ | 0.6 |
| | | | | | |
| 5/3/89 | 1st bleed | | | | |
| | | | | | |
| 5/18/89 | 2.2x10⁵ | 2 µg | 75 | 2.9x10⁶ | 2.9 |
| | | | | | |
| 6/7/89 | 2nd bleed | | | | |
| | | | | | |
| 6/29/89 | 6.3x10⁴ | 0.63 µg | 83 | 7.5x10⁵ | 0.75 |
| 7/21/89 | 1.2x10⁵ | 1.2 µg | 24 | 5x10⁶ | 5.0 |
| | | | | | |
| 8/2/89 | 3rd bleed | | | | |
| | | | | | |
| 10/19/89 | 2.1x10⁶ | (i.v.) | | | |
| 10/20/89 | 2.1x10⁶ | (i.v.) | | | |
| 10/23/89 | Fusion | | | | |

Isolation and Identification of Monoclonal Antibodies Specific for CLMF. Serum isolated at the 3rd bleed from the rat immunized with partially purified CLMF (Table 14) neutralized CLMF bioactivity (5 units/ml) as determined in the TGF assay (Fig. 27). This neutralization could be blocked by adding excess CLMF (200 units/ml) demonstrating that the neutralization by the antiserum was specific for CLMF (Fig. 27). Normal rat serum did not neutralize CLMF bioactivity (Fig. 27). Spleen cells isolated from this rat were fused with NSO cells and the resulting hybridomas were initially screened for CLMF-specific antibodies by immunoprecipitation of ¹²⁵I-labelled CLMF.

The radioiodinated partially purified CLMF preparation contains predominantly the CLMF 75 kDa heterodimer, a small amount of the free CLMF 40 kDa subunit and two other proteins of approximately 92 kDa and 25 kDa (Fig. 28). The ¹²⁵I-labelled CLMF preparation retained CLMF bioactivity in the TGF assay, indicating that the labelling procedure did not significantly alter the configuration of the CLMF molecule. The CLMF immunized rat serum immunoprecipitated the 75 kDa heterodimer and the free 40 kDa subunit (Lanes 6 and 8, Fig. 28) whereas normal rat serum did not immunoprecipitate these radiolabelled proteins (Lanes 7 and 9, Fig. 28). Four individual monoclonal antibodies also immunoprecipitated the 75 kDa heterodimer and the free 40 kDa subunit (Fig. 28) but did not immunoprecipitate the 92 kDa or 25 kDa labelled proteins. The immunoprecipitation assay identified twenty hybridomas which secreted anti-CLMF antibodies (Table 15). All the antibodies immunoprecipitated the radiolabelled 75 kDa heterodimer and the free 40 kDa subunit as determined by SDS/PAGE and autoradiography (data shown for 4 representative antibodies in Fig. 28).

**Table 15: Monoclonal Anti-CLMF Antibodies (40 kDa Subunit Specific)**

| Antibody | Western Blot | | ¹²⁵I-CLMF/Receptor Assay (% Inhibition) | Neutralization of Bioactivity |
|---|---|---|---|---|
| | Red. | N.R. | | |
| Inhibitory/Neutralizing | | | | |
| 7B2 | - | ++ | 95 | + |
| 2A3 | - | ++ | 99 | + |
| 1B8 | - | +/- | 60 | ND |
| 1C1 | - | ++ | 81 | ND |
| 4A1 | - | + | 98 | + |
| 4C8 | ND | ND | 68 | ND |
| 4D1 | - | + | 100 | ND |
| 6A2 | - | +/- | 75 | ND |
| 7A1 | +/- | ++ | 94 | ND |
| 8A1 | - | + | 99 | ND |
| 8A2 | - | ++ | 83 | ND |
| 9C8 | - | + | 62 | ND |
| 22E7 | ++ | ++ | 91 | ND |

| Non-inhibitory/Non-Neutralizing | | | | |
|---|---|---|---|---|
| 8E3 | + | ++ | 35 | - |
| 9F5 | + | ++ | 18 | ND |
| 4A6 | - | - | 17 | ND |
| 6A3 | - | + | 20 | - |
| 8C4 | - | ++ | 33 | ND |
| 8E4 | - | ++ | 1 | ND |
| 39B3 | ND | ND | 46 | ND |
| control | - | - | 12 | - |

| | | | | |
|---|---|---|---|---|
| 1: Western blots: N.R. is non-reduced and Red. is reduced SDS/PAGE For the western blots, a CLMF sample containing 5% 75 kDa heterodimer and 95% free 40 kDa subunit were separated on 10% SDS/PAGE and western blots prepared as described in methods. The blots were scored as strongly positive (++), positive (+), weakly positive (+/-) and negative (-). 2. CLMF receptor binding assay: An antibody was considered inhibitory if it would block more than 60% of radiolabelled CLMF binding to the PHA activated PBL blasts. 3. Neutralization of CLMF bioactivity as assessed by the TGF assay: An antibody was considered neutralizing if it would block more more than 50% proliferation at 200 µg/ml. The results are presented as positive (+) or negative (-). 4: ND: Not Determined | | | | |

After initially identifying specific CLMF antibodies in the immunoprecipitation assay, the antibodies were tested for their ability to immunodeplete CLMF bioactivity as assessed by the TGF and LAK cell induction assays. Increasing amounts of CLMF cause a dose dependent increase in the proliferation of PBL blasts in the TGF assay as measured by the incorporation of ³H-thymidine into the dividing blast cells (Fig. 29). Immunodepletion of CLMF activity by immobilized anti-CLMF antibodies occurs in a dose dependent manner (Fig. 29). Aliquots (0.4 and 0.1 ml) of hybridoma supernatant solution will completely deplete 50 and 200 units/ml of CLMF activity from the culture medium. 0.025 ml of supernatant solution will completely deplete 50 units/ml but only approximately 50% of 200 units/ml. 0.0062 ml of hybridoma supernatant shows even less depletion of 50 and 200 units/ml of CLMF. An aliquot (0.4 ml) of an anti-IL-1 receptor antibody supernatant solution shows no immunodepletion of CLMF bioactivity.

Increasing amounts of CLMF also cause a dose dependent increase in the lysis of target cells by LAK cells as measured by the release of ⁵¹Cr in the LAK cell induction microassay (Fig. 30). The immobilized anti-CLMF antibodies also deplete in a dose dependent manner the CLMF activity in the LAK cell induction assay (Fig. 30). These data confirm that the antibodies which immunoprecipitate the 75 kDa labelled protein from the radiolabelled partially purified CLMF preparation are specific for CLMF. The data also demonstrate that the radiolabelled 75 kDa protein is the protein responsible for CLMF bioactivity.

### Methods for Assay of Monoclonal Antibodies

### Purification of CLMF and Labelling of CLMF with ¹²⁵I.

CLMF was partially purified from cell supernatants harvested from human peripheral blood lymphocytes (PHLs) or NC-37 cells as described previously. Partially purified CLMF was labelled with ¹²⁵I by a modification of the Iodogen method. Iodogen (Pierce Chemical Co.) was dissolved in chloroform at a concentration of 0.5 mg/ml and 0.1 ml aliquots were added to 12x75 borosilicate glass tubes. The chloroform was evaporated under a stream of nitrogen and the Iodogen was dried in the center of the bottom of the glass tube. The coated tubes were stored in a dessicator at room temperature (RT) under vacuum. For radiolabeling, 0.5-1.0 mCi ¹²⁵I-Na (Amersham) was added to an Iodogen coated tube containing 0.50 ml of Tris-Iodination Buffer (25 mM Tris-HCl pH 7.5, 0.4 M NaCl, 1 mM EDTA) and incubated for 4 minutes at RT. The activated ¹²⁵I solution was transferred to a 1.5 ml tube containing 0.05-0.1 ml CLMF (approximately 5 µg in 0.125 M NaCl, 20 mM Tris-HCl pH 7.5) and the reaction mixture was further incubated for 8 minutes at RT. At the end of the incubation, 0.05 ml of Iodogen Stop Buffer (10 mg/ml tyrosine, 10% glycerol in Dulbecco's phosphate buffered saline (PBS) pH 7.4) was added and reacted for 30 seconds. The mixture was then diluted with 1.0 ml Tris-Iodination Buffer and applied to a BioRad BioGel P10DG (BioRad Laboratories) desalting column for chromatography. The column was eluted with Tris-Iodination Buffer and fractions (1 ml) containing the peak amounts of labelled protein were combined and diluted to 1 x 10⁸ cpm/ml with 0.25% gelatin in Tris-Iodination buffer. The TCA precipitable radioactivity (10% trichloroacetic acid final concentration) was typically in excess of 95% of the total radioactivity. The radiospecific activity ranged from 6000 cpm/fmol to 10,000 cpm/fmol.

Immunodepletion of CLMF. Hybridoma culture supernatants or purified monoclonal antibodies were tested for their ability to immunodeplete CLMF as follows. Goat anti-rat IgG-agarose beads (Sigma Chemical Co.. St. Louis, MO) were washed three times with 10 ml of PBS (Gibco) supplemented with 1% bovine serum albumin (BSA) (Sigma) (PBS/BSA solution). After washing, the beads were resuspended in PBS/BSA at a final concentration of 50% vol/vol. Aliquots (0.2 ml) of the bead suspension were added to 1.5 ml Eppendorf tubes, together with the indicated amounts of monoclonal antibodies or hybridoma supernatant solutions. The volume of each mixture was brought to 1.4 ml by the addition of hybridoma maintenance medium [Iscove's modification of Dulbecco's medium (IMDM) with 0.1% fetal bovine serum (FBS), 10% Nutridoma-SP (Boehringer-Mannheim), and 2 mM L-glutamine], and the mixtures were then incubated for 2 hours at room temperature on a hematology/chemistry mixer. Following this incubation, the tubes were centrifuged in a Beckman microfuge 12 (1.5 minutes at setting 5), and the supernatants were discarded. The beads were again washed three times with PBS/BSA and then resuspended in 1 ml of tissue culture medium (TCM) containing 5% human AB serum and the indicated concentration of purified human CLMF. The tubes were subsequently incubated overnight at 4°C on the mixer. Following this, the beads were removed by centrifugation in the microfuge, and the resulting immunodepleted supernatant solutions were assayed for residual CLMF activity in the TGF assay or in the microassay for LAK cell induction.

Immunoprecipitation Assay. For the immunoprecipitation reaction, 0.05 to 0.5 ml of hybridoma supernatant, diluted antisera or purified IgG was added to a 1.5 ml microfuge tube containing 0.1 ml of a 50% suspension of goat-anti-rat IgG coupled to agarose (Sigma Chemical Co.). The assay volume was brought up to 0.5 ml with RIPA Buffer (50 mM NaPO₄ pH 7.5, 150 mM NaCl, 1% Triton-X 100, 1% deoxycholic acid, 0.1 % SDS, 1% BSA, and 5 mM EDTA) and the mixture was incubated on a rotating mixer for 2 hours at RT. The beads were pelleted by centrifugation for 1 minute at 12,000 x g and then resuspended in 1 ml RIPA Buffer containing ¹²⁵I CLMF (1 x 10⁵ cpm). The mixture was then incubated on a rotating mixer for 16 hours at 4°C. Following this incubation, the beads were pelleted by centrifugation and washed twice in RIPA without BSA. The beads were then washed once with 0.125 M Tris-HCl pH 6.8 and 10% glycerol. The ¹²⁵I-CLMF bound to the solid phase antibodies was released by adding 10 µl of 2 X Sample Buffer (Laemmli, supra) with and without 5% β-mercaptoethanol and heating for 3 minutes at 95°C. The immunoprecipitated ¹²⁵I-CLMF was analyzed by SDS-PAGE on a 10% or 12% polyacrylamide gel and visualized by autoradiography.

CLMF Receptor Binding Assay. The ability of hybridoma supernatant solutions, purified IgG or antisera to inhibit the binding of ¹²⁵I-CLMF to PHA-activated human T lymphoblasts was measured as follows: 0.1 ml aliquots of serial dilutions of culture supernatants, purified IgG or antisera were mixed with 0.025 ml aliquots of Binding Buffer (RPMI-1640, 5% FBS, 25 mM HEPES pH 7.4) containing ¹²⁵I-CLMF (1 x 10⁵ cpm). The mixture was incubated on an orbital shaker for 1 hour at RT, then 0.025 ml of activated blasts (5 x 10⁷ cells/ml) was added to each tube. The mixture was further incubated for 1 hour at RT. Non-specific binding was determined by inclusion of 10 nM unlabelled CLMF in the assay. Incubations were carried out in duplicate or triplicate. Cell bound radioactivity was separated from free ¹²⁵I-CLMF by centrifugation of the assay contents through 0.1 ml of an oil mixture (1:2 mixture of Thomas Silicone Fluid 6428-R15: A.H. Thomas, and Silicone Oil AR 200: Gallard-Schlessinger) at 4°C for 90 seconds at 10,000 x g. The tip containing the cell pellet was excised and cell bound radioactivity was determined in a gamma counter.

**SDS polyacrylamide Gel Electrophoresis (SDS/PAGE) and** Western Blotting. Immunoprecipitated ¹²⁵I-labelled proteins and partially purified CLMF were treated with Laemmli sample buffer (2% SDS, 125 mM Tris-HCl, pH 6.8, 10% glycerol, 0.025% bromphenol blue) with and without 5% β-mercaptoethanol, heated at 95°C for 3 minutes and separated by SDS/PAGE on 7.5% or 12% precast gels (BioRad Laboratories). For the immunoprecipitated ¹²⁵I-labelled proteins, the gels were stained with 0.2% Coomassie Brilliant Blue in 25% isopropyl alcohol and 10% acetic acid, destained in 10% methanol and 10% acetic acid, dried and analyzed by autoradiography. For western blotting, the proteins separated by SDS/PAGE were transferred to nitrocellulose membrane (0.2 µ) for 16 hours at 100 volts in 10 mM Tris-HCl pH 8.3, 76.8 mM glycine, 20% methanol and 0.01% SDS. The nitrocellulose membrane was blocked for 1 hour at 37°C in 3% gelatin, Tris-HCl pH 7.5, 0.15 M NaCl and then probed with hybridoma supernatant solutions or purified antibody diluted in AB buffer (1% bovine serum albumin, 50 mM sodium phosphate pH 6.5, 0.5 M NaCl, 0.05% Tween 20) for 16 hours at 4°C. After washing with wash buffer (PBS, 0.05% Tween 20), the nitrocellulose strips were incubated for 2 hours at room temperature with goat anti-rat IgG antibody coupled to peroxidase (Boehringer Mannheim Biochemicals) diluted in AB buffer. The nitrocellulose membrane was washed with wash buffer and the bound antibody visualized by incubation for 30 minutes at RT with 4-chloro-1-napthol (0.5 mg/ml in 0.15% H₂O₂, 0.5 M NaCl, 50 mM Tris-HCl, pH 7.5). The reaction was stopped by extensive washing with distilled water.

Identification of the CLMF Subunit Bound by the Monoclonal Antibodies. CLMF is a 75 kDa heterodimer protein composed of 40 kDa and 35 kDa subunits. Western blot analysis was used to determine if the monoclonal anti-CLMF antibodies recognized the 40 kDa or the 35 kDa subunits. Highly purified 75 kDa CLMF heterodimer was separated by non-reducing SDS/PAGE and transferred to nitrocellulose membrane (Fig. 31). In addition, purified CLMF, which was composed of approximately 95% free 40 kDa subunit and 5% 75 kDa heterodimer, was separated by both non-reducing and reducing SDS/PAGE and the proteins were transferred to nitrocellulose membrane (Fig. 32). Individual nitrocellulose strips containing the non-reduced 75 kDa CLMF heterodimer (Fig. 31), the non-reduced 40 kDa subunit (top panel Fig. 32) and the reduced 40 kDa subunit (bottom panel Fig. 32) were probed with monoclonal anti-CLMF antibodies, control monoclonal antibody, rat anti-CLMF serum and control rat serum. The monoclonal anti-CLMF and rat polyclonal anti-CLMF antibodies bind specifically to an approximately 75 kDa heterodimer on the strips containing non-reduced 75 kDa CLMF while the control antibody preparations do not show this binding activity (Fig. 31). All the monoclonal and rat polyclonal anti-CLMF antibodies recognize the non-reduced 40 kDa subunit (top panel, Fig. 32). However, only the rat polyclonal antiserum and three monoclonal antibodies, 8E3, 9F5 and 22E7, bind to reduced 40 kDa subunit protein (bottom panel, Fig. 32). These data demonstrated that all the monoclonal antibodies were specific for the 40 kDa subunit of CLMF.

Identification of a CLMF Receptor on PHA-Activated Lymphoblasts. The previous data demonstrated that the monoclonal anti-CLMF antibodies immunoprecipitated ¹²⁵I-labelled CLMF, immunodepleted CLMF bioactivity and bound to the 40 kDa subunit of CLMF. However, the antibodies present in the hybridoma supernatant solutions could not be directly tested for their ability to neutralize CLMF bioactivity in the TGF or LAK cell induction assays due to non-specific inhibitory effects of supernatant solutions containing control antibodies. Our previous work with IL-2 monoclonal antibodies demonstrated that antibodies which would block ¹²⁵I-IL-2 binding to IL-2 receptor bearing cells would also neutralize IL-2 bioactivity. Since receptor binding assays are usually unaffected by addition of hybridoma supernatant solutions or other substances, a CLMF receptor binding assay was developed to evaluate the anti-CLMF antibodies for inhibitory/neutralization activity. A CLMF receptor binding assay was configured with ¹²⁵I-labelled CLMF and the PHA-activated peripheral blood lymphoblasts (Fig. 33). The binding of ¹²⁵I-CLMF to the PHA-activated lymphoblasts was saturable and specific (Fig. 33). Scatchard plot analysis [See Scatchard: Ann. N.Y. Acad. Sci. 51: 660-672 (1949)] of the equilbrium binding data indicated that the apparent dissociation constant for ¹²⁵I-CLMF binding to the receptor is approximately 200 pM and that each lymphoblast has about 700-800 receptors. Since the serum from the rat immunized with CLMF showed neutralization of CLMF bioactivity, it was tested for inhibition of ¹²⁵I-CLMF binding to the lymphoblasts (Fig. 34). The rat immune serum blocks 50% of ¹²⁵I-labelled CLMF binding at approximately a 1/500 dilution, while the control rat serum does not show any inhibition at this dilution. With the specificity of the receptor binding assay established, hybridoma supernatant solutions were tested for antibodies which would inhibit ¹²⁵I-CLMF binding to lymphoblasts.

The degree of inhibition of ¹²⁵I-CLMF binding to the lymphoblasts was determined at a 1/2 dilution of each hybridoma supernatant solution (Fig. 35). Twelve hybridoma supernatant solutions inhibited by greater than 60% ¹²⁵I-CLMF binding to the lymphoblasts. The antibodies present in these supernatant solutions have been classified as inhibitory/neutralizing antibodies. Six hybridoma supernatant solutions inhibited ¹²⁵I-labelled CLMF binding by less than 40% and were classified as non-inhibitory/non-neutralizing antibodies. Control antibody inhibited by approximately 10% the ¹²⁵I-CLMF binding to the lymphoblasts.

Three inhibitory antibodies, 7B2, 2A3 and 4A1, and two non-inhibitory antibodies, 6A3 and 8E3, were purified from ascites fluid by protein G affinity chromatography on GammaBind G (Genex, Gaithersburg, MD) columns. Antibodies 4A1, 2A3 and 7B2 inhibit in a dose dependent manner ¹²⁵I-CLMF binding to the lymphoblasts with IC₅₀ concentrations of 0.7 µg/ml. 7 µg/ml and 9.5 µg/ml, respectively (Fig. 36). Antibodies 6A3 and 8E3 do not block ¹²⁵I-CLMF binding at concentrations of 100 µg/ml (Fig. 36). These data demonstrated that the original classification of each antibody as either inhibitory or non-inhibitory was correct.

### Direct Neutralization of CLMF Bioactivity by

Antibodies. To determine if the antibodies classified as inhibitory by the CLMF receptor binding assay would directly neutralize CLMF bioactivity, each inhibitory antibody was tested for neutralizing activity in the TGF assay (Table 16). Two inhibitory antibodies, 4A1 and 7B2, demonstrated a dose dependent neutralization of CLMF bioactivity (40 units/ml) from 0.03 to 100 µg/ml, with IC₅₀ concentrations of approximately 1 µg/ml and 80 µg/ml, respectively. These data confirmed that antibodies inhibiting ¹²⁵I-CLMF binding to the CLMF receptor would also neutralize CLMF bioactivity.

**Table 16: Neutralization of CLMF Bioactivity by Monoclonal Anti-CLMF.**

| Assay Contents: | | | |
|---|---|---|---|
| CLMF^{a} | Antibody^{b} | Total ³H-Thymidine Incorporation | % Neutralization^{c} |
| none | none | 9923 ± 439 | |
| | | | |
| CLMF | none | 25752 ± 592 | |
| CLMF | 4Al | | |
| | 100 µg/ml | 12965 ± 938 | 81 |
| | 20 | 12215 ± 663 | 86 |
| | 4 | 12985 ± 269 | 81 |
| | .8 | 19932 ± 1016 | 37 |
| | .16 | 22379 ± 410 | 21 |
| | .03 | 25405 ± 1093 | 2 |
| | | | |
| CLMF | 7B2 | | |
| | 200 µg/ml | 10763 ± 878 | 96 |
| | 100 | 15083 ± 406 | 67 |
| | 20 | 23690 ± 1228 | 13 |
| | 4 | 25849 ± 1408 | 0 |
| | | | |
| CLMF | Control | | |
| | 200 µg/ml | 27654 ± 1086 | 0 |
| | 100 | 22221 ± 381 | 22 |
| | 20 | 27335 ± 620 | 0 |

| | | | |
|---|---|---|---|
| ^{a} Purified CLMF was used in the TGF assay at a concentration of 40 units/ml. ^{b} Purified antibodies were added at the concentrations indicated in the table. ^{c} Reduction of ³H-thymidine incorporation to the level seen in the absence of added cytokines was considered to be 100% neutralization. | | | |

### Preparation of Antibodies Against a Synthetic Peptide Fragment of the 35,000 dalton Subunit of CLMF

A peptide, comprising amino acids 3-13 of the NH₂-terminal sequence of the 35 kDa CLMF subunit and a COOH-terminal cysteine (L-P-V-A-T-P-D-P-G-M-F-C), was synthesized by solid-phase peptide methodology, purified by HPLC, and conjugated to keyhole limpet hemocyanin via the methylated bovine serum albumin procedure. Two rabbits were immunized intradermally with the conjugated peptide in Freund's complete adjuvant (300 µg peptide/rabbit). Six weeks after immunization, rabbits were boosted with free peptide (100 µg, intravenously) and KLH-conjugated peptide (150 µg, subcutaneously) dissolved in PBS. Serum samples were prepared from bleedings taken 7 days later. The boosting and bleeding schedule was repeated every 4-5 weeks.

Serum samples from the first and second bleedings from each rabbit were evaluated for reaction with the synthetic peptide in a direct ELISA assay. The synthetic, free peptide was coated on microtiter plates at 4 ng/ml and 20 ng/ml, and the plates were washed and blocked with bovine serum albumin. Serum samples were tested at various dilutions (Table 17), and antibody reactivity was detected with the use of a second antibody (HRP-conjugated goat anti-rabbit IgG) with o-phenylenediamine as substrate. Absorbance values were read at 490 nm after addition of H₂SO₄ to stop the reaction. The results indicate that antibody was produced in both rabbits against 35,000 dalton CLMF peptide (Table 17). In separate experiments, we verified that the antibody was specific for the peptide since (a) serum from non-immunized rabbits does not react with the peptide in ELISA, (b) sera from rabbits immunized with the synthetic peptide do not react with a peptide fragment from the 40,000 dalton CLMF subunit and (c) purified IgG from the serum samples also reacts with the synthetic peptide.

A serum sample from one of the rabbits (first bleed) was tested by Western blot analysis for reactivity with 75 kDa CLMF and with the 35 kDa CLMF subunit (Fig. 37). Partially purified CLMF (approximately 120 µg/ml) was run on SDS-PAGE, transferred to nitrocellulose, and treated with a 1:500 dilution of the rabbit anti-CLMF peptide antiserum. Antibody reactivity was detected by use of biotinylated goat anti-rabbit IgG and alkaline phosphatase-conjugated streptavidin. The anti-CLMF peptide antibody was found to react both with nonreduced 75 kDa CLMF protein and with the reduced 35 kDa CLMF subunit (Fig. 37).

Although the antibodies produced in this example were polyclonal, a similar approach could be used to prepare monoclonal antibodies to the 35 kDa subunit of CLMF. The synthetic peptide used in this example or other synthetic peptides based on the amino acid sequence of the 35 kDa CLMF subunit (Fig. 26) could be used to immunize rats. Fusions could be performed and hybridoma cultures screened for the production of monoclonal anti-CLMF antibodies as described above.

**TABLE 17**

| **ELISA ASSAY OF SERUM SAMPLES FROM RABBITS IMMUNIZED AGAINST A KIT-CONJUGATED SYNTHETIC PEPTIDE FROM THE 35,000 DALTON SUBUNIT OF CLMF** | | | | | | | |
|---|---|---|---|---|---|---|---|
| Serum Source | | | | A₄₉₀ | | | |
| Rabbit | Bleed | Free Peptide | Serum Dilutions (fold) | | | | |
| No. | No. | (ng/ml) | 1x10⁴ | 2.5x10⁴ | 6.2x10⁴ | 1.6x10⁵ | 3.9x10⁵ |
| 1 | 1 | 20 | 2.7 | 2.1 | 1.4 | 0.8 | 0.5 |
| 1 | 1 | 4 | 0.7 | 0.4 | 0.3 | 0.2 | 0.1 |
| 1 | 1 | 0 | 0.05 | 0.05 | 0.05 | 0.05 | 0.05 |
| | | | | | | | |
| 1 | 2 | 20 | 2.5 | 1.8 | 1.1 | 0.6 | 0.3 |
| 1 | 2 | 4 | 0.9 | 0.6 | 0.4 | 0.2 | 0.1 |
| 1 | 2 | 0 | 0.05 | 0.05 | 0.05 | 0.05 | 0.05 |
| | | | | | | | |
| 2 | 1 | 20 | 2.7 | 2.2 | 1.6 | 0.9 | 0.5 |
| 2 | 1 | 4 | 0.9 | 0.5 | 0.3 | 0.2 | 0.1 |
| 2 | 1 | 0 | 0.05 | 0.65 | 0.05 | 0.05 | 0.05 |
| | | | | | | | |
| 2 | 2 | 20 | 2.2 | 1.5 | 1.0 | 0.5 | 0.3 |
| 2 | 2 | 4 | 0.7 | 0.4 | 0.2 | 0.1 | 0.1 |
| 2 | 2 | 0 | 0.05 | 0.05 | 0.05 | 0.05 | 0.05 |

## Claims

1. A subunit of the Cytotoxic Lymphocyte Maturation Factor (CLMF) protein **characterized in that**
(a) the subunit comprises the amino acid sequence 
Arg Asn Leu Pro Val Ala Thr Pro Asp Pro Gly MET Phe Pro Cys
Leu His His Ser Gln Asn Leu Leu Arg Ala Val Ser Asn MET Leu
Gln Lys Ala Arg Gln Thr Leu Glu Phe Tyr Pro Cys Thr Ser Glu
Glu Ile Asp His Glu Asp Ile Thr Lys Asp Lys Thr Ser Thr Val
Glu Ala Cys Leu Pro Leu Glu Leu Thr Lys Asn Glu Ser Cys Leu
Asn Ser Arg Glu Thr Ser Phe Ile Thr Asn Gly Ser Cys Leu Ala
Ser Arg Lys Thr Ser Phe MET MET Ala Leu Cys Leu Ser Ser Ile
Tyr Glu Asp Leu Lys MET Tyr Gln Val Glu Phe Lys Thr MET Asn
Ala Lys Leu Leu MET Asp Pro Lys Arg Gln Ile Phe Leu Asp Gln
Asn MET Leu Ala Val Ile Asp Glu Leu MET Gln Ala Leu Asn Phe
Asn Ser Glu Thr Val Pro Gln Lys Ser Ser Leu Glu Glu Pro Asp
Phe Tyr Lys Thr Lys Ile Lys Leu Cys Ile Leu Leu His Ala Phe
Arg Ile Arg Ala Val Thr Ile Asp Arg Val Thr Ser Tyr Leu Asn
Ala Ser,
 and that
(b) if combined with the second subunit of the CLMF protein comprising the amino acid sequence 
Ile Trp Glu Leu Lys Lys Asp Val Tyr Val Val Glu Leu Asp Trp
Tyr Pro Asp Ala Pro Gly Glu MET Val Val Leu Thr Cys Asp Thr
Pro Glu Glu Asp Gly Ile Thr Trp Thr Leu Asp Gln Ser Ser Glu
Val Leu Gly Ser Gly Lys Thr Leu Thr Ile Gln Val Lys Glu Phe
Gly Asp Ala Gly Gln Tyr Thr Cys His Lys Gly Gly Glu Val Leu
Ser His Ser Leu Leu Leu Leu His Lys Lys Glu Asp Gly Ile Trp
Ser Thr Asp Ile Leu Lys Asp Gln Lys Glu Pro Lys Asn Lys Thr
Phe Leu Arg Cys Glu Ala Lys Asn Tyr Ser Gly Arg Phe Thr Cys
Trp Trp Leu Thr Thr Ile Ser Thr Asp Leu Thr Phe Ser Val Lys
Ser Ser Arg Gly Ser Ser Asp Pro Gln Gly Val Thr Cys Gly Ala
Ala Thr Leu Ser Ala Glu Arg Val Arg Gly Asp Asn Lys Glu Tyr
Glu Tyr Ser Val Glu Cys Gln Glu Asp Ser Ala Cys Pro Ala Ala
 
Glu Glu Ser Leu Pro Ile Glu Val MET Val Asp Ala Val His Lys
Leu Lys Tyr Glu Asn Tyr Thr Ser Ser Phe Phe Ile Arg Asp Ile
Ile Lys Pro Asp Pro Pro Lys Asn Leu Gln Leu Lys Pro Leu Lys
Asn Ser Arg Gln Val Glu Val Ser Trp Glu Tyr Pro Asp Thr Trp
Ser Thr Pro His Ser Tyr Phe Ser Leu Thr Phe Cys Val Gln Val
Gln Gly Lys Ser Lys Arg Glu Lys Lys Asp Arg Val Phe Thr Asp
Lys Thr Ser Ala Thr Val Ile Cys Arg Lys Asn Ala Ser Ile Ser
Val Arg Ala Gln Asp Arg Tyr Tyr Ser Ser Ser Trp Ser Glu Trp
Ala Ser Val Pro Cys Ser
 the combined CLMF protein is active in a T cell growth factor assay.

2. The subunit of claim 1, wherein the combined CLMF protein displays a specific activity of at least 5.2 x 10⁷ Units/mg when determined in a T cell growth factor assay, and when combined with the protein as defined in claim 1(b).

3. The subunit of claim 1 or 2 comprising the amino acid sequence

Arg Asn Leu Pro Val Ala Thr Pro Asp Pro Gly MET Phe Pro Cys
Leu His His Ser Gln Asn Leu Leu Arg Ala Val Ser Asn MET Leu
Gln Lys Ala Arg Gln Thr Leu Glu Phe Tyr Pro Cys Thr Ser Glu
Glu Ile Asp His Glu Asp Ile Thr Lys Asp Lys Thr Ser Thr Val
Glu Ala Cys Leu Pro Leu Glu Leu Thr Lys Asn Glu Ser Cys Leu
Asn Ser Arg Glu Thr Ser Phe Ile Thr Asn Gly Ser Cys Leu Ala
Ser Arg Lys Thr Ser Phe MET MET Ala Leu Cys Leu Ser Ser Ile
Tyr Glu Asp Leu Lys MET Tyr Gln Val Glu Phe Lys Thr MET Asn
Ala Lys Leu Leu MET Asp Pro Lys Arg Gln Ile Phe Leu Asp Gln
Asn MET Leu Ala Val Ile Asp Glu Leu MET Gln Ala Leu Asn Phe
Asn Ser Glu Thr Val Pro Gln Lys Ser Ser Leu Glu Glu Pro Asp
Phe Tyr Lys Thr Lys Ile Lys Leu Cys Ile Leu Leu His Ala Phe
Arg Ile Arg Ala Val Thr Ile Asp Arg Val Thr Ser Tyr Leu Asn
Ala Ser

4. A polynucleotide encoding a subunit as claimed in any of claims 1 to 3.

5. A polynucleotide encoding a subunit as claimed in any one of claims 1 to 4 which polynucleotide comprises the nucleotide sequence

ATG TGT CCA GCG CGC AGC CTC CTC CTT GTG GCT ACC CTG GTC CTC
CTG GAC CAC CTC AGT TTG GCC AGA AAC CTC CCC GTG GCC ACT CCA
GAC CCA GGA ATG TTC CCA TGC CTT CAC CAC TCC CAA AAC CTG CTG
AGG GCC GTC AGC AAC ATG CTC CAG AAG GCC AGA CAA ACT CTA GAA
TTT TAC CCT TGC ACT TCT GAA GAG ATT GAT CAT GAA GAT ATC ACA
AAA GAT AAA ACC AGC ACA GTG GAG GCC TGT TTA CCA TTG GAA TTA
ACC AAG AAT GAG AGT TGC CTA AAT TCC AGA GAG ACC TCT TTC ATA
ACT AAT GGG AGT TGC CTG GCC TCC AGA AAG ACC TCT TTT ATG ATG
GCC CTG TGC CTT AGT AGT ATT TAT GAA GAC TTG AAG ATG TAC CAG
GTG GAG TTC AAG ACC ATG AAT GCA AAG CTT CTG ATG GAT CCT AAG
AGG CAG ATC TTT CTA GAT CAA AAC ATG CTG GCA GTT ATT GAT GAG
CTG ATG CAG GCC CTG AAT TTC AAC AGT GAG ACT GTG CCA CAA AAA
TCC TCC CTT GAA GAA CCG GAT TTT TAT AAA ACT AAA ATC AAG CTC
TGC ATA CTT CTT CAT GCT TTC AGA ATT CGG GCA GTG ACT ATT GAC
AGA GTG ACG AGC TAT CTG AAT GCT TCC.

6. A recombinant vector comprising a polynucleotide encoding a subunit as claimed in any one of claims 1 to 3 or comprising all or parts of the polynucleotide of claim 5.

7. A microorganism transformed with a recombinant vector comprising a polynucleotide encoding a subunit as claimed in any one of claims 1 to 3 or all or parts of the polynucleotide of claim 5.

8. A polyclonal or monoclonal antibody directed to a subunit as claimed in any one of claims 1 to 3.

9. A process for producing a subunit according to any one of claims 1 to 3 which process comprises culturing a microorganism transformed with a recombinant vector comprising a polynucleotide encoding the said subunit in a culture medium under conditions permitting the expression of the encoded subunit.

10. A process for producing a subunit according to any one of claims 1 to 3 which process comprises
(a) preparing sub-unit peptides of the said subunit by conventional peptide synthesis methods; and
(b) coupling the sub-unit peptides under conditions favouring the formation of peptide bonds.

11. A process for producing the CLMF protein which comprises a process as claimed in any one of claims 9 to 10.

12. A pharmaceutical composition comprising a subunit as claimed in any one of claims 1 to 3 and a pharmaceutically acceptable diluent, adjuvant or carrier.

13. Use of a subunit as claimed in any one of claims 1 to 3 for the manufacture of a medicament for antitumor therapy.

14. Use of a subunit as claimed in any one of claims 1 to 3 for the preparation of a CLMF protein.

## Patentansprüche

1. Untereinheit des zytotoxischen Lymphozyten-Reifungsfaktor- (CLMF) Proteins, **dadurch gekennzeichnet, dass**
(a) die Untereinheit die Aminosäuresequenz 
Arg Asn Leu Pro Val Ala Thr Pro Asp Pro Gly MET Phe Pro Cys
Leu His His Ser Gln Asn Leu Leu Arg Ala Val Ser Asn MET Leu
Gln Lys Ala Arg Gln Thr Leu Glu Phe Tyr Pro Cys Thr Ser Glu
Glu Ile Asp His Glu Asp Ile Thr Lys Asp Lys Thr Ser Thr Val
Glu Ala Cys Leu Pro Leu Glu Leu Thr Lys Asn Glu Ser Cys Leu
Asn Ser Arg Glu Thr Ser Phe Ile Thr Asn Gly Ser Cys Leu Ala
Ser Arg Lys Thr Ser Phe MET MET Ala Leu Cys Leu Ser Ser Ile
Tyr Glu Asp Leu Lys MET Tyr Gln Val Glu Phe Lys Thr MET Asn
Ala Lys Leu Leu MET Asp Pro Lys Arg Gln Ile Phe Leu Asp Gln
Asn MET Leu Ala Val Ile Asp Glu Leu MET Gln Ala Leu Asn Phe
Asn Ser Glu Thr Val Pro Gln Lys Ser Ser Leu Glu Glu Pro Asp
Phe Tyr Lys Thr Lys Ile Lys Leu Cys Ile Leu Leu His Ala Phe
Arg Ile Arg Ala Val Thr Ile Asp Arg Val Thr Ser Tyr Leu Asn
Ala Ser,
 umfasst, und dass
(b) bei einer Kombination mit der zweiten Untereinheit des CLMF-Proteins, welche die Aminosäuresequenz 
Ile Trp Glu Leu Lys Lys Asp Val Tyr Val Val Glu Leu Asp Trp
Tyr Pro Asp Ala Pro Gly Glu MET Val Val Leu Thr Cys Asp Thr
Pro Glu Glu Asp Gly Ile Thr Trp Thr Leu Asp Gln Ser Ser Glu
Val Leu Gly Ser Gly Lys Thr Leu Thr Ile Gln Val Lys Glu Phe
Gly Asp Ala Gly Gln Tyr Thr Cys His Lys Gly Gly Glu Val Leu
Ser His Ser Leu Leu Leu Leu His Lys Lys Glu Asp Gly Ile Trp
Ser Thr Asp Ile Leu Lys Asp Gln Lys Glu Pro Lys Asn Lys Thr
Phe Leu Arg Cys Glu Ala Lys Asn Tyr Ser Gly Arg Phe Thr Cys
Trp Trp Leu Thr Thr Ile Ser Thr Asp Leu Thr Phe Ser Val Lys
 
Ser Ser Arg Gly Ser Ser Asp Pro Gln Gly Val Thr Cys Gly Ala
Ala Thr Leu Ser Ala Glu Arg Val Arg Gly Asp Asn Lys Glu Tyr
Glu Tyr Ser Val Glu Cys Gln Glu Asp Ser Ala Cys Pro Ala Ala
Glu Glu Ser Leu Pro Ile Glu Val MET Val Asp Ala Val His Lys
Leu Lys Tyr Glu Asn Tyr Thr Ser Ser Phe Phe Ile Arg Asp Ile
Ile Lys Pro Asp Pro Pro Lys Asn Leu Gln Leu Lys Pro Leu Lys
Asn Ser Arg Gln Val Glu Val Ser Trp Glu Tyr Pro Asp Thr Trp
Ser Thr Pro His Ser Tyr Phe Ser Leu Thr Phe Cys Val Gln Val
Gln Gly Lys Ser Lys Arg Glu Lys Lys Asp Arg Val Phe Thr Asp
Lys Thr Ser Ala Thr Val Ile Cys Arg Lys Asn Ala Ser Ile Ser
Val Arg Ala Gln Asp Arg Tyr Tyr Ser Ser Ser Trp Ser Glu Trp
Ala Ser Val Pro Cys Ser
 umfasst, das kombinierte CLMF-Protein in einem T-Zell-Wachstumsfaktor-Assay aktiv ist.

2. Untereinheit nach Anspruch 1, worin das kombinierte CLMF-Protein bei einer Bestimmung in einem T-Zell-Wachstumsfaktor-Assay und bei einer Kombination mit dem in_Anspruch 1 (b) definierten Protein eine spezifische Aktivität von mindestens 5,2 x 10⁷ Units/mg zeigt.

3. Untereinheit nach Anspruch 1 oder 2, welche die Aminosäuresequenz 
Arg Asn Leu Pro Val Ala Thr Pro Asp Pro Gly MET Phe Pro Cys
Leu His His Ser Gln Asn Leu Leu Arg Ala Val Ser Asn MET Leu
Gln Lys Ala Arg Gln Thr Leu Glu Phe Tyr Pro Cys Thr Ser Glu
Glu Ile Asp His Glu Asp Ile Thr Lys Asp Lys Thr Ser Thr Val
Glu Ala Cys Leu Pro Leu Glu Leu Thr Lys Asn Glu Ser Cys Leu
Asn Ser Arg Glu Thr Ser Phe Ile Thr Asn Gly Ser Cys Leu Ala
Ser Arg Lys Thr Ser Phe MET MET Ala Leu Cys Leu Ser Ser Ile
Tyr Glu Asp Leu Lys MET Tyr Gln Val Glu Phe Lys Thr MET Asn
Ala Lys Leu Leu MET Asp Pro Lys Arg Gln Ile Phe Leu Asp Gln
Asn MET Leu Ala Val Ile Asp Glu Leu MET Gln Ala Leu Asn Phe
Asn Ser Glu Thr Val Pro Gln Lys Ser Ser Leu Glu Glu Pro Asp
Phe Tyr Lys Thr Lys Ile Lys Leu Cys Ile Leu Leu His Ala Phe
 
Arg Ile Arg Ala Val Thr Ile Asp Arg Val Thr Ser Tyr Leu Asn
Ala Ser
 umfasst.

4. Polynukleotid, welches für eine Untereinheit nach einem der Ansprüche 1 bis 3 codiert.

5. Polynukleotid, welches für eine Untereinheit nach einem der Ansprüche 1 bis 4 codiert, worin das Polynukleotid die Nukleotidsequenz 
ATG TGT CCA GCG CGC AGC CTC CTC CTT GTG GCT ACC CTG GTC CTC
CTG GAC CAC CTC AGT TTG GCC AGA AAC CTC CCC GTG GCC ACT CCA
GAC CCA GGA ATG TTC CCA TGC CTT CAC CAC TCC CAA AAC CTG CTG
AGG GCC GTC AGC AAC ATG CTC CAG AAG GCC AGA CAA ACT CTA GAA
TTT TAC CCT TGC ACT TCT GAA GAG ATT GAT CAT GAA GAT ATC ACA
AAA GAT AAA ACC AGC ACA GTG GAG GCC TGT TTA CCA TTG GAA TTA
ACC AAG AAT GAG AGT TGC CTA AAT TCC AGA GAG ACC TCT TTC ATA
ACT AAT GGG AGT TGC CTG GCC TCC AGA AAG ACC TCT TTT ATG ATG
GCC CTG TGC CTT AGT AGT ATT TAT GAA GAC TTG AAG ATG TAC CAG
GTG GAG TTC AAG ACC ATG AAT GCA AAG CTT CTG ATG GAT CCT AAG
AGG CAG ATC TTT CTA GAT CAA AAC ATG CTG GCA GTT ATT GAT GAG
CTG ATG CAG GCC CTG AAT TTC AAC AGT GAG ACT GTG CCA CAA AAA
TCC TCC CTT GAA GAA CCG GAT TTT TAT AAA ACT AAA ATC AAG CTC
TGC ATA CTT CTT CAT GCT TTC AGA ATT CGG GCA GTG ACT ATT GAC
AGA GTG ACG AGC TAT CTG AAT GCT TCC.
 umfasst.

6. Rekombinanter Vektor, welcher ein Polynukleotid umfasst, welches für eine Untereinheit nach einem der Ansprüch 1 bis 3 codiert oder welcher das gesamte oder Teile des Polynukleotids nach Anspruch 5 umfasst.

7. Mikroorganismus, welcher mit einem rekombinanten Vektor transformiert ist, welcher ein Polynukleotid umfasst, welches für eine Untereinheit nach einem der Ansprüche 1 bis 3 codiert oder welcher das gesamte oder Teile des Polynukleotids nach Anspruch 5 umfasst.

8. Polyklonaler oder monoklonaler Antikörper, welcher auf eine Untereinheit nach einem der Ansprüche 1 bis 3 gerichtet ist.

9. Verfahren zur Herstellung einer Untereinheit nach einem der Ansprüche 1 bis 3, worin das Verfahren Kultivieren eines Mikroorganismus, welcher mit einem rekombinanten Vektor transformiert ist, welcher ein Polynukleotid umfasst, welches für die Untereinheit codiert, in einem Kulturmedium unter Bedingungen umfasst, welche die Expression der codierten Untereinheit ermöglichen.

10. Verfahren zur Herstellung einer Untereinheit nach einem der Ansprüche 1 bis 3, worin das Verfahren
(a) Herstellen von Untereinheit-Peptiden der Untereinheit durch konventionelle Peptidsyntheseverfahren und
(b) Koppeln der Untereinheit-Peptide unter Bedingungen, welche die Bildung von Peptidbindungen begünstigen, umfasst.

11. Verfahren zur Herstellung des CLMF-Proteins, welches ein Verfahren nach einem der Ansprüche 9 bis 10 umfasst.

12. Pharmazeutische Zusammensetzung, welche eine Untereinheit nach einem der Ansprüche 1 bis 3 und ein pharmazeutisch verträgliches Verdünnungsmittel, Hilfsmittel oder Trägermittel umfasst.

13. Verwendung einer Untereinheit nach einem der Ansprüche 1 bis 3 zur Herstellung eines Arzneimittels zur Anti-Tumor-Therapie.

14. Verwendung einer Untereinheit nach einem der Ansprüche 1 bis 3 zur Bereitstellung eines CLMF-Proteins.

## Revendications

1. Sous-unité de la protéine facteur de maturation des lymphocytes cytotoxiques (CLMF), **caractérisée en ce que**
(a) la sous-unité comprend la séquence d'aminoacides 
Arg Asn Leu Pro Val Ala Thr Pro Asp Pro Gly MET Phe Pro Cys
Leu His His Ser Gln Asn Leu Leu Arg Ala Val Ser Asn MET Leu
Gln Lys Ala Arg Gln Thr Leu Glu Phe Tyr Pro Cys Thr Ser Glu
Glu Ile Asp His Glu Asp Ile Thr Lys Asp Lys Thr Ser Thr Val
Glu Ala Cys Leu Pro Leu Glu Leu Thr Lys Asn Glu Ser Cys Leu
Asn Ser Arg Glu Thr Ser Phe Ile Thr Asn Gly Ser Cys Leu Ala
Ser Arg Lys Thr Ser Phe MET MET Ala Leu Cys Leu Ser Ser Ile
Tyr Glu Asp Leu Lys MET Tyr Gln Val Glu Phe Lys Thr MET Asn
Ala Lys Leu Leu MET Asp Pro Lys Arg Gln Ile Phe Leu Asp Gln
Asn MET Leu Ala Val Ile Asp Glu Leu MET Gln Ala Leu Asn Phe
Asn Ser Glu Thr Val Pro Gln Lys Ser Ser Leu Glu Glu Pro Asp
Phe Tyr Lys Thr Lys Ile Lys Leu Cys Ile Leu Leu His Ala Phe
Arg Ile Arg Ala Val Thr Ile Asp Arg Val Thr Ser Tyr Leu Asn
Ala Ser,
 et **en ce que**
(b) si elle est combinée avec la deuxième sous-unité de la protéine CLMF comprenant la séquence d'aminoacides 
Ile Trp Glu Leu Lys Lys Asp Val Tyr Val Val Glu Leu Asp Trp
Tyr Pro Asp Ala Pro Gly Glu MET Val Val Leu Thr Cys Asp Thr
Pro Glu Glu Asp Gly Ile Thr Trp Thr Leu Asp Gln Ser Ser Glu
Val Leu Gly Ser Gly Lys Thr Leu Thr Ile Gln Val Lys Glu Phe
Gly Asp Ala Gly Gln Tyr Thr Cys His Lys Gly Gly Glu Val Leu
Ser His Ser Leu Leu Leu Leu His Lys Lys Glu Asp Gly Ile Trp
Ser Thr Asp Ile Leu Lys Asp Gln Lys Glu Pro Lys Asn Lys Thr
Phe Leu Arg Cys Glu Ala Lys Asn Tyr Ser Gly Arg Phe Thr Cys
Trp Trp Leu Thr Thr Ile Ser Thr Asp Leu Thr Phe Ser Val Lys
Ser Ser Arg Gly Ser Ser Asp Pro Gln Gly Val Thr Cys Gly Ala
Ala Thr Leu Ser Ala Glu Arg Val Arg Gly Asp Asn Lys Glu Tyr
Glu Tyr Ser Val Glu Cys Gln Glu Asp Ser Ala Cys Pro Ala Ala
 
Glu Glu Ser Leu Pro Ile Glu Val MET Val Asp Ala Val His Lys
Leu Lys Tyr Glu Asn Tyr Thr Ser Ser Phe Phe Ile Arg Asp Ile
Ile Lys Pro Asp Pro Pro Lys Asn Leu Gln Leu Lys Pro Leu Lys
Asn Ser Arg Gln Val Glu Val Ser Trp Glu Tyr Pro Asp Thr Trp
Ser Thr Pro His Ser Tyr Phe Ser Leu Thr Phe Cys Val Gln Val
Gln Gly Lys Ser Lys Arg Glu Lys Lys Asp Arg Val Phe Thr Asp
Lys. Thr Ser Ala Thr Val Ile Cys Arg Lys Asn Ala Ser Ile Ser
Val Arg Ala Gln Asp Arg Tyr Tyr Ser Ser Ser Trp Ser Glu Trp
Ala Ser Val Pro Cys Ser
 la protéine CLMF combinée est active dans une analyse du facteur de croissance des lymphocytes T.

2. Sous-unité selon la revendication 1, avec laquelle, lorsqu'elle est combinée avec la protéine telle que définie dans la revendication 1(b), la protéine CLMF combinée présente une activité spécifique, déterminée dans une analyse du facteur de croissance des lymphocytes T, d'au moins 5,2 × 10⁷ unités/mg.

3. Sous-unité selon la revendication 1 ou 2, comprenant la séquence d'aminoacides

Arg Asn Leu Pro Val Ala Thr Pro Asp Pro Gly MET Phe Pro Cys
Leu His His Ser Gln Asn Leu Leu Arg Ala Val Ser Asn MET Leu
Gln Lys Ala Arg Gln Thr Leu Glu Phe Tyr Pro Cys Thr Ser Glu
Glu Ile Asp His Glu Asp Ile Thr Lys Asp Lys Thr Ser Thr Val
Glu Ala Cys Leu Pro Leu Glu Leu Thr Lys Asn Glu Ser Cys Leu
Asn Ser Arg Glu Thr Ser Phe Ile Thr Asn Gly Ser Cys Leu Ala
Ser Arg Lys Thr Ser Phe MET MET Ala Leu Cys Leu Ser Ser Ile
Tyr Glu Asp Leu Lys MET Tyr Gln Val Glu Phe Lys Thr MET Asn
Ala Lys Leu Leu MET Asp Pro Lys Arg Gln Ile Phe Leu Asp Gln
Asn MET Leu Ala Val Ile Asp Glu Leu MET Gln Ala Leu Asn Phe
Asn Ser Glu Thr Val Pro Gln Lys Ser Ser Leu Glu Glu Pro Asp
Phe Tyr Lys Thr Lys Ile Lys Leu Cys Ile Leu Leu His Ala Phe
Arg Ile Arg Ala Val Thr Ile Asp Arg Val Thr Ser Tyr Leu Asn
Ala Ser

4. Polynucléotide codant pour une sous-unité selon l'une quelconque des revendications 1 à 3.

5. Polynucléotide codant pour une sous-unité selon l'une quelconque des revendications 1 à 4, ce polynucléotide comprenant la séquence de nucléotides

ATG TGT CCA GCG CGC AGC CTC CTC CTT GTG GCT ACC CTG GTC CTC
CTG GAC CAC CTC AGT TTG GCC AGA AAC CTC CCC GTG GCC ACT CCA
GAC CCA GGA ATG TTC CCA TGC CTT CAC CAC TCC CAA AAC CTG CTG
AGG GCC GTC AGC AAC ATG CTC CAG AAG GCC AGA CAA ACT CTA GAA
TTT TAC CCT TGC ACT TCT GAA GAG ATT GAT CAT GAA GAT ATC ACA
AAA GAT AAA ACC AGC ACA GTG GAG GCC TGT TTA CCA TTG GAA TTA
ACC AAG AAT GAG AGT TGC CTA AAT TCC AGA GAG ACC TCT TTC ATA
ACT AAT GGG AGT TGC CTG GCC TCC AGA AAG ACC TCT TTT ATG ATG
GCC CTG TGC CTT AGT AGT ATT TAT GAA GAC TTG AAG ATG TAC CAG
GTG GAG TTC AAG ACC ATG AAT GCA AAG CTT CTG ATG GAT CCT AAG
AGG CAG ATC TTT CTA GAT CAA AAC ATG CTG GCA GTT ATT GAT GAG
CTG ATG CAG GCC CTG AAT TTC AAC AGT GAG ACT GTG CCA CAA AAA
TCC TCC CTT GAA GAA CCG GAT TTT TAT AAA ACT AAA ATC AAG CTC
TGC ATA CTT CTT CAT GCT TTC AGA ATT CGG GCA GTG ACT ATT GAC
AGA GTG ACG AGC TAT CTG AAT GCT TCC.

6. Vecteur recombiné comprenant un polynucléotide codant pour une sous-unité selon l'une quelconque des revendications 1 à 3 ou comprenant la totalité ou des parties du polynucléotide selon la revendication 5.

7. Micro-organisme transformé avec un vecteur recombiné comprenant un polynucléotide codant pour une sous-unité selon l'une quelconque des revendications 1 à 3 ou la totalité ou des parties du polynucléotide selon la revendication 5.

8. Anticorps polyclonal ou monoclonal dirigé contre une sous-unité selon l'une quelconque des revendications 1 à 3.

9. Procédé de production d'une sous-unité selon l'une quelconque des revendications 1 à 3, ce procédé comprenant la culture d'un micro-organisme transformé avec un vecteur recombiné comprenant un polynucléotide codant pour ladite sous-unité dans un milieu de culture dans des conditions permettant l'expression de la sous-unité codée.

10. Procédé de production d'une sous-unité selon l'une quelconque des revendications 1 à 3, ce procédé comprenant
(a) la préparation de peptides sous-unités de ladite sous-unité par des procédés de synthèse peptidique classiques; et
(b) le couplage des peptides sous-unités dans des conditions favorisant la formation de liaisons peptidiques.

11. Procédé de production de la protéine CLMF qui comprend un procédé selon l'une quelconque des revendications 9 à 10.

12. Composition pharmaceutique comprenant une sous-unité selon l'une quelconque des revendications 1 à 3 et un diluant, adjuvant ou support pharmaceutiquement acceptable.

13. Utilisation d'une sous-unité selon l'une quelconque des revendications 1 à 3 pour la fabrication d'un médicament destiné à une thérapie antitumorale.

14. Utilisation d'une sous-unité selon l'une quelconque des revendications 1 à 3 pour la préparation d'une protéine CLMF.
